(19) Europäisches Patentamt
European Patent Office
Office européen des brevets

(11) EP 4 151 597 B1

(12) **EUROPEAN PATENT SPECIFICATION**

(45) Date of publication and mention of the grant of the patent:
**15.04.2026 Bulletin 2026/16**

(21) Application number: **21804320.6**

(22) Date of filing: **12.05.2021**

(51) International Patent Classification (IPC):
***C01B 39/36*** *(2006.01)* ***B01J 32/00*** *(2006.01)*
***C01B 33/12*** *(2006.01)* ***C07F 7/18*** *(2006.01)*
***C07H 21/00*** *(2006.01)* ***C07F 7/12*** *(2006.01)*
***C07F 9/6558*** *(2006.01)* ***C07H 1/02*** *(2006.01)*
***C07F 7/08*** *(2006.01)* ***C07F 7/10*** *(2006.01)*
***C07F 9/6561*** *(2006.01)*

(52) Cooperative Patent Classification (CPC):
**C07F 7/1804; C01B 33/12; C01B 39/36; C07F 7/0838; C07H 1/02;** C07F 7/0836; C07F 7/10; C07F 7/12; C07F 9/65583; C07F 9/65616; Y02P 20/55

(86) International application number:
**PCT/JP2021/018075**

(87) International publication number:
**WO 2021/230293 (18.11.2021 Gazette 2021/46)**

(54) **INORGANIC POROUS SUBSTRATE, INORGANIC POROUS SUPPORT, AND NUCLEIC ACID PRODUCTION METHOD**

ANORGANISCHES PORÖSES SUBSTRAT, ANORGANISCHER PORÖSER TRÄGER UND NUKLEINSÄUREHERSTELLUNGSVERFAHREN

SUBSTRAT POREUX INORGANIQUE, SUPPORT POREUX INORGANIQUE ET MÉTHODE DE PRODUCTION D’ACIDE NUCLÉIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.05.2020 JP 2020084641**

(43) Date of publication of application:
**22.03.2023 Bulletin 2023/12**

(73) Proprietor: **SUMITOMO CHEMICAL COMPANY, LIMITED**
**Tokyo 103-6020 (JP)**

(72) Inventors:
- **ISHIYAMA, Takeshi**
  **Ichihara-shi, Chiba 299-0195 (JP)**
- **OSHIRO, Ikuya**
  **Osaka-shi, Osaka 555-0021 (JP)**
- **HARA, Takashi**
  **Osaka-shi, Osaka 555-0021 (JP)**

(74) Representative: **Marks & Clerk LLP**
**15 Fetter Lane**
**London EC4A 1BW (GB)**

(56) References cited:
**EP-A1- 3 950 126** **WO-A1-2013/062105**
**WO-A1-2020/202951** **WO-A1-2020/202952**
**WO-A2-00/50432** **CN-A- 108 176 387**
**JP-A- 2003 104 996** **JP-A- 2006 502 856**
**JP-A- 2016 202 097** **JP-A- H03 181 334**
**US-A1- 2014 135 478**

- **FARRE CAROLE ET AL.: "Automated Oligonucleotide Solid-Phase Synthesis on Nanosized Silica Particle Using Nano-on-Micro Assembled Particle Supports", LANGMUIR, vol. 26, no. 7, 2010, pages 4941 - 4950, XP055767353, DOI: 10.1021/1a903572q**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 4 151 597 B1

Remarks:

The complete document including Reference Table(s) and the Sequence Listing(s) can be downloaded from the EPO website

## Description

### TECHNICAL FIELD

This application claims priority to and the benefit of priority from the Paris Convention based on Japanese Patent Application No. 2020-084641 filed on May 13, 2020.

The present invention relates to an inorganic porous substrate, an inorganic porous support derived from the inorganic porous substrate, and a nucleic acid production method using the inorganic porous support.

### BACKGROUND ART

As a chemical synthesis method of a nucleic acid, a solid-phase synthesis method by a phosphoramidite method has been widely used. In this method, first, a functional group such as an amino group is introduced onto an inorganic porous body using a silane coupling agent and the like, and a nucleoside providing a 3' end of the nucleic acid is bound to the functional group. Then, a nucleic acid elongation reaction is carried out on the solid-phase support starting from the nucleoside. In the solid-phase synthesis method, in particular, when a strand length of the nucleic acid to be synthesized becomes long, a synthesis efficiency drastically decreases, and consequently a large amount of by-products is prone to be mixed. Thus, the purity of the synthesized nucleic acid is not always satisfactory, and the synthesis is not efficient.

In the solid-phase synthesis method, since various reactive substrates/solvents are sequentially reacted with the solid-phase support, it is considered that a surface functional group of the solid-phase support, solvent affinity of the surface of the solid-phase support derived therefrom, and the like may affect the reaction. For example, an example in which a solid-phase support is modified with methyltrimethoxysilane has been reported (see Patent Document 1). EP 3 950 126 A1 discloses an inorganic porous carrier having a pore distribution in which a pore diameter is 0.04 μm or more, and comprises a linker represented by formula (1):

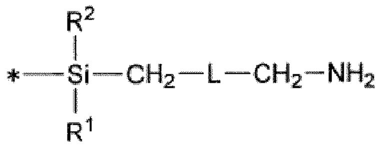

···(1)

wherein, a bond marked with * represents a bond to an oxygen atom of a silanol group in an inorganic porous substance, $R^1$ and $R^2$ represent each independently an alkyl group containing 3 to 10 carbon atoms, or a phenyl group, L represents a single bond; an alkylene group containing 1 to 20 carbon atoms; or an alkylene group containing 2 to 20 carbon atoms which contains $-CH_2$-Q-$CH_2$-group, wherein any group Q selected from a group consisting of -O-, -NH-, -NH-CO-, and -NH-CO-NH- is inserted into at least one of $-CH_2-CH_2-$ group constituting the alkylene group (see Patent Document 2). Methods and compositions to label oligonucleotides and analogs directly on a solid-support having the structure (I) are disclosed in WO 00/50432 A2 (Patent document 3):

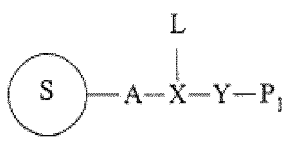

where S is a solid-support, A is a cleavable linker, X is a moiety with three or more attachment sites, L is a label, Y is a nucleophile, and P1 is an acid cleavable protecting group.

A material composed of a porous support on which functionalized nanoparticles are grafted by covalent bonding is disclosed in US 2014/135478 A1 (Patent Document 4), wherein at least part of the nanoparticles grafted by covalent bonding is housed inside surface pores of the support, and in that the support is silica-based and is in the form of porous particles of heterogeneous shape and size, the size of the particles being larger than 1 μm. Farre et *al* (Non-patent Document 1) discloses the reversible immobilization of silica nanoparticles (NPs) on micrometric silica beads.

A magnetic carrier for bonding a nucleic acid in which ferromagnetic iron oxide particles are coated with silica, and amino groups or carboxylic groups are imparted to the silica coated surface as functional groups are disclosed by JP 2003-104996 A (Patent Document 5). A carrier for nucleic acid solid-phase synthesis represented by the following formula (III) is disclosed by JP 2016-202097 A (Patent Document 6):

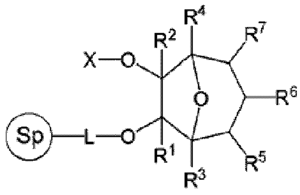 (III)

Compositions for the separation of metals or biomolecules such as polypeptides, nucleic acids, or endotoxins using modified solid supports are disclosed by JP 2006-502856 A (Patent Document 7). Patent Document 8 (CN 108176387 A) discloses a silica gel chromatographic packing is prepared from silica gel and organosilane, which is bound to the surface of the silica gel; and the organosilane is prepared from aminopropyl silane and other silanes.

WO 2013/062105 A1 (Patent Document 9) discloses silica spheres, which satisfy the following conditions:

(a) the average particle size is from 30 μm to 40 μm as measured by a laser light scattering method;
(b) the ratio (D10/D90) of the particle size (D10) of smaller 10% cumulative volume to the particle size (D90) of 90% cumulative volume in a particle size distribution as measured by a Coulter counter method, is at most 1.50; and
(c) the average pore size is from 85 nm to 115 nm and the pore volume is at least 1.5 mL/g, as measured by a mercury intrusion technique.

WO 2020/202951 A1 (Patent Document 10) discloses an inorganic porous carrier comprising a linker represented by general formula (1):

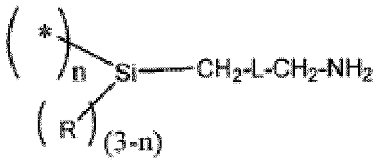 ···(1)

wherein a mode diameter in a pore distribution as measured by mercury intrusion method is 0.04 μm to 1 μm, and also a predetermined cumulative pore volume ratio is 30 % or less in the formula (1), a bond marked with * represents a linkage to the oxygen atom of a silanol group in an inorganic porous substance; n is an integer of 1 etc.; R represents independently of each other an alkyl group containing 3 to 10 carbon atoms; and L represents a single bond; an alkylene group of 1 to 20 carbon atoms; or an alkylene group containing 2 to 20 carbon atoms which contains $-CH_2-Q-CH_2-$ group wherein any group Q selected from a group consisting of -O- etc. is inserted into at least one of $-CH_2-CH_2-$ group constituting the alkylene group.

WO 2020/202952 A1 (Patent Document 11) discloses an inorganic porous carrier that comprises a linker represented by general formula (1):

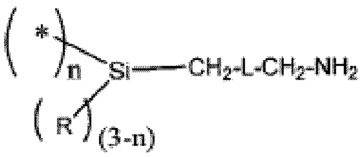 ···(1)

and has mode diameter of 0.04 μm to 1 μm in a pore distribution as measured by mercury intrusion method, and the density of voids having an opening area of 0.0025 $\mu m^2$ or more as obtained by image analysis of the surface of the inorganic porous substance is 12 to 30 voids/$\mu m^2$ in the formula (1), a bond marked with * represents a linkage to the oxygen atom in a silanol group of an inorganic porous substance; n is an integer of 1 etc.; R represents independently of each other an alkyl group containing 3 to 10 carbon atoms which may optionally have a substituent such as an alkoxy group etc.; and L represents a single bond; an alkylene group of 1 to 20 carbon atoms; or an alkylene group containing 2 to 20 carbon atoms which contains $-CH_2-Q-CH_2-$ group wherein any group Q selected from a group consisting of -O- etc. is inserted into at least one of $-CH_2-CH_2-$ group constituting the alkylene group.

PRIOR ART DOCUMENT

PATENT DOCUMENT

Patent Document 1: JP-A-3-181334
Patent Document 2: EP 3 950 126 A1
Patent Document 3: WO 00/50432 A2
Patent Document 4: US 2014/135478 A1
Patent Document 5: JP 2003-104996 A
Patent Document 6: JP 2016-202097 A
Patent Document 7: JP 2006-502856 A
Patent Document 8: CN 108176387 A
Patent Document 9: WO 2013/062105 A1
Patent Document 10: WO 2020/202951 A1
Patent Document 11: WO 2020/202952 A1

NON-PATENT DOCUMENT

Non-patent Document 1: Farre, C. et al, Langmuir, 2010, 26(7), pages 4941-4950

SUMMARY OF THE INVENTION

PROBLEMS TO BE SOLVED BY THE INVENTION

The present invention has been made in view of the above circumstances, and an object thereof is to provide an inorganic porous substrate serving as a precursor of an inorganic porous support with which purity and the like can be further enhanced in the production of a nucleic acid, an inorganic porous support, and a nucleic acid production method using the inorganic porous support.

MEANS FOR SOLVING THE PROBLEMS

As a result of intensive studies to achieve the above object, the present inventors have found that by substituting a silanol (SiOH) group on a surface of an inorganic porous support with a specific silyl group in synthesis of a nucleic acid, formation of a byproduct in production of an oligonucleic acid product can be suppressed. Thus, the present invention provides an inorganic porous substrate serving as a precursor of an inorganic porous support, which has a silyl group having a specific structure and has a constant pore diameter, an inorganic porous support, and a nucleic acid production method using the inorganic porous support.

In order to solve the above problems, the following aspects are specifically included, but not limited thereto.

[1] An inorganic porous substrate that comprises silyl groups represented by the following (i) and (ii) and has the following characteristics (iii) to (v):

(i) a silyl group (A): a silyl group represented by the following formula (i-1),
(ii) a silyl group (B): at least one silyl group selected from the group consisting of silyl groups represented by the following formulas (ii-1), (ii-2), and (ii-3),
(iii) a particle diameter of 1 μm or more, as determined by scanning electron microscopy as an average value of the major diameters of 50 arbitrary particles,
(iv) a pore diameter of 20 nm or more, as determined by the mercury intrusion method, and
(v) a cumulative pore volume in a pore diameter range of 40 nm to 1000 nm of more than 0.32 mL/g and 4 mL/g or less, as determined by the mercury intrusion method:
[Chemical Formula 1]

$$(X1)(Y1)_a(Z1)_bSi\text{-}A1\text{-}NH\text{-}B1 \quad \cdots \text{(i-1)}$$

wherein in the formula (i-1),

X1 represents a bond with the inorganic porous substrate,
Y1 each independently represents any one selected from the group consisting of a bond with an inorganic

porous substrate, a hydroxyl group, an amino group, an alkoxy group having 1 to 6 carbon atoms, and an alkylamino group having 1 to 12 carbon atoms,
Z1 each independently represents an alkyl group having 1 to 6 carbon atoms or an aryl group having 6 to 12 carbon atoms,
a represents an integer represented by 2-b,
b represents an integer of 0 to 2,
A1 represents an organic group having 1 to 20 carbon atoms, and
B1 represents any one selected from the group consisting of a hydrogen atom, an alkyl group having 1 to 6 carbon atoms, and an aryl group having 6 to 12 carbon atoms.],
[Chemical Formula 2]

(P1)(Q1)(R1)Si-J1 ··· (ii-1),

[Chemical Formula 3]

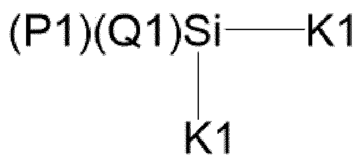

・・・ (ii－2)

,

[Chemical Formula 4]

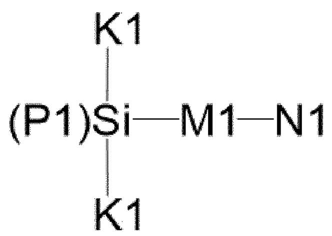

・・・ (ii－3)

,

wherein in the formula (ii-1), (ii-2) or (ii-3),
P1 represents a bond with the inorganic porous substrate,
Q1 and R1 each independently represents any one selected from the group consisting of a bond with an inorganic porous substrate, a hydroxyl group, an amino group, an alkoxy group having 1 to 6 carbon atoms, and an alkylamino group having 1 to 12 carbon atoms,
J1 represents an alkyl group having 2 to 20 carbon atoms or an aryl group having 7 to 20 carbon atoms,
K1 each independently represents an alkyl group having 1 to 20 carbon atoms or an aryl group having 6 to 20 carbon atoms,
M1 represents an alkylene group having 1 to 6 carbon atoms, and
N1 represents an alkyl group having 2 to 20 carbon atoms or an aryl group having 6 to 20 carbon atoms.

[2] The inorganic porous substrate according to [1], wherein the inorganic porous substrate has a pore diameter of from 40 nm to 500 nm, as determined by the mercury intrusion method.
[3] The inorganic porous substrate according to any one of [1] to [2], wherein the inorganic porous substrate has a specific surface area of from 0.1 $m^2/g$ to 200 $m^2/g$, as determined by nitrogen adsorption/desorption isotherm measurement.
[4] The inorganic porous substrate according to any one of [1] to [3], comprising silica, silica gel, zeolite, or glass.
[5] The inorganic porous substrate according to any one of [1] to [4], wherein the inorganic porous substrate comprises an active NH group represented by the following formula (NH-1) , wherein the amount of the active NH group satisfies the following mathematical formula (NH#1).
[Chemical Formula 5]

Si-A1-NH-B1 ··· (NH-1)

wherein in the formula (NH-1),

A1 represents an organic group having 1 to 20 carbon atoms,
B1 represents any one selected from the group consisting of a hydrogen atom, an alkyl group having 1 to 6 carbon atoms, and an aryl group having 6 to 12 carbon atoms.
[Math. 1]

$$0.05 \leq I1/S1 \leq 6.0 \quad \cdots \text{(NH\#1)}$$

I1: the amount of the active NH group in the inorganic porous substrate (μmol/g),
S1: the specific surface area ($m^2/g$) of the inorganic porous substrate obtained by nitrogen adsorption/desorption isotherm measurement

[6] The inorganic porous substrate according to any one of [1] to [5], wherein the silyl group (B) is represented by the formula (ii-3).
[7] The inorganic porous substrate according to any one of [1] to [6], wherein the silyl group represented by the formula (ii-3) is a silyl group represented by the following formula (ii-3-1).

[Chemical Formula 6]

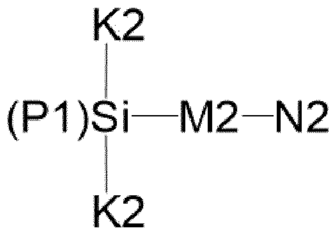

· · · (ii-3-1)

wherein in the formula (ii-3-1),

P1 represents a bond with the inorganic porous substrate,
K2 each independently represents an alkyl group having 1 to 6 carbon atoms or an aryl group having 6 to 10 carbon atoms,
M2 represents an alkylene group having 1 to 4 carbon atoms, and
N2 represents an alkyl group having 2 to 6 carbon atoms or an aryl group having 6 to 10 carbon atoms.

[8] The inorganic porous substrate according to any one of [1] to [7], wherein A1 in the formula (i-1) is an alkylene group having 1 to 20 carbon atoms which may optionally contain any one or more of an imino group, an oxy group, and a thio group.
[9] The inorganic porous substrate according to any one of [1] to [8], wherein the silyl group (A) is represented by the following formula (i-1-1).
[Chemical Formula 7]

$$(X1)(Z1)_2Si\text{-}A2\text{-}NH\text{-}B2 \quad \cdots \text{(i-1-1)}$$

wherein in the formula (i-1-1),

X1 represents a bond with the inorganic porous substrate,
Z1 each independently represents an alkyl group having 1 to 6 carbon atoms or an aryl group having 6 to 12 carbon atoms,
A2 represents an alkylene group having 1 to 15 carbon atoms which may optionally contain any one or more of an imino group, an oxy group, and a thio group, and
B2 represents any one selected from the group consisting of a hydrogen atom and an alkyl group having 1 to 2 carbon atoms.

[10] An inorganic porous support having silyl groups of the following (vi) and (vii) and having the following

characteristic (viii).

(vi) a silyl group (C): a silyl group represented by the following formula (vi-1),
(vii) a silyl group (D): at least one silyl group selected from the group consisting of silyl groups represented by the following formulas (vii-1), (vii-2), and (vii-3), and
(viii) a pore diameter of 20 nm or more, as determined by the mercury intrusion method.

[Chemical Formula 8]

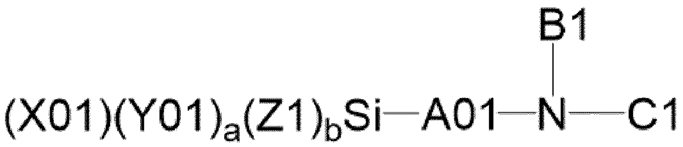

··· (vi-1)

wherein in the formula (vi-1),

X01 represents a bond with the inorganic porous support,
Y01 each independently represents any one selected from the group consisting of a bond with an inorganic porous support, a hydroxyl group, an amino group, an alkoxy group having 1 to 6 carbon atoms, and an alkylamino group having 1 to 12 carbon atoms,
Z1 each independently represents an alkyl group having 1 to 6 carbon atoms or an aryl group having 6 to 12 carbon atoms,
a represents an integer represented by 2-b,
b represents an integer of 0 to 2,
A01 represents an organic group having 1 to 20 carbon atoms,
B1 represents any one selected from the group consisting of a hydrogen atom, an alkyl group having 1 to 6 carbon atoms, and an aryl group having 6 to 12 carbon atoms, and
C1 represents a group having a nucleoside or nucleotide structure in which a reactive group is protected or deprotected,
[Chemical Formula 9]

(P01)(Q01)(R01)Si-J1 ··· (vii-1),

[Chemical Formula 10]

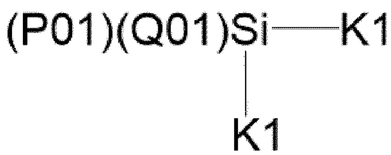

··· (vii-2)

,

[Chemical Formula 11]

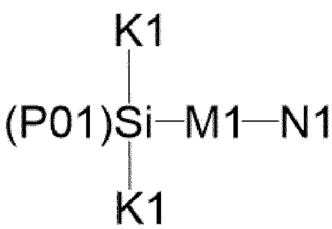

··· (vii-3)

wherein in the formula (vii-1), (vii-2) or (vii-3),

P01 represents a bond with the inorganic porous support,
Q01 and R01 each independently represents any one selected from the group consisting of a bond with an inorganic porous support, a hydroxyl group, an amino group, an alkoxy group having 1 to 6 carbon atoms, and an alkylamino group having 1 to 12 carbon atoms,
J1 represents an alkyl group having 2 to 20 carbon atoms or an aryl group having 7 to 20 carbon atoms,
K1 each independently represents an alkyl group having 1 to 20 carbon atoms or an aryl group having 6 to 20 carbon atoms,
M1 represents an alkylene group having 1 to 6 carbon atoms, and
N1 represents an alkyl group having 2 to 20 carbon atoms or an aryl group having 6 to 20 carbon atoms.]

[11] The inorganic porous support according to [10], wherein the inorganic porous support has a pore diameter of from 40 nm to 500 nm, as determined by the mercury intrusion method.
[12] The inorganic porous support according to any one of [10] to [11], wherein the inorganic porous support has a cumulative pore volume in a pore diameter range of 40 nm to 1000 nm of more than 0.32 mL/g and 4 mL/g or less, as determined by the mercury intrusion method.
[13] The inorganic porous support according to any one of [10] to [12], wherein the inorganic porous support has a specific surface area of 0.1 $m^2/g$ or more and 200 $m^2/g$ or less, as determined by nitrogen adsorption/desorption isotherm measurement.
[14] The inorganic porous support according to any one of [10] to [13], wherein the inorganic porous support comprises an inorganic porous body which is composed of silica, silica gel, zeolite, or glass.
[15] The inorganic porous support according to any one of [10] to [14], wherein an amount of a group containing a nucleoside or a nucleotide structure in which a reactive group is protected or deprotected satisfies the following mathematical formula (Nu#1).
[Math. 2]

$$0.05 \leq I01/S01 \leq 3.0 \cdots \text{(Nu\#1)}$$

I01: a group (μmol/g) having a nucleoside or nucleotide structure in which a reactive group is protected or deprotected in the inorganic porous support, and
S01: a specific surface area ($m^2/g$) of the inorganic porous support obtained by nitrogen adsorption/desorption isotherm measurement

[16] The inorganic porous support according to any one of [10] to [15], wherein C1 in the general formula (vi-1) contains a succinyl linker.
[17] The inorganic porous support according to any one of [10] to [16], having a silyl group represented by the formula (vii-3) as a silyl group (D).
[18] The inorganic porous support according to any one of [10] to [17], wherein the silyl group represented by the formula (vii-3) is a silyl group represented by the following formula (vii-3-1).

[Chemical Formula 12]

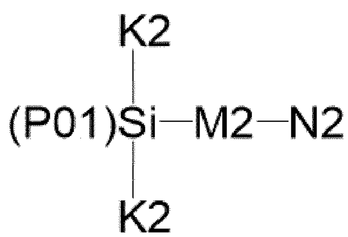

· · · (vii−3−1)

wherein in the formula (ii-3-1),

P01 represents a bond with the inorganic porous support,
K2 each independently represents an alkyl group having 1 to 6 carbon atoms or an aryl group having 6 to 10 carbon atoms,
M2 represents an alkylene group having 1 to 4 carbon atoms, and
N2 represents an alkyl group having 2 to 6 carbon atoms or an aryl group having 6 to 10 carbon atoms.

[19] The inorganic porous support according to any one of [10] to [18], wherein A01 in the formula (vi-1) is an alkylene group having 1 to 20 carbon atoms which may optionally contain any one or more of an acylimino group, an oxy group, and a thio group.
[20] The inorganic porous support according to any one of [10] to [19], wherein the silyl group (C) is represented by the following formula (vi-1-1).

[Chemical Formula 13]

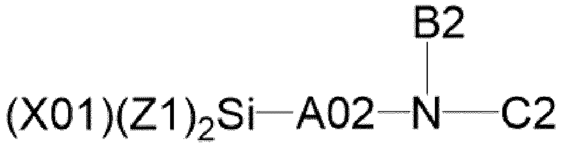

· · · (vi-1-1)

wherein in the formula (vi-1-1),

X01 represents a bond with the inorganic porous support,
Z1 each independently represents an alkyl group having 1 to 6 carbon atoms or an aryl group having 6 to 12 carbon atoms,
A02 represents an alkylene group having 1 to 15 carbon atoms which may optionally contain any one or more of an acylimino group, an oxy group, and a thio group,
B2 represents any one selected from the group consisting of a hydrogen atom and an alkyl group having 1 to 2 carbon atoms, and
C2 represents a group including a succinyl linker and having a nucleoside or nucleotide structure in which a reactive group is protected or deprotected.

[21] A nucleic acid production method using the inorganic porous support according to any one of [10] to [20], in which C1 in the formula (vi-1) has a group having nucleoside or nucleotide structure in which the hydroxyl group as a reactive group is protected, the nucleic acid production method including:

a step (A) of deprotecting a protecting group of the hydroxyl group at the 5'-position of the nucleoside;
a step (B) of producing a phosphite by subjecting the hydroxyl group at the 5'-position of the nucleoside produced in the step (A) to a condensation reaction with an amidite compound having a second nucleoside base;
a step (C) of oxidizing the phosphite produced in the step (B) to produce a nucleotide; and
a step (D) of deprotecting the protecting group of the hydroxyl group at the 5'-position of the nucleotide produced in the step (C).

[22] The nucleic acid production method according to [21], further including:

a step (B') of further subjecting a product produced in the step (D) to a condensation reaction with an amidite compound having a nucleoside base to be introduced next to produce a phosphite;
a step (C') of oxidizing the phosphite produced in the step (B') to produce an oligonucleotide; and
a step (D') of deprotecting the protecting group for the hydroxyl group at the 5'-position of a terminal of an oligonucleotide chain produced in the step (C').

[23] The nucleic acid production method according to [22], including a step (E) of further repeating a series of steps including the step (B'), the step (C'), and the step (D') m times (m represents an integer of 1 or more) to react m amidite compounds, and then cutting out an elongated nucleic acid.
[24] Use of the inorganic porous support according to any one of [10] to [20] in production of a nucleic acid by a phosphoramidite method.

EFFECT OF THE INVENTION

According to the present invention, there are provided an inorganic porous substrate serving as a precursor of an inorganic porous support capable of improving purity even in long-chain nucleic acid synthesis by substituting a silanol group on a surface of the inorganic porous support with a specific silyl group, the inorganic porous support, and a method of

producing a high-purity nucleic acid using the inorganic porous support.

## MODE FOR CARRYING OUT THE INVENTION

An inorganic porous substrate of the present invention has a silyl group represented by the following (i) and (ii) and has the following characteristics (iii) to (v) :

(i) a silyl group (A): a silyl group represented by the following formula (i-1),
(ii) a silyl group (B): at least one silyl group selected from the group consisting of silyl groups represented by the following formulas (ii-1), (ii-2), and (ii-3),
(iii) a particle diameter of 1 μm or more,
(iv) a pore diameter of 20 nm or more, and
(v) a cumulative pore volume in a pore diameter range of 40 nm to 1000 nm of more than 0.32 mL/g and 4 mL/g or less.

[Chemical Formula 14]

$$(X1)(Y1)_a(Z1)_bSi\text{-}A1\text{-}NH\text{-}B1 \quad \cdots \text{(i-1)}$$

[In the formula (i-1),

X1 represents a bond with the inorganic porous substrate,
Y1 each independently represents any one selected from the group consisting of a bond with an inorganic porous substrate, a hydroxyl group, an amino group, an alkoxy group having 1 to 6 carbon atoms, and an alkylamino group having 1 to 12 carbon atoms,
Z1 each independently represents an alkyl group having 1 to 6 carbon atoms or an aryl group having 6 to 12 carbon atoms,
a represents an integer represented by 2-b,
b represents an integer of 0 to 2,
A1 represents an organic group having 1 to 20 carbon atoms, and
B1 represents any one selected from the group consisting of a hydrogen atom, an alkyl group having 1 to 6 carbon atoms, and an aryl group having 6 to 12 carbon atoms.],
[Chemical Formula 15]

$$(P1)(Q1)(R1)Si\text{-}J1 \cdots \quad \text{(ii-1)},$$

[Chemical Formula 16]

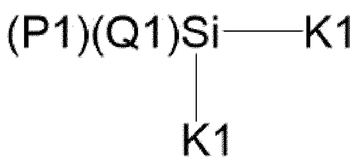

· · · (ii-2)

,

[Chemical Formula 17]

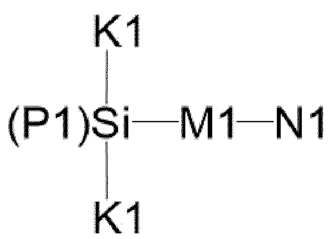

· · · (ii-3)

[In the formula (ii-1), (ii-2) or (ii-3),

P1 represents a bond with the inorganic porous substrate,
Q1 and R1 each independently represents any one selected from the group consisting of a bond with an inorganic porous substrate, a hydroxyl group, an amino group, an alkoxy group having 1 to 6 carbon atoms, and an alkylamino group having 1 to 12 carbon atoms,
J1 represents an alkyl group having 2 to 20 carbon atoms or an aryl group having 7 to 20 carbon atoms,
K1 each independently represents an alkyl group having 1 to 20 carbon atoms or an aryl group having 6 to 20 carbon atoms,
M1 represents an alkylene group having 1 to 6 carbon atoms, and
N1 represents an alkyl group having 2 to 20 carbon atoms or an aryl group having 6 to 20 carbon atoms.].

In the formula (i-1), the bond with the inorganic porous substrate in X1 is specifically a bond via an -O-bond on the surface of the inorganic porous substrate.
In the formula (i-1), Y1 each independently represents any one selected from the group consisting of the bond with the inorganic porous substrate, a hydroxyl group, an amino group, an alkoxy group having 1 to 6 carbon atoms, and an alkylamino group having 1 to 12 carbon atoms.
Here, the bond with the inorganic porous substrate in Y1 is the same as in X1 above.
The alkoxy group having 1 to 6 carbon atoms in Y1 is preferably an alkoxy group having 1 to 3 carbon atoms, and specific examples thereof include a methoxy group, an ethoxy group, an n-propoxy group, and an i-propoxy group.
The alkylamino group having 1 to 12 carbon atoms in Y1 is preferably an alkylamino group having 1 to 6 carbon atoms. The alkylamino group herein may be a monoalkylamino group or a dialkylamino group, and examples thereof preferably include a dialkylamino group. Specific examples thereof include a dimethylamino group, a diethylamino group, a di(n-propyl) amino group, and a di(i-propyl) amino group.
In the formula (i-1), Z1 represents an alkyl group having 1 to 6 carbon atoms or an aryl group having 6 to 12 carbon atoms.
The alkyl group having 1 to 6 carbon atoms in Z1 may include a linear or branched alkyl group and a cycloalkyl group, and preferably means a linear or branched alkyl group. Specific examples thereof include a methyl group, an ethyl group, a n-propyl group, a n-butyl group, a n-pentyl group, a n-hexyl group, an i-propyl group, an i-butyl group, a sec-butyl group, a tert-butyl group, a cyclopentyl group, and a cyclohexyl group. A methyl group, an ethyl group, a n-propyl group, a n-butyl group, an i-propyl group, an i-butyl group, a sec-butyl group, and a cyclohexyl group are preferable, an ethyl group, a n-propyl group, a n-butyl group, an i-propyl group, an i-butyl group, a sec-butyl group, and a cyclohexyl group are more preferable, an ethyl group, a n-butyl group, and an i-propyl group are still more preferable, and an i-propyl group is particularly preferable.
Examples of the aryl group having 6 to 12 carbon atoms in Z1 include a phenyl group and a phenyl group having one or more substituents. Examples of the substituent of the phenyl group having a substituent include an alkyl group having 1 to 6 carbon atoms and an alkoxy group having 1 to 6 carbon atoms.
In the formula (i-1), a represents an integer represented by 2-b, and b represents an integer of 0 to 2. Preferably, b is 2 (a is 0).
When a is 2, two Y1s may be the same group or different groups. From the viewpoint of synthesis, they are preferably the same.
When b is 2, two Z1s may be the same group or different groups. From the viewpoint of synthesis, they are preferably the same.
In the formula (i-1), A1 represents an organic group having 1 to 20 carbon atoms. Specific examples thereof include an alkylene group having 1 to 20 carbon atoms, an arylene group having 1 to 20 carbon atoms, an alkylene group having 1 to 20 carbon atoms which may optionally contain any one or more of an imino group, an oxy group, and a thio group, and an arylene group having 1 to 20 carbon atoms which may optionally contain any one or more of an imino group, an oxy group, and a thio group.
In the formula (i-1), B1 represents any one selected from the group consisting of a hydrogen atom, an alkyl group having 1 to 6 carbon atoms, and an aryl group having 6 to 12 carbon atoms.
Specific examples and preferable examples of the alkyl group having 1 to 6 carbon atoms in B1 may include those similar to the alkyl group having 1 to 6 carbon atoms in Z1.
Specific examples and preferable examples of the aryl group having 6 to 12 carbon atoms in B1 may include those similar to the aryl group having 6 to 12 carbon atoms in Z1.
In the formula (ii-1), P1 represents a bond with the inorganic porous substrate, and the bond with the inorganic porous substrate in P1 is the same as the bond with the inorganic porous substrate in X1.
In the formula (ii-1), Q1 and R1 each independently represents any one selected from the group consisting of the bond with the inorganic porous substrate, a hydroxyl group, an amino group, an alkoxy group having 1 to 6 carbon atoms, and an alkylamino group having 1 to 6 carbon atoms. Here, the bond with the inorganic porous substrate in Q1 and R1 is the same as in X1 above. The alkoxy group having 1 to 6 carbon atoms and the alkylamino group having 1 to 6 carbon atoms in

Q1 and R1 are as defined in Y1 above.

J1 represents an alkyl group having 2 to 20 carbon atoms or an aryl group having 7 to 20 carbon atoms. The alkyl group having 2 to 20 carbon atoms in J1 is preferably an alkyl group having 2 to 6 carbon atoms, and specific examples thereof include an ethyl group, a n-propyl group, a n-butyl group, a n-pentyl group, a n-hexyl group, an i-propyl group, an i-butyl group, a sec-butyl group, a tert-butyl group, a cyclopentyl group, and a cyclohexyl group. The alkyl group having 2 to 20 carbon atoms in J1 may have one or more substituents. Examples of the substituent include a fluoro group, a chloro group, a bromo group, a cyano group, an aryl group having 6 to 10 carbon atoms, an alkoxy group having 1 to 6 carbon atoms, an aryloxy group having 6 to 10 carbon atoms, an acyloxy group having 1 to 10 carbon atoms, an acyl group having 1 to 10 carbon atoms, an alkylcarbamoyl group having 1 to 6 carbon atoms, and an arylcarbamoyl group having 6 to 10 carbon atoms.

The aryl group having 7 to 20 carbon atoms in J1 is preferably an aryl group having 7 to 10 carbon atoms, and specific examples thereof include a phenyl group having at least one alkyl having 1 to 6 carbon atoms and a phenyl group having one or more other substituents. Examples of other substituents of the phenyl group having a substituent include a fluoro group, a chloro group, a bromo group, a cyano group, an alkoxy group having 1 to 6 carbon atoms, an aryloxy group having 6 to 10 carbon atoms, an acyloxy group having 1 to 10 carbon atoms, an acyl group having 1 to 10 carbon atoms, an alkylcarbamoyl group having 1 to 6 carbon atoms, and an arylcarbamoyl group having 6 to 10 carbon atoms.

In the formula (ii-2), P1 represents the bond with the inorganic porous substrate, and the bond with the inorganic porous substrate in P1 is the same as the definition of P1 in the above X1 and the formula (ii-1).

Q1 represents any one selected from the group consisting of the bond with the inorganic porous substrate, a hydroxyl group, an amino group, an alkoxy group having 1 to 6 carbon atoms, and an alkylamino group having 1 to 12 carbon atoms, and the alkoxy group having 1 to 6 carbon atoms and the alkylamino group having 1 to 12 carbon atoms are each as defined in the formula (ii-1).

In the formula (ii-2), K1 represents an alkyl group having 1 to 20 carbon atoms or an aryl group having 6 to 20 carbon atoms. The alkyl group having 1 to 20 carbon atoms is preferably an alkyl group having 1 to 6 carbon atoms, and specific examples thereof include a methyl group, an ethyl group, a n-propyl group, a n-butyl group, a n-pentyl group, a n-hexyl group, an i-propyl group, an i-butyl group, a sec-butyl group, a tert-butyl group, a cyclopentyl group, and a cyclohexyl group. The alkyl group having 1 to 20 carbon atoms in K1 may have one or more substituents. Examples of the substituent include a fluoro group, a chloro group, a bromo group, a cyano group, an aryl group having 6 to 10 carbon atoms, an alkoxy group having 1 to 6 carbon atoms, an aryloxy group having 6 to 10 carbon atoms, an acyloxy group having 1 to 10 carbon atoms, an acyl group having 1 to 10 carbon atoms, an alkylcarbamoyl group having 1 to 6 carbon atoms, and an arylcarbamoyl group having 6 to 10 carbon atoms. The aryl group having 6 to 20 carbon atoms is preferably an aryl group having 6 to 10 carbon atoms, and specific examples thereof include a phenyl group and a phenyl group having one or more substituents. Examples of the substituent of the phenyl group having a substituent include a fluoro group, a chloro group, a bromo group, a cyano group, an alkoxy group having 1 to 6 carbon atoms, an aryloxy group having 6 to 10 carbon atoms, an acyloxy group having 1 to 10 carbon atoms, an acyl group having 1 to 10 carbon atoms, an alkylcarbamoyl group having 1 to 6 carbon atoms, and an arylcarbamoyl group having 6 to 10 carbon atoms.

In the formula (ii-3), P1 represents the bond with the inorganic porous substrate, and the bond with the inorganic porous substrate in P1 is the same as the definition of P1 in the above X1 and the formula (ii-1) or (ii-2).

K1 represents an alkyl group having 1 to 20 carbon atoms or an aryl group having 6 to 20 carbon atoms, and is the same as each definition of the formula (ii-2).

In the formula (ii-3), M1 represents a divalent alkylene group having 1 to 6 carbon atoms. The alkylene group having 1 to 6 carbon atoms in M1 is preferably an alkylene group having 1 to 4 carbon atoms, and specific examples thereof include a methylene group, an ethylene group, an n-propylene group, and an n-butylene group. More preferable examples include a methylene group and an ethylene group.

In the formula (ii-3), N1 represents an alkyl group having 2 to 20 carbon atoms or an aryl group having 6 to 20 carbon atoms.

The alkyl group having 2 to 20 carbon atoms in N1 is preferably an alkyl group having 2 to 10 carbon atoms, more preferably an alkyl group having 2 to 6 carbon atoms, and specifically an ethyl group, a propyl group, a butyl group, a pentyl group, or a hexyl group. The alkyl group having 2 to 20 carbon atoms in N1 may have one or more substituents. Examples of the substituent include a fluoro group, a chloro group, a bromo group, a cyano group, an aryl group having 6 to 10 carbon atoms, an alkoxy group having 1 to 6 carbon atoms, an aryloxy group having 6 to 10 carbon atoms, an acyloxy group having 1 to 10 carbon atoms, an acyl group having 1 to 10 carbon atoms, an alkylcarbamoyl group having 1 to 6 carbon atoms, and an arylcarbamoyl group having 6 to 10 carbon atoms.

The aryl group having 6 to 20 carbon atoms in N1 is preferably an aryl group having 6 to 10 carbon atoms. Specific examples thereof include a phenyl group and a phenyl group having one or more substituents. Examples of the substituent of the phenyl group having a substituent include a fluoro group, a chloro group, a bromo group, a cyano group, an alkoxy group having 1 to 6 carbon atoms, an aryloxy group having 6 to 10 carbon atoms, an acyloxy group having 1 to 10 carbon atoms, an acyl group having 1 to 10 carbon atoms, an alkylcarbamoyl group having 1 to 6 carbon atoms, and an

arylcarbamoyl group having 6 to 10 carbon atoms.

The inorganic porous substrate in the present invention has a pore diameter of 20 nm or more from the viewpoint of application to nucleic acid synthesis. The inorganic porous substrate to be used can be appropriately selected according to a strand length of the nucleic acid to be synthesized. Usually, when the strand length of the nucleic acid to be synthesized is long, it is preferable to select the inorganic porous substrate having a large pore diameter. The pore diameter is preferably 20 nm or more and 500 nm or less, more preferably 25 nm or more and 500 nm or less, still more preferably 30 nm or more and 500 nm or less, even more preferably 40 nm or more and 500 nm or less, and particularly preferably 40 nm or more and 300 nm or less.

In this case, the pore diameter can be determined from the pore diameter (mode diameter) obtained from the value of the X axis at a maximal value of the Y axis in a pore diameter distribution graph (plotted using the value of the pore diameter on the X-axis and on the Y-axis a value obtained by differentiating a pore volume with the pore diameter) obtained by analyzing an inorganic molded body, which is a raw material of the inorganic porous substrate, by a mercury intrusion method.

The particle diameter of the inorganic porous substrate of the present invention is characterized by being 1 μm or more from the viewpoint of preventing clogging of piping, and the like when the inorganic porous substrate is used in a nucleic acid synthesizer after being guided to an inorganic porous support described later. The particle diameter is preferably 5 μm or more, more preferably 10 μm or more, still more preferably 20 μm or more, and particularly preferably 30 μm or more. The particle diameter can be controlled by sieving the inorganic molded body or the inorganic porous substrate, which is a raw material, with a sieve having an opening equal to or larger than the corresponding particle diameter to remove fine components.

The particle diameter of the inorganic porous substrate of the present invention can be determined as an average value of major diameters of arbitrary 50 particles in observation of the inorganic molded body as a raw material in a visual field of 200 magnification with a scanning electron microscope.

From the viewpoint of application to nucleic acid synthesis as described later, the inorganic porous substrate according to the present invention is characterized by having a cumulative pore volume in the pore diameter range of 40 nm to 1000 nm of more than 0.32 mL/g and 4 mL/g or less. The cumulative pore volume in the same range is preferably more than 0.35 mL/g and 3.5 mL/g or less, more preferably more than 0.4 mL/g and 3 mL/g or less, still more preferably more than 0.43 mL/g and 3 mL/g or less, and even more preferably more than 0.45 mL/g and 2.5 mL/g or less. In this case, the cumulative pore volume in the pore diameter range of 40 nm to 1000 nm can be determined by analyzing an inorganic molded body described later, which is a raw material, by a mercury intrusion method.

Hereinafter, a method of preparing the inorganic porous substrate of the present invention will be described. The inorganic porous substrate is produced by reacting an inorganic molded body as a starting material with a specific silane coupling agent, and introducing a silyl group (A) and a silyl group (B) each having an active NH group.

In this case, the active NH group represents an NH group having a structure of the following formula (NH-1).

[Chemical Formula 18]

Si-A1-NH-B1 ⋯ (NH-1)

[In the formula (NH-1),

A1 represents an organic group having 1 to 20 carbon atoms,
B1 represents any one selected from the group consisting of a hydrogen atom, an alkyl group having 1 to 6 carbon atoms, and an aryl group having 6 to 12 carbon atoms, and
an NH group satisfying the requirement of the above structure is an active NH group.].

Specific examples and preferable examples of A1 and B1 in the formula (NH-1) may include the same as A1 and B1 in the formula (i-1), respectively.

[Method of producing inorganic molded body]

The inorganic molded body which is a raw material of the inorganic porous substrate of the present embodiment is an inorganic molded body having a pore distribution having a pore diameter of 20 nm or more and having a hydroxyl group capable of supporting a silyl group using a silane coupling agent. Typical examples of such an inorganic molded body include those containing a silanol group, and examples of the constituent material include silica, silica gel, zeolite, glass, and quartz. The inorganic molded body is preferably silica, silica gel, zeolite, or glass. As these inorganic molded bodies, commercially available products may be used, or products prepared by the following synthetic method may be used.

Examples of a method of producing an inorganic molded body containing a silanol group include a dry method

and a wet method. Specific examples of the former include a combustion method and an arc method, and specific examples of the latter include synthesis methods such as a sedimentation method, a sol-gel method, and a hydrothermal synthesis method (reference: TOSOH Research & Technology Review Vol. 45 (2001)). The preparation of such an inorganic porous body is carried out by, for example, using silicate, alkoxysilane, chlorosilanes or the like as raw materials according to the synthesis method as described above using a solvent and a template.

The imparting of the porous structure to the inorganic molded body can be carried out, for example, by using the following method: 1. a method of precipitating silica, and then removing a solvent contained in a framework of the silica; 2. a method of precipitating a solid after mixing silica with dissimilar metal other than silica such as aluminum, boron, or the like, then phase-separating the resulting mixture into a silica component and a component other than silica, and removing the component other than silica; 3. a method of precipitating silica after mixing silica with an ammonium salt or a polymer as a template agent, and then removing the template agent; or 4. a method of aggregating a precipitated silica. A combination of two or more of the above methods may be used.

The methods of removing the solvent or the template agent in the above methods may include drying, supercritical extraction, calcination or the like.

The resulting inorganic molded body is preferably in a form of particles, and may be formed into a spherical shape, or may be formed into a massive shape or a crushed shape, whereas, when they are used as supports, the spherical shape or the crushed shape is preferable from the viewpoint of filling into a column for nucleic acid synthesis. The molding method is not particularly limited, but a spray drying method or an emulsion method may be used. Grinding, sieving, and the like may be appropriately performed.

[Method of producing inorganic porous substrate]

The inorganic porous substrate is produced by a method in which the inorganic molded body is reacted with a silane coupling agent containing a structure of the silyl group (A) and a silane coupling agent containing a structure of the silyl group (B). As a result, these silane coupling agents react with a hydroxyl groups on a surface of the inorganic molded body, and the silyl group (A) and the silyl group (B) are introduced. In the same reaction, a solvent may be appropriately used in combination.

In the reaction of the inorganic molded body with the silane coupling agent containing the structure of the silyl group (A) and the silane coupling agent containing the structure of the silyl group (B), the inorganic molded body may be reacted with the silane coupling agent containing the structure of the silyl group (A) and then reacted with the silane coupling agent containing the structure of the silyl group (B), the inorganic molded body may be reacted with the silane coupling agent containing the structure of the silyl group (B) and then reacted with the silane coupling agent containing the structure of the silyl group (A), or the inorganic molded body, the silane coupling agent containing the structure of the silyl group (A), and the silane coupling agent containing the structure of the silyl group (B) may be reacted simultaneously.

As the silane coupling agent that has the structure of the silyl group (A) and imparts the silyl group (A) to the inorganic molded body, a silane coupling agent represented by the following formula (i-1a) or (i-1b) can be used.
[Chemical Formula 19]

$$(Y0)_h(Z1)_b\text{Si-A1-NH-B1} \cdots \qquad \text{(i-1a)}$$

[In the formula (i-1a),

Y0 each independently represents any one selected from the group consisting of a hydroxyl group, an amino group, an alkoxy group having 1 to 6 carbon atoms, and an alkylamino group having 1 to 12 carbon atoms,
h represents an integer represented by 3-b,
b represents an integer of 0 to 2, and
Z1, A1, and B1 represent groups similar to Z1, A1, and B1 in the formula (i-1), respectively],

[Chemical Formula 20]

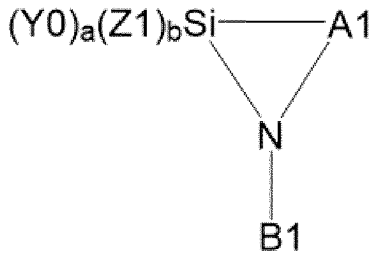

· · · (i-1b)

[In the formula (i-1b),

Y0 each independently represents any one selected from the group consisting of a hydroxyl group, an amino group, an alkoxy group having 1 to 6 carbon atoms, and an alkylamino group having 1 to 12 carbon atoms,
a represents an integer represented by 2-b,
b represents an integer of 0 to 2, and
Z1, A1, and B1 represent groups similar to Z1, A1, and B1 in the formula (i-1), respectively.]

Specific examples and preferable examples of the alkoxy group having 1 to 6 carbon atoms and the alkylamino group having 1 to 12 carbon atoms in Y0 in the formula (i-1a) and Y0 in the formula (i-1b) can be the same as specific examples and preferable examples of the alkoxy group having 1 to 6 carbon atoms and the alkylamino group having 1 to 12 carbon atoms in Y1 in the formula (i-1).

As the silane coupling agent that has the structure of the silyl group (B) and imparts the silyl group (B) to the inorganic molded body, a silane coupling agent represented by the following formulas (ii-1a), (ii-2a), and (ii-3a) can be used.

[Chemical Formula 21]

(Q0)(R0)(S0)Si-J1 ··· (ii-1a),

[Chemical Formula 22]

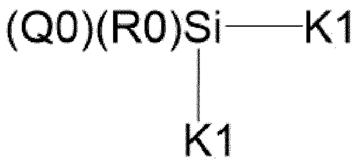

· · · (ii-2a)

,

[Chemical Formula 23]

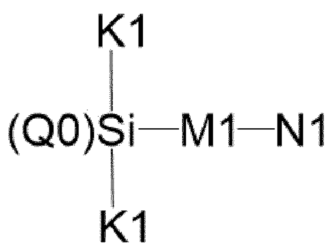

· · · (ii-3a)

[In the formulas (ii-1a), (ii-2a), and (ii-3a),

Q0, R0, and S0 each independently represents any one selected from the group consisting of a hydroxyl group, an amino group, a chloro group, a bromo group, an iodo group, an alkoxy group having 1 to 6 carbon atoms, and an alkylamino group having 1 to 12 carbon atoms, and
J1, K1, M1, and N1 represent groups similar to J1, K1, M1, and N1 in the formulas (ii-1), (ii-2), and (ii-3).]

Specific examples and preferable examples of the alkoxy group having 1 to 6 carbon atoms and the alkylamino group having 1 to 12 carbon atoms in Q0, R0, and S0 in the formulas (ii-1a), (ii-2a), and (ii-3a) can be the same as specific examples and preferable examples of the alkoxy group having 1 to 6 carbon atoms and the alkylamino group having 1 to 12 carbon atoms in Q1 and R1 in the formulas (ii-1), (ii-2), and (ii-3).

In the reaction of the inorganic molded body and the silane coupling agent, after the inorganic molded body may be reacted with the silane coupling agent represented by the formula (i-1a) or (i-1b), the silane coupling agent represented by the formula (ii-1a), (ii-2a), or (ii-3a) may be reacted, or after the inorganic molded body may be reacted with the silane coupling agent represented by the formula (ii-1a), (ii-2a), or (ii-3a), the silane coupling agent represented by the formula (i-1a) or (i-1b) may be reacted, or the inorganic molded body may be simultaneously reacted with the silane coupling agent represented by the formula (i-1a) or (i-1b) and the silane coupling agent represented by the formula (ii-1a), (ii-2a), or (ii-3a).

Specific examples of the silane coupling agent represented by the formula (i-1a) or (i-1b) include the following.

[Chemical Formula 24]

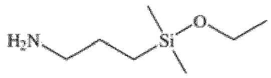

(i-1a-A-1)

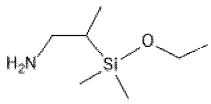

(i-1a-A-2)

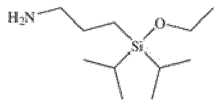

(i-1a-A-3)

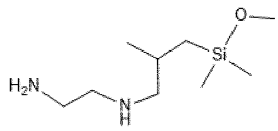

(i-1a-A-4)

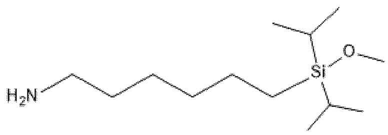

(i-1a-A-5)

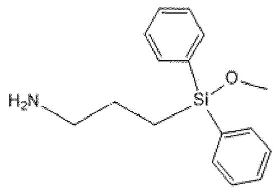

(i-1a-A-6)

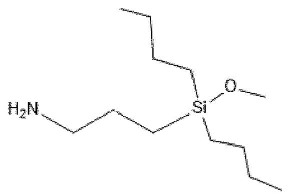

(i-1a-A-7)

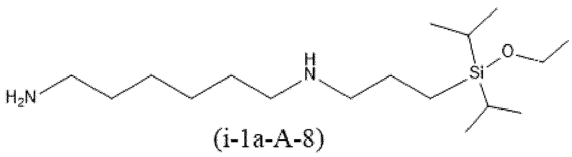

(i-1a-A-8)

[Chemical Formula 25]

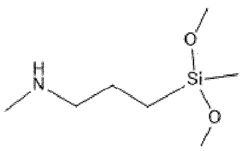

(i-1a-B-1)

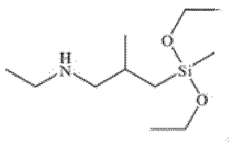

(i-1a-B-2)

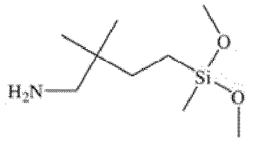

(i-1a-B-3)

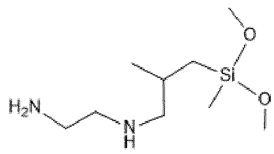

(i-1a-B-4)

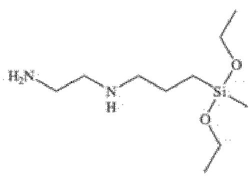

(i-1a-B-5)

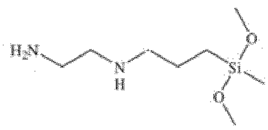

(i-1a-B-6)

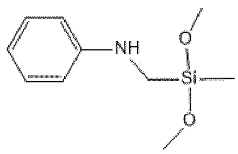

(i-1a-B-7)

[Chemical Formula 26]

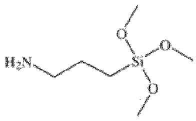
(i-1a-C-1)

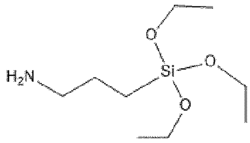
(i-1a-C-2)

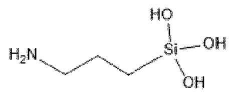
(i-1a-C-3)

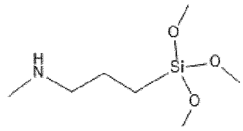
(i-1a-C-4)

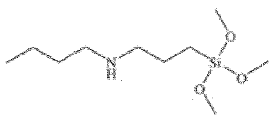
(i-1a-C-5)

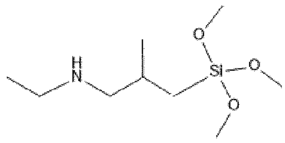
(i-1a-C-6)

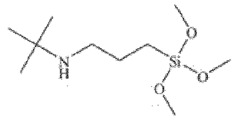
(i-1a-C-7)

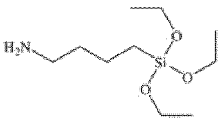
(i-1a-C-8)

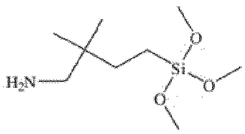
(i-1a-C-9)

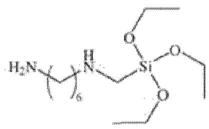
(i-1a-C-10)

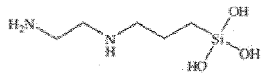
(i-1a-C-11)

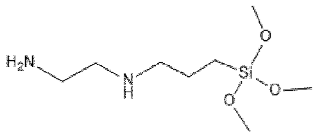
(i-1a-C-12)

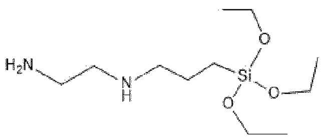
(i-1a-C-13)

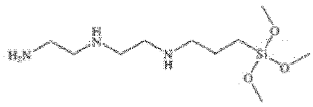
(i-1a-C-14)

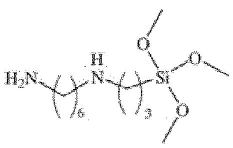
(i-1a-C-15)

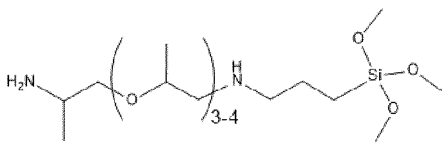
(i-1a-C-16)

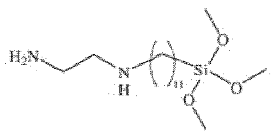
(i-1a-C-17)

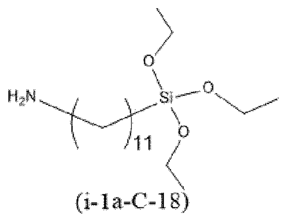
(i-1a-C-18)

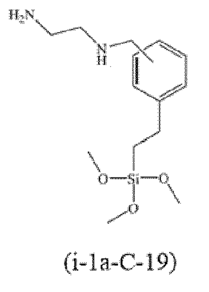
(i-1a-C-19)

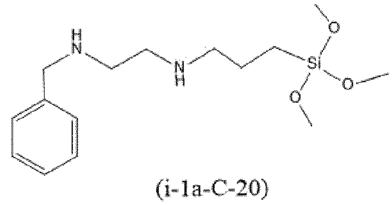
(i-1a-C-20)

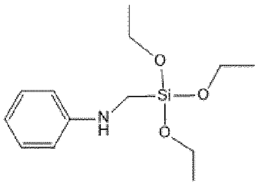
(i-1a-C-21)

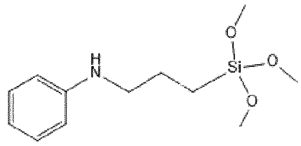
(i-1a-C-22)

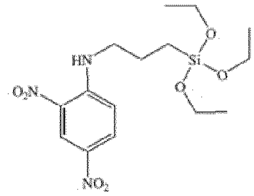
(i-1a-C-23)

[Chemical Formula 27]

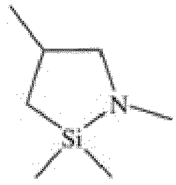

(i-1b-A-1)

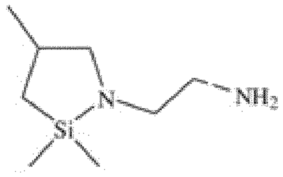

(i-1b-A-2)

[Chemical Formula 28]

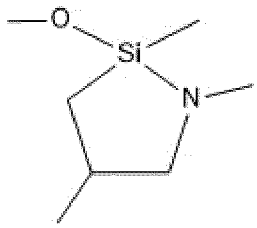

(i-1b-B-1)

[Chemical Formula 29]

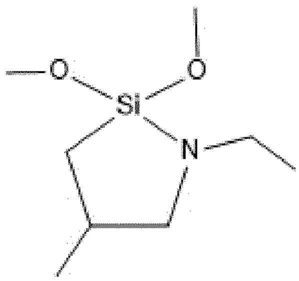

(i-1b-C-1)

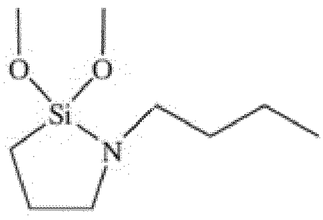

(i-1b-C-2)

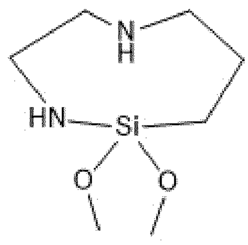

(i-1b-C-3)

Specific examples of the silane coupling agent represented by the formula (ii-1a) include the following.

[Chemical Formula 30]

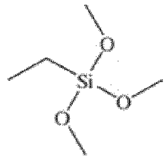
(ii-1a-A-1)

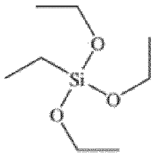
(ii-1a-A-2)

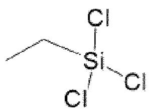
(ii-1a-A-3)

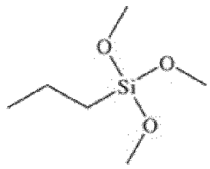
(ii-1a-A-4)

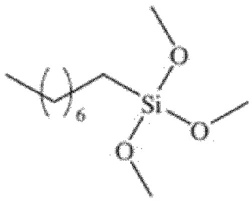
(ii-1a-A-5)

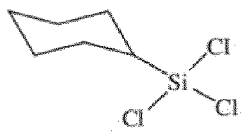
(ii-1a-A-6)

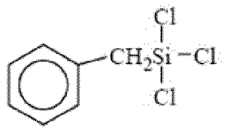
(ii-1a-A-7)

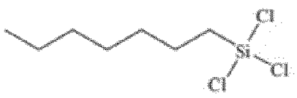
(ii-1a-A-8)

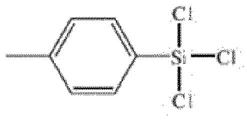
(ii-1a-A-9)

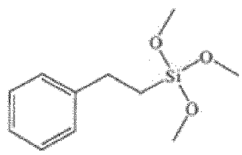
(ii-1a-A-10)

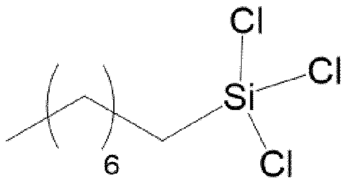
(ii-1a-A-11)

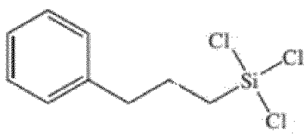
(ii-1a-A-12)

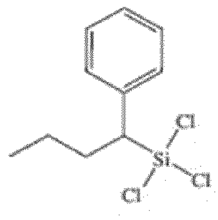
(ii-1a-A-13)

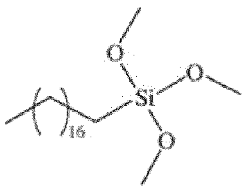
(ii-1a-A-14)

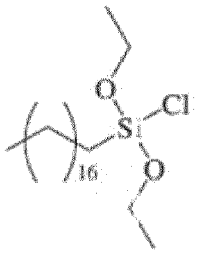
(ii-1a-A-15)

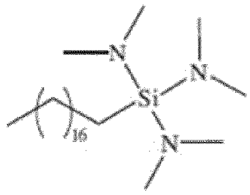
(ii-1a-A-16)

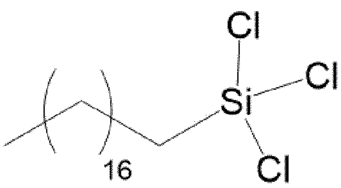
(ii-1a-A-17)

Specific examples of the silane coupling agent represented by the formula (ii-2a) include the following.

[Chemical Formula 31]

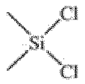
(ii-2a-A-1)

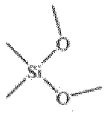
(ii-2a-A-2)

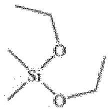
(ii-2a-A-3)

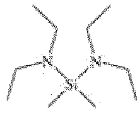
(ii-2a-A-4)

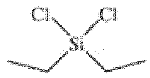
(ii-2a-A-5)

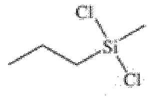
(ii-2a-A-6)

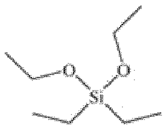
(ii-2a-A-7)

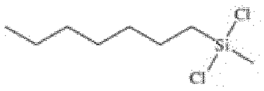
(ii-2a-A-8)

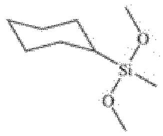
(ii-2a-A-9)

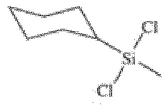
(ii-2a-A-10)

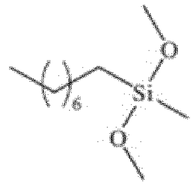
(ii-2a-A-11)

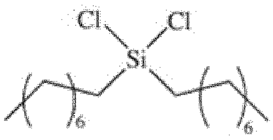
(ii-2a-A-12)

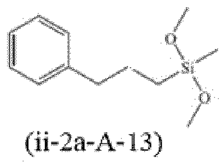
(ii-2a-A-13)

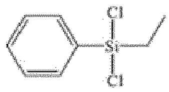
(ii-2a-A-14)

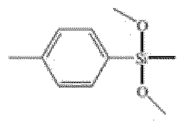
(ii-2a-A-15)

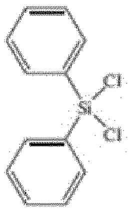
(ii-2a-A-16)

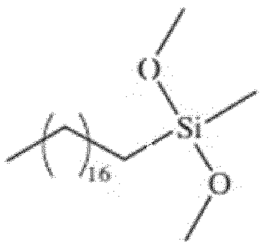
(ii-2a-A-17)

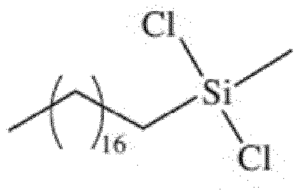
(ii-2a-A-18)

Specific examples of the silane coupling agent represented by the formula (ii-3a) include the following.

[Chemical Formula 32]

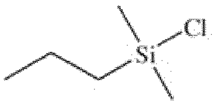

(ii-3a-A-1)

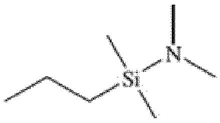

(ii-3a-A-2)

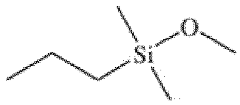

(ii-3a-A-3)

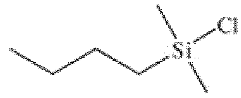

(ii-3a-A-4)

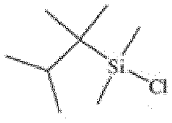

(ii-3a-A-5)

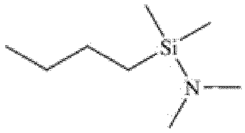

(ii-3a-A-6)

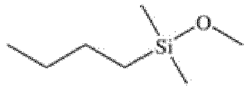

(ii-3a-A-7)

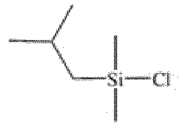

(ii-3a-A-8)

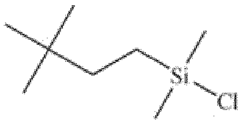

(ii-3a-A-9)

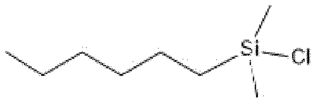

(ii-3a-A-10)

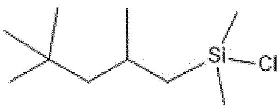

(ii-3a-A-11)

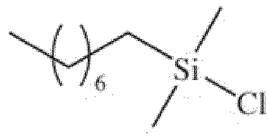

(ii-3a-A-12)

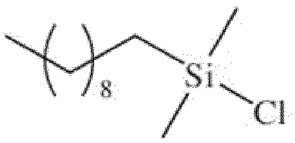

(ii-3a-A-13)

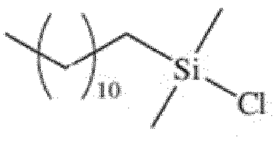

(ii-3a-A-14)

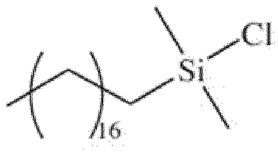

(ii-3a-A-15)

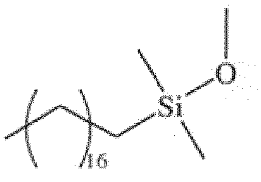

(ii-3a-A-16)

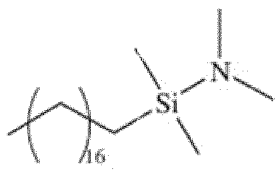

(ii-3a-A-17)

[Chemical Formula 33]

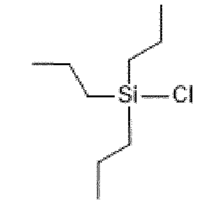
(ii-3a-A-18)

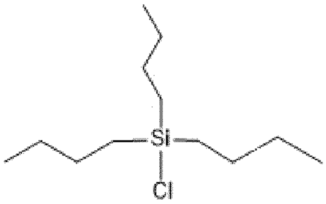
(ii-3a-A-19)

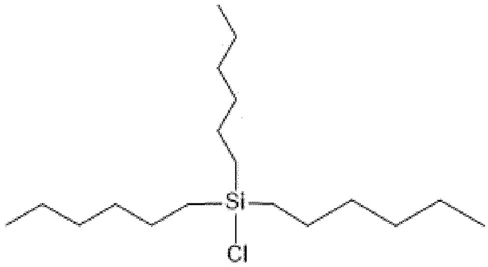
(ii-3a-A-20)

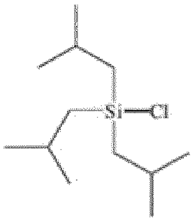
(ii-3a-A-21)

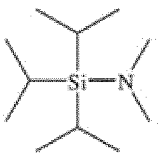
(ii-3a-A-22)

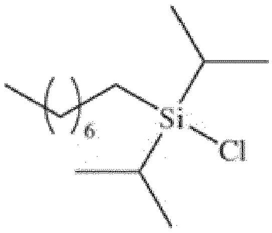
(ii-3a-A-23)

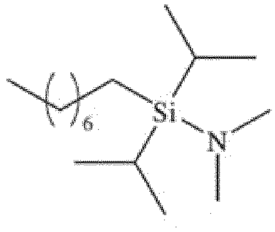
(ii-3a-A-24)

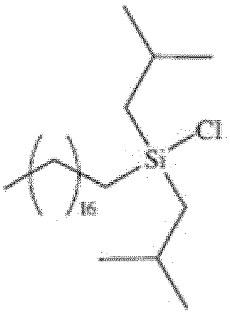
(ii-3a-A-25)

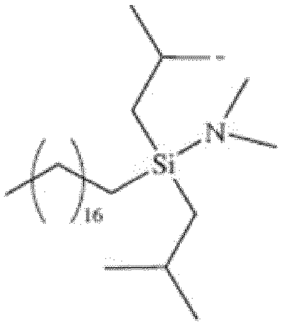
(ii-3a-A-26)

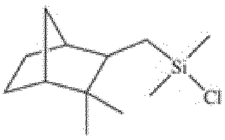
(ii-3a-A-27)

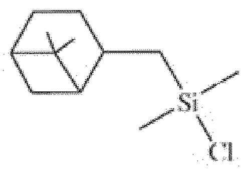
(ii-3a-A-28)

[Chemical Formula 34]

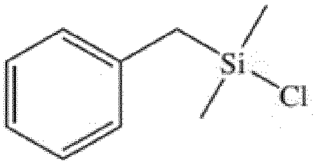

(ii-3a-A-29)

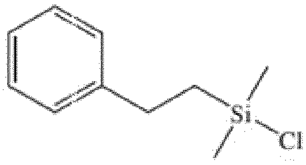

(ii-3a-A-30)

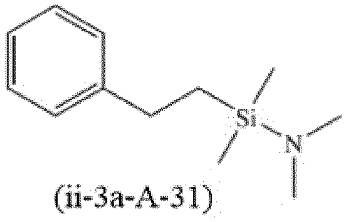

(ii-3a-A-31)

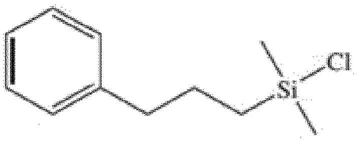

(ii-3a-A-32)

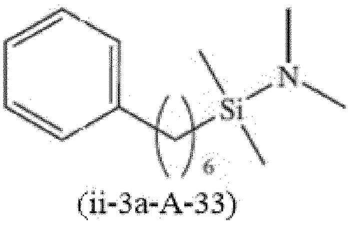

(ii-3a-A-33)

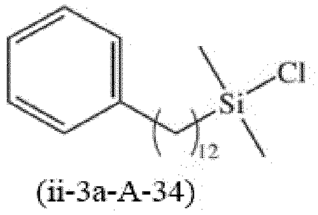

(ii-3a-A-34)

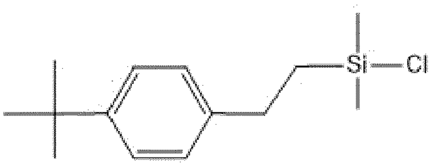

(ii-3a-A-35)

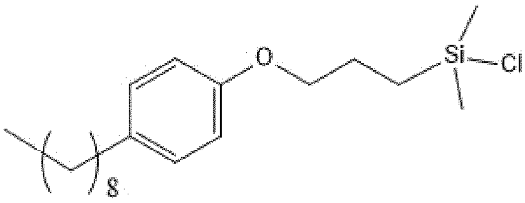

(ii-3a-A-36)

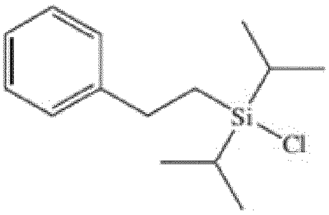

(ii-3a-A-37)

[Chemical Formula 35]

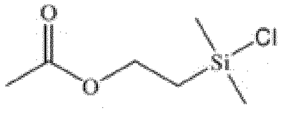

(ii-3a-A-38)

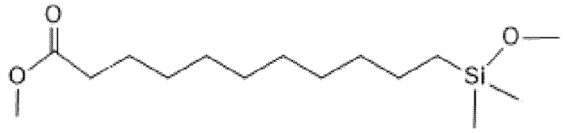

(ii-3a-A-39)

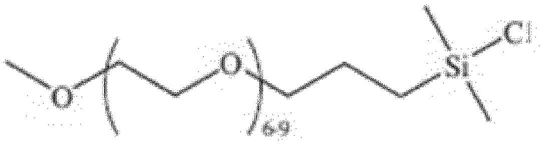

(ii-3a-A-40)

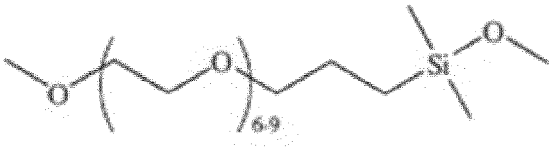

(ii-3a-A-41)

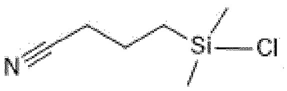

(ii-3a-A-42)

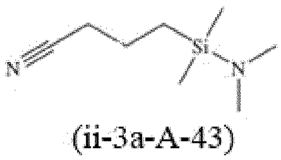

(ii-3a-A-43)

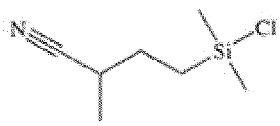

(ii-3a-A-44)

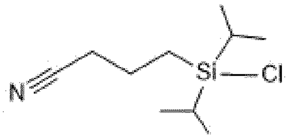

(ii-3a-A-45)

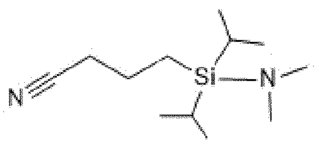

(ii-3a-A-46)

[Chemical Formula 36]

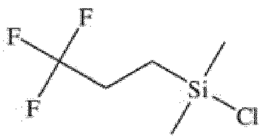

(ii-3a-A-47)

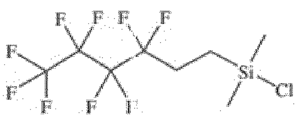

(ii-3a-A-48)

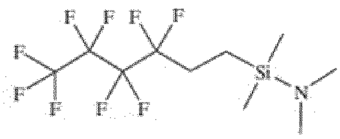

(ii-3a-A-49)

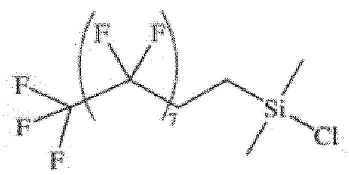

(ii-3a-A-50)

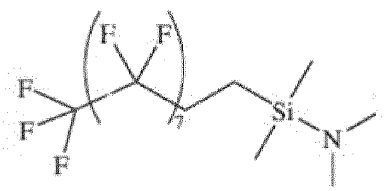

(ii-3a-A-51)

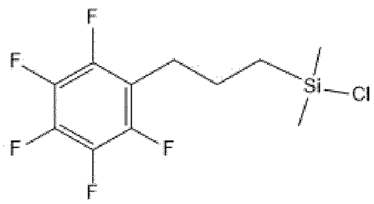

(ii-3a-A-52)

An addition amount of the silane coupling agent represented by the formula (i-1a) or (i-1b) is an amount at which an active NH group amount is 0.05 to 60 μmol/$m^2$, preferably 0.05 to 6.0 μmol/$m^2$, with respect to the specific surface area

per mass of the inorganic molded body obtained by nitrogen adsorption/desorption measurement. As the specific surface area per mass of the inorganic molded body, a value of the specific surface area determined from an average gradient in a range of $\alpha s$ = 1.7 to 2.1 according to an as-plot method by performing nitrogen adsorption/desorption isotherm measurement on the inorganic molded body as a raw material can be applied.

The addition amount of the silane coupling agent containing the structure of the silyl group (B) is not particularly limited, and in the case of reacting the inorganic molded body with the silane coupling agent containing the structure of the silyl group (B) and then reacting the inorganic molded body with the silane coupling agent containing the structure of the silyl group (A), and in the case of simultaneously reacting the inorganic molded body, the silane coupling agent containing the structure of the silyl group (A), and the silane coupling agent containing the structure of the silyl group (B), it is desirable to appropriately adjust the addition amount of the silane coupling agent containing the structure of the silyl group (B) so that an introduction amount of the silyl group (A) is not significantly inhibited.

The solvent that may be used when the inorganic molded body is reacted with the silane coupling agent containing the structure of the silyl group (A) and the silane coupling agent containing the structure of the silyl group (B) is not particularly limited, and pentane, hexane, heptane, toluene, xylene, mesitylene, 1,2,3,4-tetrahydronaphthalene, anisole, chlorobenzene, dichloromethane, tetrahydrofuran, acetonitrile, 2-heptanone, propylene glycol monomethyl ether acetate, N,N-dimethylformamide, water, or the like, or a mixture of two or more thereof can be used. Among them, pentane, hexane, heptane, toluene, xylene, mesitylene, and 1,2,3,4-tetrahydronaphthalene are preferable, and toluene, xylene, and mesitylene are more preferable.

When the silane coupling agent represented by the formula (ii-1a), (ii-2a), or (ii-3a) contains a chloro group, a bromo group, and an iodo group in any of Q0, R0, and S0, a nitrogen-containing organic base may be used in combination in the reaction of the inorganic molded body with the silane coupling agent represented by the formula (ii-1a), (ii-2a), or (ii-3a). Specific examples of the nitrogen-containing organic base include nitrogen-containing aromatic heterocyclic compounds such as 1-methylimidazole, 2,6-lutidine, pyridine, and 4-dimethylaminopyridine, and tertiary alkylamines such as N,N-diisopropylethylamine and triethylamine.

The inorganic molded body and the solvent are preferably dehydrated and used from the viewpoint of suppressing polymerization between the silane coupling agents and accelerating the reaction between the silane coupling agent and the surface of the inorganic molded body. The dehydration method is not particularly limited, and examples thereof include a method of heating the inorganic molded body under normal pressure or reduced pressure, and a method of dispersing the inorganic molded body in a solvent, and then distilling off the solvent under normal pressure or reduced pressure to perform azeotropic dehydration.

In the reaction between the inorganic molded body and the silane coupling agent, heating may be performed for accelerating the reaction, the present invention is not limited thereto, and the reaction may be performed at room temperature, or cooling may be performed to room temperature or less. The same applies when two or more solvents are used in combination.

Although the reaction is usually carried out for about 1 to 12 hours, the reaction time may be appropriately lengthened or shortened.

After the above reaction, washing is performed with a solvent such as tetrahydrofuran or ethanol, and drying is performed to obtain an inorganic porous substrate.

As one of preferable examples of the inorganic porous substrate of the present invention, the inorganic porous substrate has a specific surface area of 0.1 $m^2/g$ or more and 200 $m^2/g$ or less from the viewpoint of being used for nucleic acid synthesis. As the specific surface area, the value of the specific surface area determined from the average gradient in the range of $\alpha s$ = 1.7 to 2.1 according to the as-plot method by performing nitrogen adsorption/desorption isotherm measurement on the inorganic molded body as a raw material can be applied. The specific surface area is preferably 1 $m^2/g$ or more and 150 $m^2/g$ or less, more preferably 5 $m^2/g$ or more and 100 $m^2/g$ or less, still more preferably 8 $m^2/g$ or more and 80 $m^2/g$ or less, and particularly preferably 10 $m^2/g$ or more and 60 $m^2/g$ or less.

The specific surface area of the inorganic porous substrate is adjusted by the specific surface area of the inorganic molded body which is a raw material of the inorganic porous substrate, and it is desirable to use the inorganic molded body within the above range as a raw material.

As an inorganic material used for the inorganic porous substrate, various inorganic materials can be used, and an inorganic material containing a silicon oxide with a silanol group is preferable. Specifically, inorganic materials containing silica, silica gel, zeolite, or glass are preferable, and it is preferable to use an inorganic molded body containing such an inorganic material as a raw material.

One of preferred embodiments of the present invention is an embodiment in which a loading of the active NH group contained in the inorganic porous substrate satisfies the following formula (NH#1).

[Math. 3]

$$0.05 \leq I1/S1 \leq 6.0 \quad \cdots \quad (NH\#1)$$

I1: active NH group amount (μmol/g) in inorganic porous substrate,
S1: specific surface area ($m^2$/g) of inorganic porous substrate obtained by nitrogen adsorption/desorption isotherm measurement

In the formula (NH#1), the loading (μmol/g) of the active NH group can be determined by the following method.
The loading of the active NH group in the inorganic porous substrate is measured by a 2-nitrobenzenesulfonic acid adsorption method. The "2-nitrobenzenesulfonic acid adsorption method" means a method of quantifying primary to tertiary amino groups based on an amount of 2-nitrobenzenesulfonic acid ionically bonded to the primary to tertiary amino groups.
The amount of the primary to tertiary amino groups by the 2-nitrobenzenesulfonic acid adsorption method can be measured as follows.
About 30 mg of the inorganic porous substrate is collected on a filter such as a Pasteur pipette stoppered with absorbent cotton, and 1 mL of a tetrahydrofuran (THF) solution (DIPEA 5% by volume) of N,N-diisopropylethylamine (DIPEA) is passed through to perform washing. Subsequently, 1 mL of a THF solution of 2-nitrobenzenesulfonic acid (50 mM of 2-nitrobenzenesulfonic acid) is passed four times, and then 1 mL of THF is passed through four times to perform washing. Next, a container such as a 10 mL volumetric flask is used as a receiver to pass a mixed solution obtained by mixing dilute aqueous ammonia (solution obtained by mixing 28% ammonia water and water at a volume ratio of 1 : 100) and acetonitrile at a volume ratio of 1 : 1. An aqueous solution of acetonitrile (acetonitrile 15% by volume) is added to an eluted solution received in the receiver to prepare 10 mL of the solution, and the solution is analyzed by high performance liquid chromatography (HPLC) to measure a peak area value of 2-nitrobenzenesulfonic acid. The HPLC analysis conditions are not particularly limited as long as the 2-nitrobenzenesulfonic acid can be measured, and the following analysis conditions are exemplified.

Example of HPLC analysis condition

Column: Scherzo SM-C18 (produced by Imtakt), 4.6 mmφ × 150 mm, 3 μm
Mobile phase: A solution 10 mM ammonium formate aqueous solution
B solution acetonitrile
Gradient condition: A/B = 85%/15% (constant)
Flow rate: 1.0 mL/min
Column temperature: 40°C
Detection wavelength: 210 nm
Injection volume: 10 μL

When the above peak area value of 2-nitrobenzenesulfonic acid is referred to as AR, a mass of the inorganic porous substrate used in the analysis is referred to as MA, a slope of a calibration line prepared by using the standard solution of 2-nitrobenzenesulfonic acid is referred to as a01, and an intercept of the calibration line is referred to as b01, the amount of primary to tertiary amino groups can be calculated as follows. In the following formula, "203.17" is a molecular weight of 2-nitrobenzenesulfonic acid ($C_6H_5NO_5S$).

«amount of primary to tertiary amino groups (μmol/g)» = (AR - b01)/(203.17 × MA × a01) × 10 [Math. 4]

Based on the calculated amount of the primary to tertiary amino groups, the active NH group amount can be derived as follows.

«active NH group amount (μmol/g)» = «primary to tertiary amino groups (μmol/g)» × «number of active NH groups in formula (i-1)»/«number of primary to tertiary amino groups in formula (i-1)» [Math. 5]

A preferable range of I1/S1 is 0.1 or more and 5.0 or less, a more preferable range is 0.2 or more and 3.0 or less, and a still more preferable range 0.3 or more and 2.0 or less.
The loading of the silyl group (B) introduced into the inorganic porous substrate can be determined by the following method. That is, the inorganic porous substrate and a deuterated solvent containing an alkali component such as sodium hydroxide are mixed, and then an internal standard such as 1,3,5-trioxane is added thereto and mixed. The resulting mixture is filtered, and the filtrate is subjected to $^1$H NMR measurement. A component derived from the released silyl group (B) contained in the filtrate is quantified from an integral ratio with the internal standard. The silyl group (B)

loading (μmol/g) can be determined by dividing the obtained quantitative value by a weight of the inorganic porous substrate used. One specific example is as follows.

A 3.60 wt% NaOD aq./dimethyl sulfoxide-$d^6$ mixed solution (3.60 wt% NaOD aq./dimethyl sulfoxide-$d^6$ = 1/3.5 to 2/1 weight ratio) is prepared. In a glass container, 60 to 200 mg of the inorganic porous substrate as a precursor is weighed, and 800 to 1200 mg of the prepared 3.60 wt% NaOD aq./dimethyl sulfoxide-$d^6$ mixed solution is added. The mixture is sonicated at 45°C for 2 hours. An aqueous 1,3,5-trioxane solution (30 mg) is added as an internal standard to the sonicated mixture, and the mixture is mixed. The resulting mixture is introduced into a filter such as a Pasteur pipette stoppered with glass wool, and a solid content is filtered off to obtain a filtrate. The obtained filtrate is subjected to $^1$H NMR measurement, and a component derived from the silyl group (B) -1 introduced by the silane coupling agent and released by the reaction with NaOD contained in the filtrate is quantified from the integral ratio with the internal standard. The loading (μmol/g) of the silyl group (B) can be determined by dividing the obtained quantitative value by a weight of the inorganic porous substrate used.

Furthermore, the silyl group (B) loading may be calculated as follows using the value of the specific surface area of the inorganic porous substrate defined below.

«silyl group (B) loading (μmol/$m^2$)» = «silyl group (B) loading (μmol/g)»/«specific surface area of inorganic porous substrate ($m^2$/g)» [Math. 6]

One of preferred embodiments of the present invention is an inorganic porous substrate having the silyl group represented by the formula (ii-3) as the silyl group (B). Among the silyl groups represented by the formula (ii-3), a silyl group represented by the following formula (ii-3-1) is preferable.

[Chemical Formula 37]

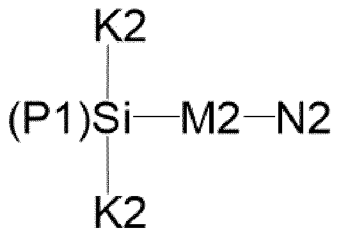

・・・(ii-3-1)

[In the formula (ii-3-1),

P1 represents a bond with the inorganic porous substrate,
K2 each independently represents an alkyl group having 1 to 6 carbon atoms or an aryl group having 6 to 10 carbon atoms,
M2 represents an alkylene group having 1 to 4 carbon atoms, and
N2 represents an alkyl group having 2 to 6 carbon atoms or an aryl group having 6 to 10 carbon atoms.]

The definition of P1 in the above formula is as described in the specification.

In the formula (ii-3-1), K2 each independently represents an alkyl group having 1 to 6 carbon atoms or an aryl group having 6 to 10 carbon atoms.

Specific examples and preferable examples of the alkyl group having 1 to 6 carbon atoms in K2 may include those similar to the alkyl group having 1 to 6 carbon atoms in K1.

Specific examples and preferable examples of the aryl group having 6 to 10 carbon atoms in K2 may include those similar to the aryl group having 6 to 10 carbon atoms in K1.

In the formula (ii-3-1), M2 represents an alkylene group having 1 to 4 carbon atoms, preferably an alkylene group having 1 to 2 carbon atoms, and specifically a methylene group and an ethylene group.

In the formula (ii-3-1), N2 represents an alkyl group having 2 to 6 carbon atoms or an aryl group having 6 to 10 carbon atoms.

Specific examples and preferable examples of the alkyl group having 2 to 6 carbon atoms in N2 may include those similar to the alkyl group having 2 to 6 carbon atoms in N1.

Specific examples and preferable examples of the aryl group having 6 to 10 carbon atoms in N2 may include those similar to the aryl group having 6 to 10 carbon atoms in N1.

Specific examples of the silyl group represented by the formula (ii-3-1) include the following formula (ii-3-1-A).

[Chemical Formula 38]

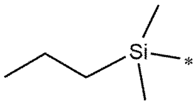

(ii-3-1-A-1)

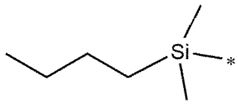

(ii-3-1-A-2)

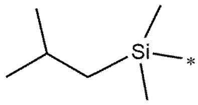

(ii-3-1-A-3)

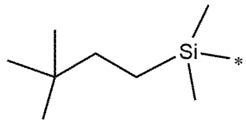

(ii-3-1-A-4)

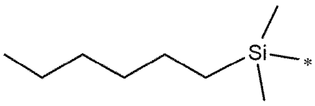

(ii-3-1-A-5)

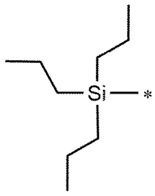

(ii-3-1-A-6)

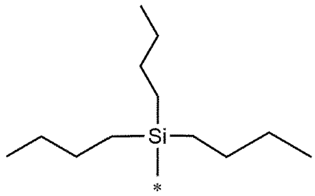

(ii-3-1-A-7)

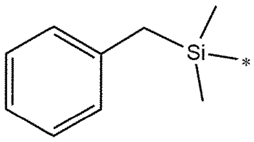

(ii-3-1-A-8)

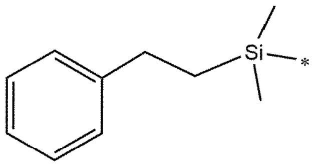

(ii-3-1-A-9)

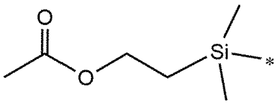

(ii-3-1-A-10)

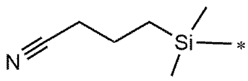

(ii-3-1-A-11)

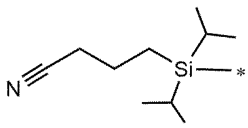

(ii-3-1-A-12)

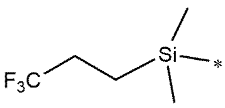

(ii-3-1-A-13)

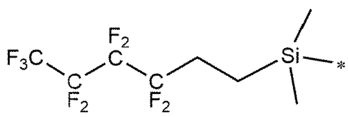

(ii-3-1-A-14)

・・・(ii－3－1－A)

In the formula (ii-3-1-A), * represents a bond with the inorganic porous substrate or the inorganic porous support.]

One of preferable examples of the inorganic porous substrate of the present invention is an inorganic porous substrate in which A1 in the formula (i-1) is an alkylene group having 1 to 20 carbon atoms which may optionally contain any one or more of an imino group, an oxy group, and a thio group. Specific examples of such A1 include the following formula (A1-ex).

[Chemical Formula 39]

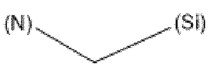
(A1-1)

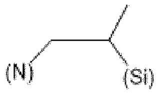
(A1-2)

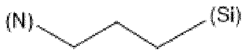
(A1-3)

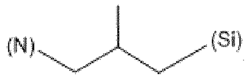
(A1-4)

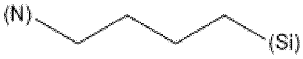
(A1-5)

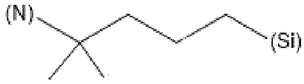
(A1-6)

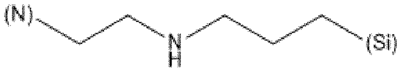
(A1-7)

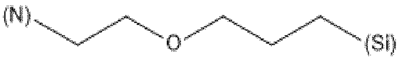
(A1-8)

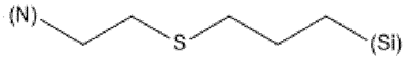
(A1-9)

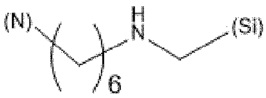
(A1-10)

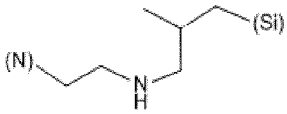
(A1-11)

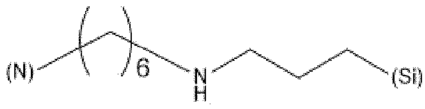
(A1-12)

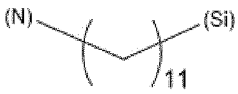
(A1-13)

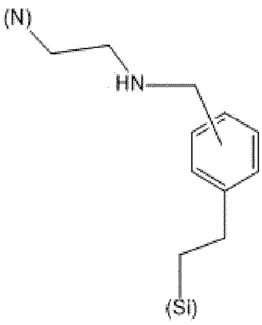
(A1-14)

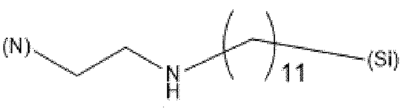
(A1-15)

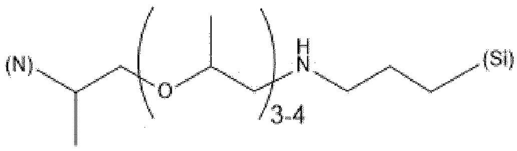
(A1-16)

・・・ (A1-ex)

[In the formula (A1-ex),

(N) represents a bond with N in the general formula (i-1), and
(Si) represents a bond with Si in the general formula (i-1).]

One of preferable examples of the inorganic porous support of the present invention is an inorganic porous support in which the silyl group (A) is represented by the following formula (i-1-1).
[Chemical Formula 40]

$$(X1)(Z1)_2Si\text{-}A2\text{-}NH\text{-}B2 \cdots \qquad (i\text{-}1\text{-}1)$$

[In the formula (i-1-1),

X1 represents a bond with the inorganic porous substrate,
Z1 represents an alkyl group having 1 to 6 carbon atoms or an aryl group having 6 to 12 carbon atoms,
A2 represents an alkylene group having 1 to 15 carbon atoms which may optionally contain any one or more of an imino group, an oxy group, and a thio group, and
B2 represents any one selected from the group consisting of a hydrogen atom and an alkyl group having 1 to 2 carbon atoms.]

The definitions of X1 and Z1 in the above formula are as described in the specification.
In the formula (i-1-1), A2 represents an alkylene group having 1 to 15 carbon atoms which may optionally contain any one of an imino group, an oxy group, and a thio group. Specific examples of A2 include (A1-1) to (A1-16) in the formula (A1-ex).
In the formula (i-1-1), B2 represents either a hydrogen atom or an alkyl group having 1 to 2 carbon atoms. Examples of the alkyl group having 1 to 2 carbon atoms in B2 include a methyl group and an ethyl group. Preferable examples of B2 include a hydrogen atom and a methyl group, and more preferable examples thereof include a hydrogen atom.
Examples of preferable combinations of X1, Z1, A2, and B2 for the group represented by the formula (i-1-1) are shown in the following formula (i-1-1#).

[Chemical Formula 41]

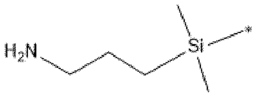
(i-1-1#-1)

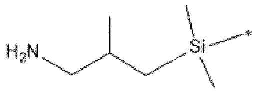
(i-1-1#-2)

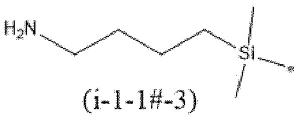
(i-1-1#-3)

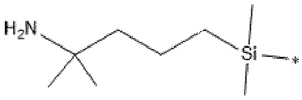
(i-1-1#-4)

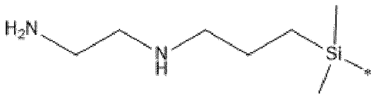
(i-1-1#-5)

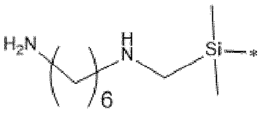
(i-1-1#-6)

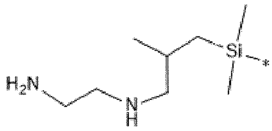
(i-1-1#-7)

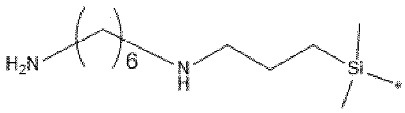
(i-1-1#-8)

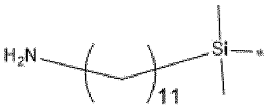
(i-1-1#-9)

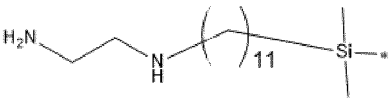
(i-1-1#-10)

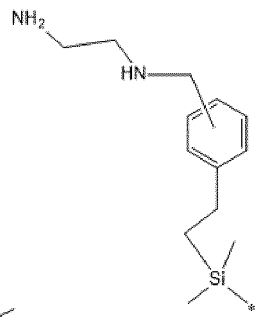
(i-1-1#-11)

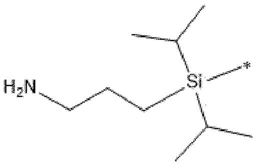
(i-1-1#-12)

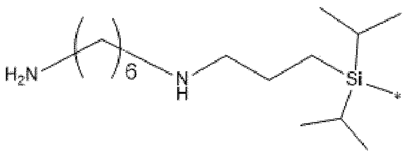
(i-1-1#-13)

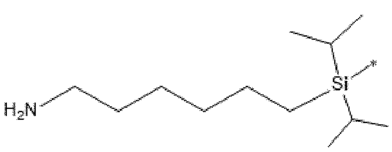
(i-1-1#-14)

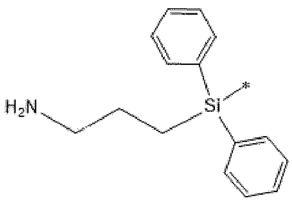
(i-1-1#-15)

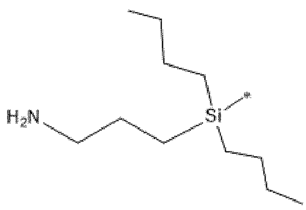
(i-1-1#-16)

・・・ (i－1－1#)

[In the formula (i-1-1#), * represents a bond with the inorganic porous substrate.]

In the present invention, an inorganic porous support having silyl groups of the following (vi) and (vii) and having the following characteristic (viii) is provided.

(vi) a silyl group (C): a silyl group represented by the following formula (vi-1),
(vii) a silyl group (D): at least one silyl group selected from the group consisting of silyl groups represented by the following formulas (vii-1), (vii-2), and (vii-3), and
(viii) a pore diameter of 20 nm or more.

[Chemical Formula 42]

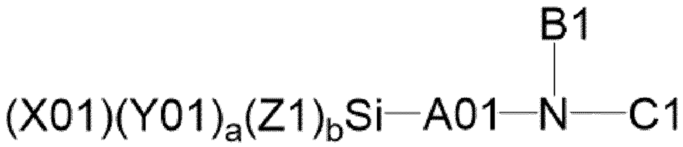

··· (vi−1)

[In the formula (vi-1),

X01 represents a bond with the inorganic porous support,
Y01 each independently represents any one selected from the group consisting of a bond with an inorganic porous support, a hydroxyl group, an amino group, an alkoxy group having 1 to 6 carbon atoms, and an alkylamino group having 1 to 12 carbon atoms,
Z1 each independently represents an alkyl group having 1 to 6 carbon atoms or an aryl group having 6 to 12 carbon atoms,
a represents an integer represented by 2-b,
b represents an integer of 0 to 2,
A01 represents an organic group having 1 to 20 carbon atoms,
B1 represents any one selected from the group consisting of a hydrogen atom, an alkyl group having 1 to 6 carbon atoms, and an aryl group having 6 to 12 carbon atoms, and
C1 represents a group having a nucleoside or nucleotide structure in which a reactive group is protected or deprotected.],
[Chemical Formula 43]

**(P01)(Q01)(R01)Si-J1** ··· (vii - 1),

[Chemical Formula 44]

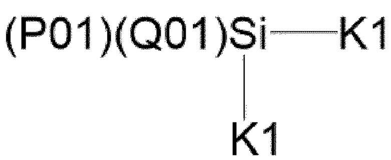

··· (vii−2)

,

[Chemical Formula 45]

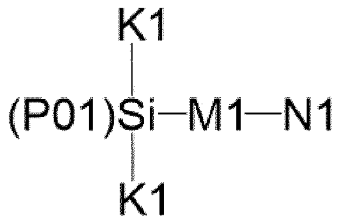

· · · (vii－3)

,

[In the formula (vii-1), (vii-2) or (vii-3),

P01 represents a bond with the inorganic porous support,
Q01 and R01 each independently represents any one selected from the group consisting of a bond with an inorganic porous support, a hydroxyl group, an amino group, an alkoxy group having 1 to 6 carbon atoms, and an alkylamino group having 1 to 12 carbon atoms,
J1 represents an alkyl group having 2 to 20 carbon atoms or an aryl group having 7 to 20 carbon atoms,
K1 each independently represents an alkyl group having 1 to 20 carbon atoms or an aryl group having 6 to 20 carbon atoms,
M1 represents an alkylene group having 1 to 6 carbon atoms, and
N1 represents an alkyl group having 2 to 20 carbon atoms or an aryl group having 6 to 20 carbon atoms.]

In the formula (vi-1), the bond with the inorganic porous support in X01 is specifically a bond via an -O-bond on the surface of the inorganic porous support.
In the formula (vi-1), Y01 each independently represents any one selected from the group consisting of the bond with the inorganic porous support, a hydroxyl group, an amino group, an alkoxy group having 1 to 6 carbon atoms, and an alkylamino group having 1 to 12 carbon atoms.
The bond with the inorganic porous support in Y01 is the same as in X01.
Specific examples and preferable examples of the alkoxy group having 1 to 6 carbon atoms and the alkylamino group having 1 to 12 carbon atoms in Y01 may include those similar to the alkoxy group having 1 to 6 carbon atoms and the alkylamino group having 1 to 12 carbon atoms in Y1.
Specific examples and preferable examples of Z1, a, b, and B1 in the formula (vi-1) may include those similar to Z1, a, and b in the formula (i-1).
A01 represents an organic group having 1 to 20 carbon atoms. Specific examples thereof include an alkylene group having 1 to 20 carbon atoms, an arylene group having 1 to 20 carbon atoms, an alkylene group having 1 to 20 carbon atoms which may optionally contain any one or more of an acylimino group, an oxy group, and a thio group, and an arylene group having 1 to 20 carbon atoms which may optionally contain any one or more of an acylimino group, an oxy group, and a thio group.
The acylimino group in A01 is a group in which the imino group in A1 in the formula (i-1) contained in the inorganic porous substrate which is a precursor of the inorganic porous support is derived to an acylimino group by the following capping treatment. Specific examples of the acylimino group in A01 include -N($COCH_3$)- and - N($COCH_2OPh$)-, and -N($COCH_3$)- is preferable.
In the formulas (vii-1), (vii-2), and (vii-3), the bond with the inorganic porous support in P01 is the same as in X01.
Q01 and R01 each independently represents any one selected from the group consisting of a bond with an inorganic porous support, a hydroxyl group, an amino group, an alkoxy group having 1 to 6 carbon atoms, and an alkylamino group having 1 to 12 carbon atoms.
The bond with the inorganic porous support in Q01 and R01 is the same as in X01.
Specific examples and preferable examples of the alkoxy group having 1 to 6 carbon atoms and the alkylamino group having 1 to 12 carbon atoms in Q01 and R01 may include those similar to the alkoxy group having 1 to 6 carbon atoms and the alkylamino group having 1 to 12 carbon atoms in Q1 and R1.
J1, K1, M1, and N1 in the formulas (vii-1), (vii-2), and (vii-3) have the same meanings as J1, K1, M1, and N1 in the above formulas (ii-1), (ii-2), and (ii-3), respectively.
The inorganic porous support in the present invention has a pore diameter of 20 nm or more from the viewpoint of application to nucleic acid synthesis. The inorganic porous support to be used can be appropriately selected according to the strand length of the nucleic acid to be synthesized. Usually, when the strand length of the nucleic acid to be synthesized is long, it is preferable to select the inorganic porous substrate having a large pore diameter. The pore diameter is preferably 40 nm or more and 500 nm or less, more preferably 45 nm or more and 300 nm or less, still more preferably 50

nm or more and 250 nm or less, and particularly preferably 60 nm or more and 200 nm or less.

In this case, the pore diameter can be determined from the pore diameter (mode diameter) obtained from the value of the X axis at a maximal value of the Y axis in a pore diameter distribution graph (plotted using the value of the pore diameter on the X-axis and on the Y-axis a value obtained by differentiating a pore volume with the pore diameter) obtained by analyzing an inorganic molded body, which is a raw material of the inorganic porous support, by a mercury intrusion method.

[Method of producing inorganic porous support]

Hereinafter, a method of producing the inorganic porous support of the present invention will be described.

The inorganic porous support of the present invention is produced by introducing a group (C1 in the formula (vi-1) or C2 in the following formula (vi-1-1)) having a nucleoside or nucleotide structure in which a reactive group is protected or deprotected into the active NH group in the inorganic porous substrate. As one of preferable examples of the inorganic porous support of the present invention, from the viewpoint of support synthesis and nucleic acid synthesis, an inorganic porous support in which C1 in the general formula (vi-1) or C2 in the following formula (vi-1-1) includes a succinyl linker or a universal linker is exemplified. More preferably, it is preferable to introduce a group having a nucleoside structure linked by a succinyl linker as described later.

As a specific example of the production, a production method of reacting a compound, having a nucleoside structure in which a reactive group is protected and a succinyl linker, with an active NH group in the inorganic porous substrate can be mentioned. Preferable examples of the compound having the nucleoside structure in which the reactive group is protected and the succinyl linker include compounds having a carboxylic acid terminal (hereinafter, such a compound may be referred to as an nsuc compound). Specifically, those represented by the following formula (nsuc-1) can be exemplified. Examples of the preferable reaction include a condensation reaction between the active NH group in the inorganic porous substrate and the nsuc compound.

[Chemical Formula 46]

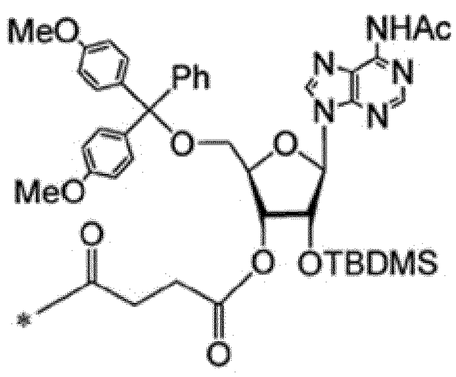

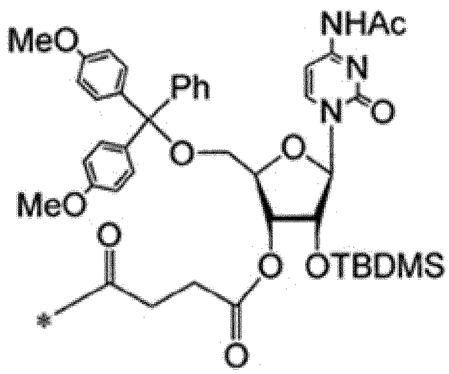

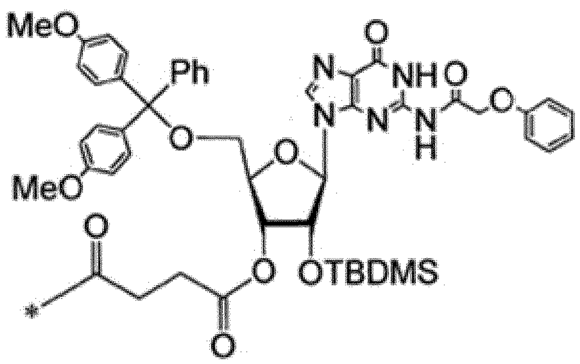

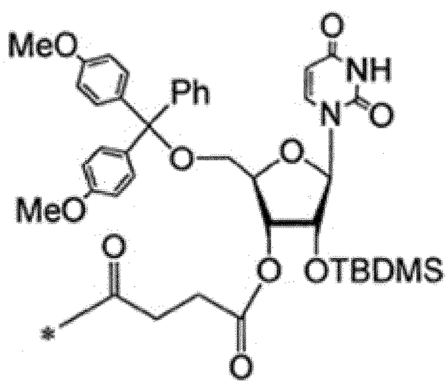

・・・(nsuc−1)

[In the formula (nsuc-1), * represents a hydroxyl group, a salt formed of a hydroxyl group and pyridine, or a salt formed of a hydroxyl group and triethylamine, and TBDMS represents a tert-butyldimethylsilyl group.]

The condensation reaction as described above is carried out by mixing the inorganic porous substrate, the nsuc compound, the condensing agent, and an appropriate solvent, and usually shaking the mixture at room temperature or increasing the temperature of the mixture to facilitate the condensation reaction. The condensation reaction may also be carried out by allowing the mixture to stand still without shaking or to be stirred.

Although the condensation reaction is carried out for about 4 to 30 hours, the reaction time may be appropriately lengthened or shortened.

As the condensing agent for the condensation reaction, any condensing agent to be usually used for an amide condensation can be used. Specific examples of the condensing agent include N,N'-dicyclohexylcarbodiimide (DCC), N,N'-diisopropylcarbodiimide (DIC), 1-ethyl-3-(3-dimethylaminopropyl) carbodiimide hydrochloride (EDAC), 1-ethyl-3-(3-dimethylaminopropyl)carbodiimide (EDC), 1-[bis(dimethylamino)methylene]-1H-1,2,3-triazolo[4,5-b]pyridinium 3-oxide hexafluorophosphate (HATU), 1-[bis(dimethylamino)methylene]-1H-1,2,3-benzotriazolium 3-oxide hexafluorophosphate (HBTU), 1-[bis(dimethylamino)methylene]-1H-1,2,3-triazolo[4,5-b]pyridinium 3-oxide tetrafluoroborate (TATU), 1-[bis(dimethylamino)methylene]-1H-1,2,3-benzotriazolium 3-oxide tetrafluoroborate (TBTU), (1-cyano-2-ethoxy-2-oxoethylideneaminooxy)dimethylaminomorpholinocarbenium hexafluorophosphate (COMU), and O-[(ethoxycarbonyl)cyanomethyleneamino]-N,N,N',N'-tetramethyluronium hexafluorophosphate (TOTU).

In the condensation reaction, additives such as N,N-dimethyl-4-aminopyridine (DMAP), N,N-diisopropylethylamine, and 1-methylimidazole may be appropriately added.

After the condensation reaction, the solid content is filtered off and washed. Examples of a solvent for washing include acetonitrile. An unreacted active NH group and an amino group having another N-H site contained in the silyl group on the inorganic porous support are subjected to a capping treatment. As a reagent used for the capping treatment, a mixture of an acid anhydride and a nitrogen-containing organic base can be used. Specific examples of the acid anhydride include acetic anhydride and phenoxyacetic anhydride, and acetic anhydride is preferable. Specific examples of the nitrogen-containing organic base include nitrogen-containing aromatic heterocyclic compounds such as 1-methylimidazole, 2,6-lutidine, pyridine, and 4-dimethylaminopyridine, and tertiary alkylamines such as triethylamine. As an addition amount of the reagent used for the capping treatment, 10 times molar equivalents or more of an amount of the NH group contained in the inorganic porous substrate is used in the acid anhydride, and an equimolar equivalent or greater of the acid anhydride to be used is used in the nitrogen-containing organic base. When the addition amount is such an amount, the NH group contained in the inorganic porous substrate is sufficiently capped. As the reagent used in the capping treatment, an organic solvent may be used in combination as appropriate, and specific examples thereof include tetrahydrofuran, dichloromethane, and acetonitrile. An amount of the organic solvent used is desirably a volume amount in which the solid content can be sufficiently immersed.

After the capping treatment, washing is performed with acetonitrile and the like, and drying is performed to obtain an inorganic porous support.

The particle diameter of the inorganic porous support is characterized by being 1 μm or more from the viewpoint of preventing clogging of nucleic acid synthesizer piping, and the like in the production of a nucleic acid described later. The particle diameter is preferably 5 μm or more, more preferably 10 μm or more, still more preferably 20 μm or more, and particularly preferably 30 μm or more. The particle diameter can be controlled by sieving the inorganic molded body, the inorganic porous substrate, or the inorganic porous support, which is a raw material, with a sieve having an opening equal to or larger than the corresponding particle diameter to remove fine components.

The particle diameter of the inorganic porous support of the present invention can be determined as an average value of major diameters of arbitrary 50 particles in observation of the inorganic molded body as a raw material in a visual field of 200 magnification with a scanning electron microscope.

From the viewpoint of application to nucleic acid synthesis, the inorganic porous support according to the present invention is characterized by having a cumulative pore volume in the pore diameter range of 40 nm to 1000 nm of more than 0.32 mL/g and 4 mL/g or less. The cumulative pore volume in the same range is preferably more than 0.35 mL/g and 3.5 mL/g or less, more preferably more than 0.4 mL/g and 3 mL/g or less, still more preferably more than 0.43 mL/g and 3 mL/g or less, and even more preferably more than 0.45 mL/g and 2.5 mL/g or less. In this case, the cumulative pore volume in the pore diameter range of 40 nm to 1000 nm can be determined by analyzing the inorganic molded body, which is a raw material, by the mercury intrusion method.

As one of preferable examples of the inorganic porous support of the present invention, the inorganic porous support has a specific surface area of 0.1 $m^2/g$ or more and 200 $m^2/g$ or less from the viewpoint of being used for nucleic acid synthesis. As the specific surface area, the value of the specific surface area determined from the average gradient in the range of $\alpha s$ = 1.7 to 2.1 according to the as-plot method by performing nitrogen adsorption/desorption isotherm measurement on the inorganic molded body as a raw material can be applied. The specific surface area is preferably 1 $m^2/g$ or more and 150 $m^2/g$ or less, more preferably 5 $m^2/g$ or more and 100 $m^2/g$ or less, still more preferably 8 $m^2/g$ or more and 80 $m^2/g$ or less, and particularly preferably 10 $m^2/g$ or more and 60 $m^2/g$ or less.

The specific surface area of the inorganic porous support is adjusted by the specific surface area of the inorganic molded body which is a raw material, and it is desirable to use the inorganic molded body within the above range as a raw material.

As an inorganic material used for the inorganic porous support, various inorganic materials can be used, and an inorganic material containing a silicon oxide with a silanol group is preferable. Specifically, inorganic materials containing silica, silica gel, zeolite, or glass are preferable, and it is preferable to use an inorganic molded body containing such an inorganic material as a raw material.

The inorganic porous body according to the present invention is characterized in that an amount of a group containing a nucleoside or a nucleotide structure in which a reactive group is protected or deprotected satisfies the following formula (Nu#1):

[Math. 7]

$$0.05 \leq I01/S01 \leq 3.0 \cdots (Nu\#1)$$

I01: a group (μmol/g) having a nucleoside or nucleotide structure in which a reactive group is protected or deprotected in the inorganic porous support, and

S01: a specific surface area ($m^2/g$) of the inorganic porous support obtained by nitrogen adsorption/desorption isotherm measurement

The specific surface area ($m^2/g$) of the inorganic porous support obtained by nitrogen adsorption/desorption isotherm measurement can be determined by the above-described method.

The group (μmol/g) having the nucleoside or nucleotide structure in which the reactive group is protected or deprotected in the inorganic porous support can be determined by the following method.

The loading (hereinafter, may be referred to as the nucleoside loading) of the group having the nucleoside or nucleotide structure in which the reactive group is protected or deprotected in the inorganic porous support can be determined by the following measurement method when a protecting group such as a 4,4'-dimethoxytrityl group is introduced. That is, first, 70% perchloric acid is diluted with methanol to prepare a 30% perchloric acid solution. Then, 20 to 50 mg of the inorganic porous support is taken in a volumetric flask and diluted to 10 mL with a 30% perchloric acid solution. This solution is further diluted 10 times with the 30% perchloric acid solution, and then an absorbance of the desorbed 4,4'-dimethoxytrityl cation at 498 nm is measured to calculate the nucleoside loading.

A preferable range of I01/S01 is 0.1 or more and 2.0 or less, a more preferable range is 0.3 or more and 1.8 or less, and a still more preferable range 0.4 or more and 1.6 or less.

As one of preferable examples of the inorganic porous support of the present invention, from the viewpoint of support synthesis and nucleic acid synthesis, an inorganic porous support in which C1 in the general formula (i-1) as the inorganic porous support includes a succinyl linker or a universal linker is exemplified.

The universal linker contains a functional group (typically, a hydroxyl group) which forms a phosphite with the hydroxyl group of the nucleotide that provides a starting point of nucleic acid synthesis, and a functional group which has the ability to bond with the active NH group, and further contains an adjacent protected functional group (for example, a protected amino group, a protected hydroxyl group, or a protected thiol group) in the same molecule, which has the ability to nucleophilically attack a phosphorus atom of phosphoric acid under the conditions for cleaving the synthesized nucleic acid.

More specifically, examples of the succinyl linker include a linking group represented by the following formula L10#, and examples of the universal linker include a linking group represented by the following formula L11#.

[Chemical Formula 47]

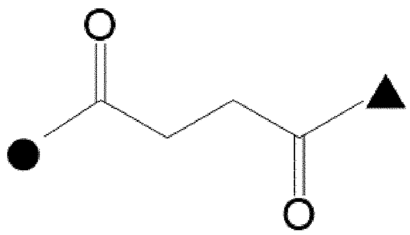

· · · (L10#)

,

[Chemical Formula 48]

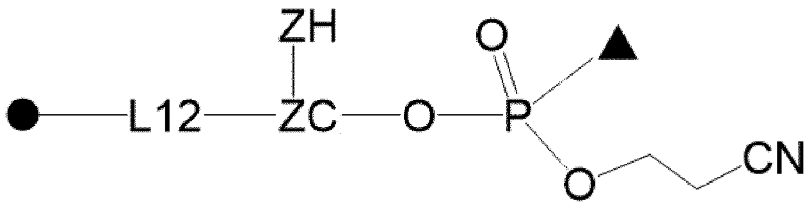

・・・(L11#)

In the formulas L10# and L11#, a bond marked with • represents a bond with N in the formula (i-1). A bond marked with ▲ represents a bond with an oxygen atom of a primary or secondary hydroxyl group in a nucleoside or nucleotide structural site, in which the reactive group at C1 is protected or deprotected.

In the formula L11#, ZH represents a protected amino, hydroxyl, or thiol group. ZC represents any group containing a carbon atom, and an oxygen atom bonded to ZC and ZH represent groups adjacent to each other (for example, the oxygen atom and ZH are present in the vicinal, and the carbon atoms in ZC to which each of the oxygen atom and ZH is directly bonded to each other). L12 represents a group linking N in the formula (i-1) and ZC in the formula (L11#) (specific examples thereof include a group represented by L10#).

When the universal linker is used, even though the 3' end of the nucleic acid to be synthesized is any kinds of nucleoside or nucleotide, the nucleoside phosphoramidide providing the 3' end can be reacted and introduced in the same manner as the step of elongating the nucleic acid according to the usual nucleic acid automatic synthesis. Examples of such a universal linker include the compounds described in the following references, but are not limited thereto:

Reference: A.P. Guzaev, and M. Manoharan, J Am Chem Soc, 2003, 125, 2380-2381.
Reference: R.K. Kumar, A.P. Guzaev, C. Rentel, and V.T Ravikumar, Tetrahedron, 2006, 62, 4528.

Among those in which C1 in the general formula (i-1) contains a succinyl linker or a universal linker, those containing a succinyl linker are more preferable.

It is preferable for the nucleoside or nucleotide structural site in which the reactive group at C1 is protected or deprotected that the hydroxyl group at the 5'-position of the nucleoside, which provides the starting point of the nucleic acid elongation reaction, is protected with a trityl-based protecting group (for example, 4,4'-dimethoxytrityl (DMTr) group, etc.).

Similarly, when the universal linker is used, it is preferable that the hydroxyl group, which provides the starting point of the nucleic acid elongation reaction, is protected with a trityl-based protecting group (for example, 4,4'-dimethoxytrityl (DMTr) group, etc.).

Specific examples of C1 containing a succinyl linker include C1 represented by the following formula (nsuc-2).

[Chemical Formula 49]

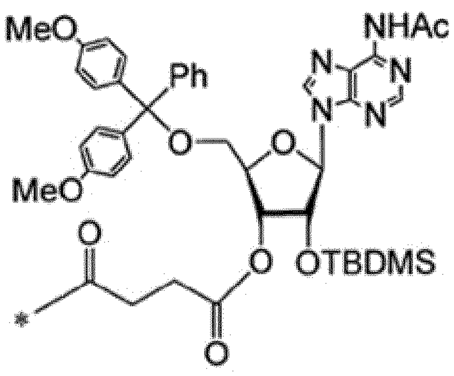

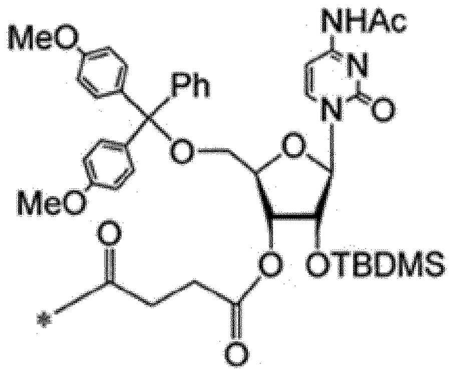

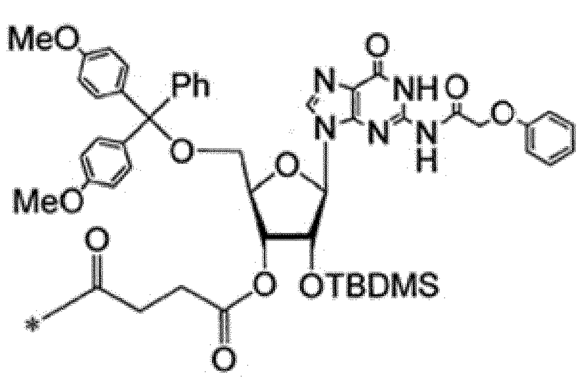

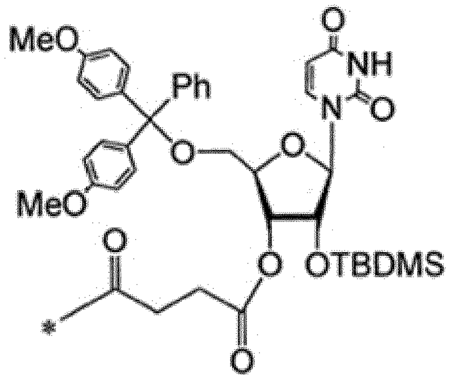

・・・(nsuc-2)

[In the formula (nsuc-2), * represents a bond with N in the general formula (vi-1).]

As one of preferable examples of the inorganic porous support of the present invention, an inorganic porous support having a silyl group, represented by the formula (vii-3), as the silyl group (D) is exemplified, and among them, an inorganic porous support having a silyl group, represented by the following formula (vii-3-1), as the silyl group (D) is preferable.

[Chemical Formula 50]

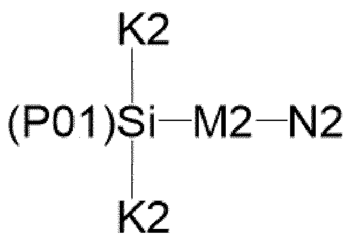

・・・(vii-3-1)

[In the formula (ii-3-1),

P01 represents a bond with the inorganic porous support,
K2 each independently represents an alkyl group having 1 to 6 carbon atoms or an aryl group having 6 to 10 carbon atoms,
M2 represents an alkylene group having 1 to 4 carbon atoms, and
N2 represents an alkyl group having 2 to 6 carbon atoms or an aryl group having 6 to 10 carbon atoms.]

In the formula (vii-3-1), P01, K2, M2, and N2 have the same meanings as P1, K2, M2, and N2 in the formula (ii-3-1), respectively. Specific examples of the silyl group represented by the formula (vii-3-1) include the formula (ii-3-1-A).

One of preferable examples of the inorganic porous support of the present invention is an inorganic porous

support in which A01 in the formula (vi-1) is an alkylene group having 1 to 20 carbon atoms which may optionally contain any one or more of an acylimino group, an oxy group, and a thio group.

Specific examples of A01 include the following formula (A01-ex).

[Chemical Formula 51]

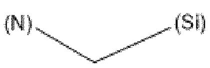
(A01-1)

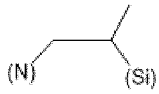
(A01-2)

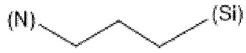
(A01-3)

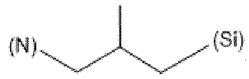
(A01-4)

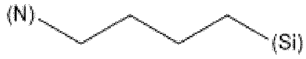
(A01-5)

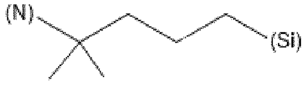
(A01-6)

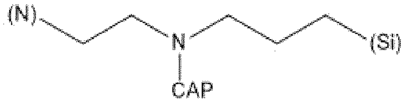
(A01-7)

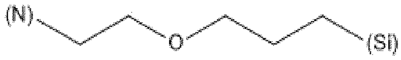
(A01-8)

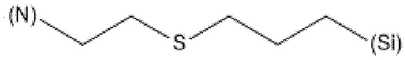
(A01-9)

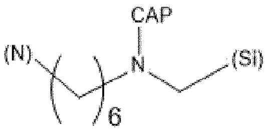
(A01-10)

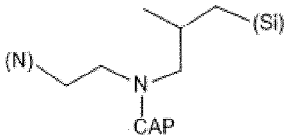
(A01-11)

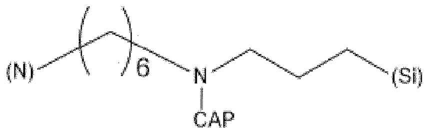
(A01-12)

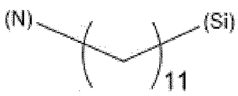
(A01-13)

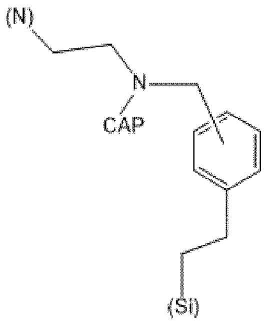
(A01-14)

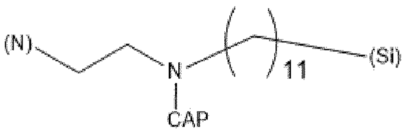
(A01-15)

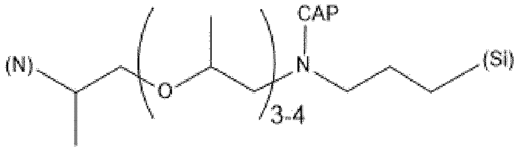
(A01-16)

・・・ (A01−ex)

[In the formula (A01-ex),

(N) represents a bond with N in the general formula (vi-1),
(Si) represents a bond with Si in the general formula (vi-1), and
CAP is a cap group introduced by the cap treatment, and represents an acetyl group as a specific example.]

One of preferable examples of the inorganic porous support of the present invention is an inorganic porous support in which the silyl group (C) is a silyl group represented by the following formula (vi-1-1).

[Chemical Formula 52]

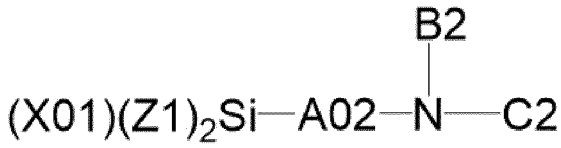

···(vi-1-1)

[In the formula (vi-1-1),

X01 represents a bond with the inorganic porous support,
Z1 each independently represents an alkyl group having 1 to 6 carbon atoms or an aryl group having 6 to 12 carbon atoms,
A02 represents an alkylene group having 1 to 15 carbon atoms which may optionally contain any one or more of an acylimino group, an oxy group, and a thio group,
B2 represents either a hydrogen atom or an alkyl group having 1 to 2 carbon atoms, and
C2 represents a group including a succinyl linker and having a nucleoside or nucleotide structure in which a reactive group is protected or deprotected.]

In the formula (vi-1-1), X01 has the same meaning as X01 in the formula (vi-1).
In the formula (vi-1-1), A02 represents an alkylene group having 1 to 15 carbon atoms which may optionally contain any one of an acylimino group, an oxy group, and a thio group.
The acylimino group in A02 is a group in which the imino group in A2 in the formula (i-1-1) contained in the inorganic porous substrate which is a precursor of the inorganic porous support is derived to an acylimino group by the capping treatment. Specific examples of the acylimino group in A02 include $-N(COCH_3)-$ and $-N(COCH_2OPh)-$, and $-N(COCH_3)-$ is preferable.
Specific examples of A02 include (A01-1) to (A01-16) in the formula (A01-ex).
In the formula (vi-1-1), B2 represents either a hydrogen atom or an alkyl group having 1 to 2 carbon atoms. Examples of the alkyl group having 1 to 2 carbon atoms in B2 include a methyl group and an ethyl group. Preferable examples of B2 include a hydrogen atom and a methyl group, and more preferable examples thereof include a hydrogen atom.
In the formula (vi-1-1), C2 represents a group including a succinyl linker and having a nucleoside or nucleotide structure in which a reactive group is protected or deprotected. Specifically, those represented by the formula (nsuc-2) can be exemplified.
Examples of preferable combinations of X01, Z1, A02, and B2 for the group represented by the formula (vi-1-1) are shown in the following formula (vi-1-1#).

[Chemical Formula 53]

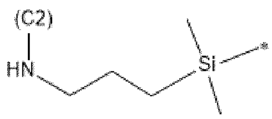

(vi-1-1#-1)

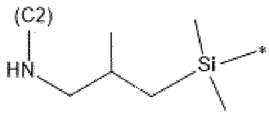

(vi-1-1#-2)

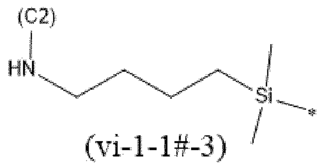

(vi-1-1#-3)

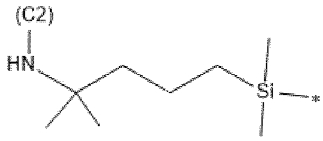

(vi-1-1#-4)

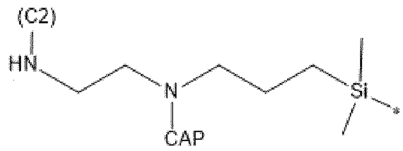

(vi-1-1#-5)

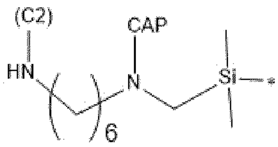

(vi-1-1#-6)

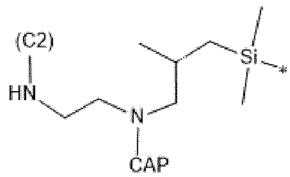

(vi-1-1#-7)

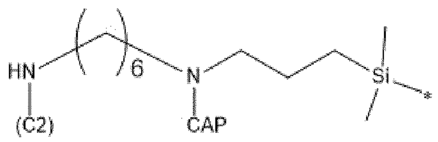

(vi-1-1#-8)

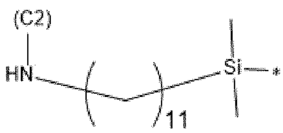

(vi-1-1#-9)

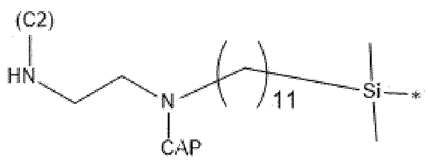

(vi-1-1#-10)

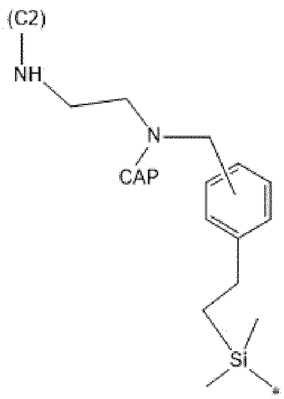

(vi-1-1#-11)

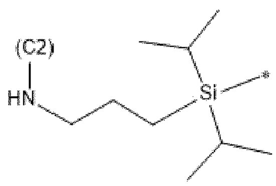

(vi-1-1#-12)

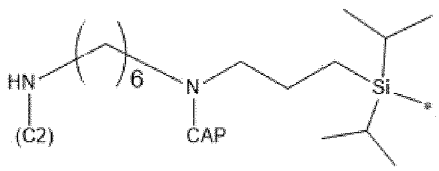

(vi-1-1#-13)

· · · (vi-1-1#)

[In the formula (vi-1-1#),

(C2) represents a bond with C2 in the formula (vi-1-1),
* represents a bond with the inorganic porous support, and
CAP is a cap group introduced by the cap treatment, and represents an acetyl group as a specific example.]

The solid-phase support of the present embodiment is preferable as a substrate for a solid-phase synthesis of nucleic acid (DNA and RNA). Further, the solid-phase support of the present embodiment is particularly suitable for the synthesis of RNA, which has been considered to have a problem in stability as compared with DNA.

Hereinafter, the solid-phase synthesis of RNA is illustrated as an example of the preparation method, and the nucleic acid production method is described with reference to a reaction route shown below (condensation reaction, oxidation, and deprotection). Here, relative to the reaction route illustrated below, an example in which a group having a nucleoside structure is used as C1 in the formula (vi-1) is shown.

Example of reaction route

[Chemical Formula 54]

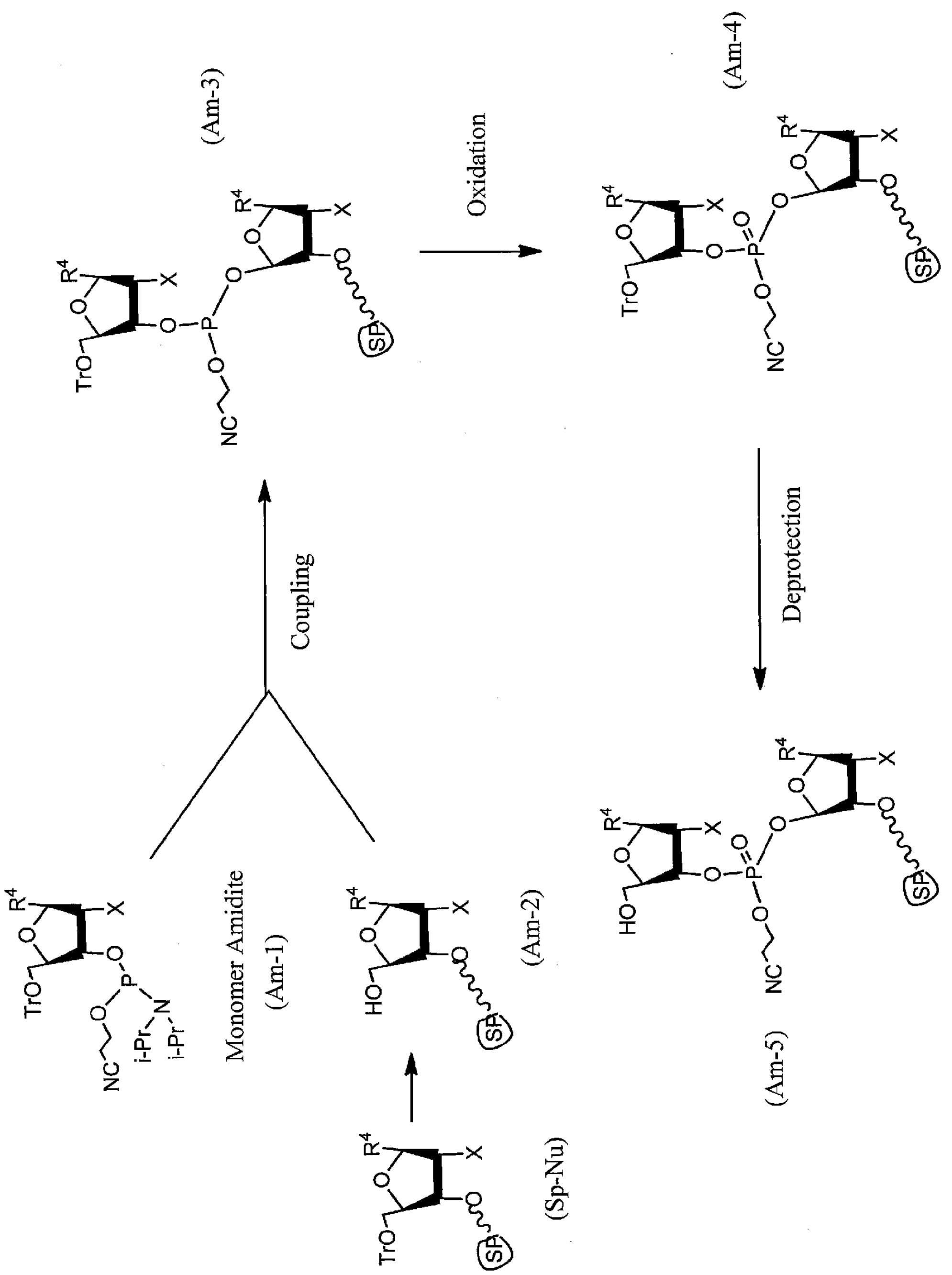


In the chemical formula shown in the reaction route, $R^4$ represents a base; Tr represents a protecting group; X represents -H, -OH or -$OR^5$ (wherein, $R^5$ represents a protecting group), and SP represents a moiety other than the nucleoside structure of the inorganic porous support.

The base ($R^4$) constituting the inorganic porous support (Sp-Nu) having the nucleoside structure and the nucleoside of the amidite monomer (Am-1) is usually a nucleic acid, and typically a natural base which is composed of RNA, however, a non-natural base may be used appropriately. Examples of such the non-natural base include modified analogs of the natural base or non-natural base.

Examples of the base represented by $R^4$ include purine bases such as adenine, isoguanine, xanthine, hypoxanthine and guanine; and pyrimidine bases such as cytosine, uracil and thymine; and the like.

Examples of the base represented by $R^4$ include alkyl derivatives such as 2-aminoadenine and 6-methylated purine; alkyl derivatives such as 2-propylated purine; 5-halouracil and 5-halocytosine; 5-propynyluracil and 5-propynylcytosine; 6-azouracil, 6-azocytosine, and 6-azothymine; 5-uracil (pseudouracil), 4-thiouracil, 5-halouracil, 5-(2-aminopropyl)uracil, and 5-aminoallyluracil; 8-halogenated, aminated, thiolated, thioalkylated, hydroxylated and other 8-substituted purines; 5-trifluoromethylated and other 5-substituted pyrimidines; 7-methylguanine; 5-substituted pyrimidine; 6-aza pyrimidine; N-2, N-6, and O-6-substituted purines (including 2-aminopropyladenine); 5-propynyluracil and 5-propynylcytosine; dihydrouracil; 3-deaza-5-aza cytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazole; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N4-acetylcytosine; 2-thiocytosine; N6-methyladenine; N6-isopentyladenine; 2-methylthio-N6-isopentenyladenine; N-methylguanine; and O-alkylated base.

The purine compound and the pyrimidine compound include, for example, those disclosed in U.S. Pat. No. 3,687,808, "Concise Encyclopedia Of Polymer Science And Engineering", pages 858-859, ed. Kroschwitz J. I., John Wiley & Sons, 1990, and Englisch et al., Angewandte Chemie, International Edition, 1991, vol. 30, p. 613.

Examples of the amidite monomer (Am-1) preferably include TBDMS amidite (TBDMS RNA Amidites, product name, ChemGenes Corporation), ACE amidite, TOM amidite, CEE amidite, CEM amidite, TEM amidite (Reviews by Chakhmakhcheva: Protective Groups in the Chemical Synthesis of Oligoribonucleotides, Russian Journal of Bioorganic Chemistry, 2013, Vol. 39, No. 1, pp. 1-21), and EMM amidite (as described in WO 2013/027843 A1), or the like, in which $R^5$ in the compound represented by the following chemical formula (Am-1') is protected with tert-butyldimethylsilyl (TBDMS) group, bis(2-acetoxy)methyl (ACE) group, (triisopropylsilyloxy)methyl (TOM) group, (2-cyanoethoxy)ethyl (CEE) group, (2-cyanoethoxy)methyl (CEM) group, para-tolylsulfonylethoxymethyl (TEM) group, (2-cyanoethoxy)methoxymethyl (EMM) group, or the like.

[Chemical Formula 55]

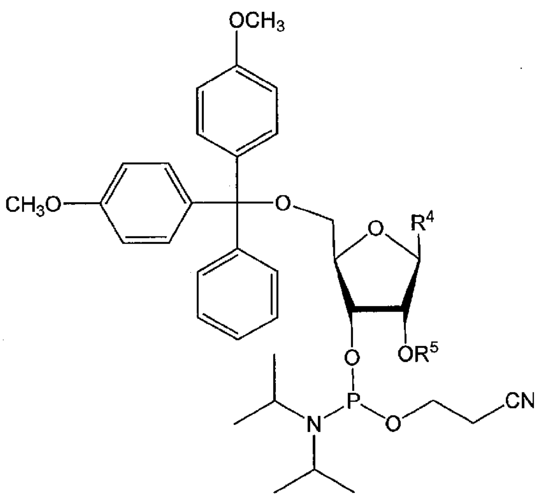

(Am−1')

[Wherein, $R^5$ represents a protecting group of the hydroxyl group; and $R^4$ represents a protected nucleobase.]

The inorganic porous support of the present embodiment may also be used to incorporate a divalent group other than a nucleoside and nucleotide into a nucleic acid sequence. For example, an amidite having a proline framework (for example, Amidite P as described later) can be incorporated into a nucleic acid sequence according to the amidite method (see the same method as the method of Example A4 of WO 2012/017919 A1). Further, the amidite represented by each of the following structural formulae (Am-11), (Am-12), and (Am-13) (see Examples A1 to A3 of WO 2013/103146 A1) may also be used.

[Chemical Formula 56]

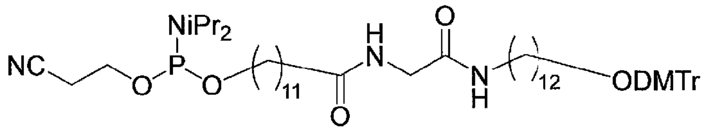

···(Am−11)

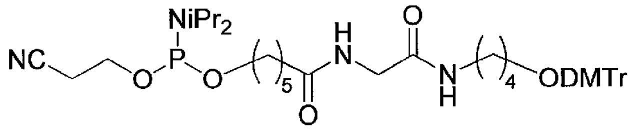

···(Am−12)

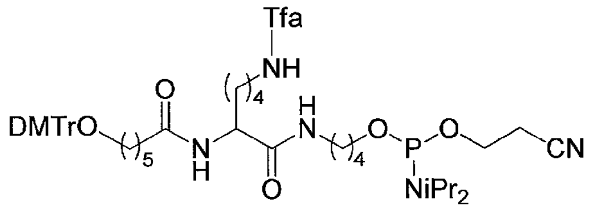

···(Am−13)

[Wherein iPr represents an isopropyl group, DMTr represents a 4,4'-dimethoxytrityl group, and Tfa represents a trifluoroacetyl group.]

[Solid-phase synthesis of RNA]

The Tr group of the inorganic porous support (Sp-Nu) is deprotected to obtain a solid-phase support (Am-2). Then, the amidite monomer (Am-1) and the solid-phase support (Am-2) are subjected to a condensation reaction to obtain a reaction product (Am-3). Then, the reaction product (Am-3) is oxidized to obtain the product (Am-4). Then, the product (Am-4) is deprotected (-Tr) to obtain the product (Am-5). Then, the amidite monomer (Am-1) and the product (Am-5) are further subjected to a condensation reaction to elongate the phosphodiester bond. As described above, the hydroxyl group of the 5'-position at the end of the elongated oligonucleotide strand is repeatedly subjected to a series of cycle including deprotection, condensation reaction and oxidation as many times as necessary so as to provide a desired sequence, and then the resulting product can be cleaved from the solid-phase support to produce a nucleic acid molecule having a desired sequence.

More specifically, a nucleic acid is prepared according to a preparation method including the following steps:

step (A): a step of deprotecting the protecting group of the hydroxyl group at the 5'-position of the nucleoside using the inorganic porous support wherein R in the general formula (2) represents a nucleoside or nucleotide in which a hydroxyl group as a reactive group is protected;
step (B): a condensation step of subjecting the hydroxyl group at the 5'-position of the nucleoside produced in the step (A) to a condensation reaction with an amidite compound having a second nucleoside base to produce a phosphite;
step (C): an oxidation step of oxidizing the phosphite produced in the step (B) to produce a nucleotide; and
step (D): a step of deprotecting the protecting group of the hydroxyl group at the 5'-position of the nucleotide produced in the step (C).

The preparation method including the steps (A) to (D) may optionally include the following steps:

step (B'): a step of further subjecting the product produced in the step (D) to a condensation reaction with an amidite compound having a nucleoside base to be introduced in next time to produce a phosphite;
step (C'): a step of oxidizing the phosphite produced in the step (B') to produce an oligonucleotide;
step (D'): a step of deprotecting the protecting group of the hydroxyl group at the 5'-position in the end of the oligonucleotide strand produced in the step (C'); and
step (E): a step of carrying out a series of steps consisting of the step (B'), step (C'), and step (D') repeatedly m times (wherein m is an integer of 1 or more) to react the number of m of amidite compounds (nucleic acid elongation reaction), and then cleaving an elongated nucleic acid.

The nucleic acid elongation reaction of the present embodiment can be carried out according to the procedure of a general phosphoramidite method.

The "nucleic acid elongation reaction" herein refers to a reaction in which a nucleic acid strand, particularly RNA strand, is elongated by sequentially binding nucleotides through a phosphodiester bond. The nucleic acid elongation

reaction may be carried out by means of an automatic nucleic acid synthesizer or the like that employs the phosphoramidite method.

In the deprotection step, the protecting group of the hydroxyl group at the 5'-position in the end of the RNA strand supported on the solid-phase support is deprotected. As a general protecting group, a trityl-based protecting group (typically, a DMTr group) is used. The deprotection can be carried out with an acid. Examples of the acid for deprotection include trifluoroacetic acid, trichioroacetic acid, dichloroacetic acid, trifluoromethanesulfonic acid, methanesulfonic acid, hydrochloric acid, acetic acid, and p-toluenesulfonic acid.

In the condensation step, the nucleoside phosphoramidite is bound to the hydroxyl group at the 5'-position in the end of the RNA strand which is deprotected by the above-mentioned deprotection step so as to produce the phosphite. As the nucleoside phosphoramidite, a nucleoside phosphoramidite in which the hydroxyl group at the 5'-position is protected with a protecting group (for example, DMTr group) is used.

Further, the condensation step can be carried out with an activator which activates the nucleoside phosphoramidite. Examples of the activator include 5-benzylthio-1H-tetrazole (BTT), 1H-tetrazole, 4,5-dicyanoimidazole (DCI), 5-ethylthio-1H-tetrazole (ETT), N-methylbenzimidazolium triflate (N-MeBIT), benzimidazolium triflate (BIT), N-phenylimidazolium triflate (N-PhIMT), imidazolium triflate (IMT), 5-nitrobenzimidazolium triflate (NBT), 1-hydroxybenzotriazole (HOBT), and 5-(bis-3,5-trifluoromethylphenyl)-1H-tetrazole (Activator-42).

After the condensation step, an unreacted hydroxyl group at the 5'-position may be capped as needed. The capping can be carried out with a publicly known capping solution such as acetic anhydride-tetrahydrofuran solution and phenoxyacetic acid/N-methylimidazole solution.

The oxidation step refers to a step of oxidizing the phosphite formed by the condensation step. The oxidation step can be carried out with an oxidizing agent. Examples of the oxidizing agent include iodine, m-chloroperbenzoic acid, tert-butylhydroperoxide, 2-butanoneperoxide, bis(trimethylsilyl)peroxide, 1,1-dihydroperoxycyclododecane, and hydrogen peroxide.

The oxidation step may be carried out after the capping operation as described above, or conversely, the capping operation may be carried out after the oxidation step, and accordingly an order of them is not limited thereto.

After the oxidation step, the method returns to the deprotection step, and a series of steps consisting of the condensation reaction, the oxidation, and the deprotection is repeated depending on a nucleotide sequence of RNA to be synthesized, whereby RNA having a desired sequence can be synthesized.

After the synthesis of the RNA strand having the desired sequence is completed, the RNA strand is cleaved from the solid-phase support by ammonia, amines, or the like, and collected.

Examples of the amines as describe above include methylamine, ethylamine, isopropylamine, ethylenediamine, diethylamine, and triethylamine. When the universal linker is used, after the completion of the synthesis of RNA strand, the RNA strand is cleaved from the solid-phase support by ammonia, amines, or the like, and the universal linker is eliminated with a nucleophile. Once the elimination is completed, the 3'-position of a terminal nucleotide is changed to a hydroxyl group, and the phosphate is bound to the universal linker to form a cyclic phosphodiester. The collected RNA may be purified by a publicly known method, as needed.

In addition, by applying the inorganic porous support of the present embodiment to nucleic acid synthesis, a high-purity oligonucleic acid can be obtained in good yield.

In the present specification, the "yield of RNA" refers to a ratio (%) of RNA actually isolated to an amount of RNA theoretically calculated from an amount of nucleoside subjected to the reaction. An amount of nucleic acid is calculated from the measurement of the absorbance of UV. As a method of such measurement, specifically, a nucleic acid is dissolved in water or a buffered aqueous solution and placed in a cell having an optical path length of 1 cm. Using a UV absorptiometer, an optical density C is calculated from the absorbance at a wavelength of 260 nm according to the following formula to calculate the amount of nucleic acid. A coefficient $\alpha$ is 40 $\mu$g/mL.

$$C = \alpha \times L \times A$$ (A: absorbance, $\alpha$: coefficient, L: optical path length, C: optical density) [Math. 8]

"Purity of RNA" refers to a proportion (%) at which a nucleic acid of a target strand length is obtained. The purity of RNA is determined from an area percentage (that is, area percentage value) in a chromatogram by liquid chromatography or a 10% width of a main peak.

## EXAMPLES

Hereinafter, the present invention is described in more detail with reference to Examples; however, the present invention should not be limited to these examples.

Hereinafter, representative reaction schemes relating to the production of the inorganic porous support of the present invention will be described; however, the production method of the present invention is not limited thereto.

[Chemical Formula 57]

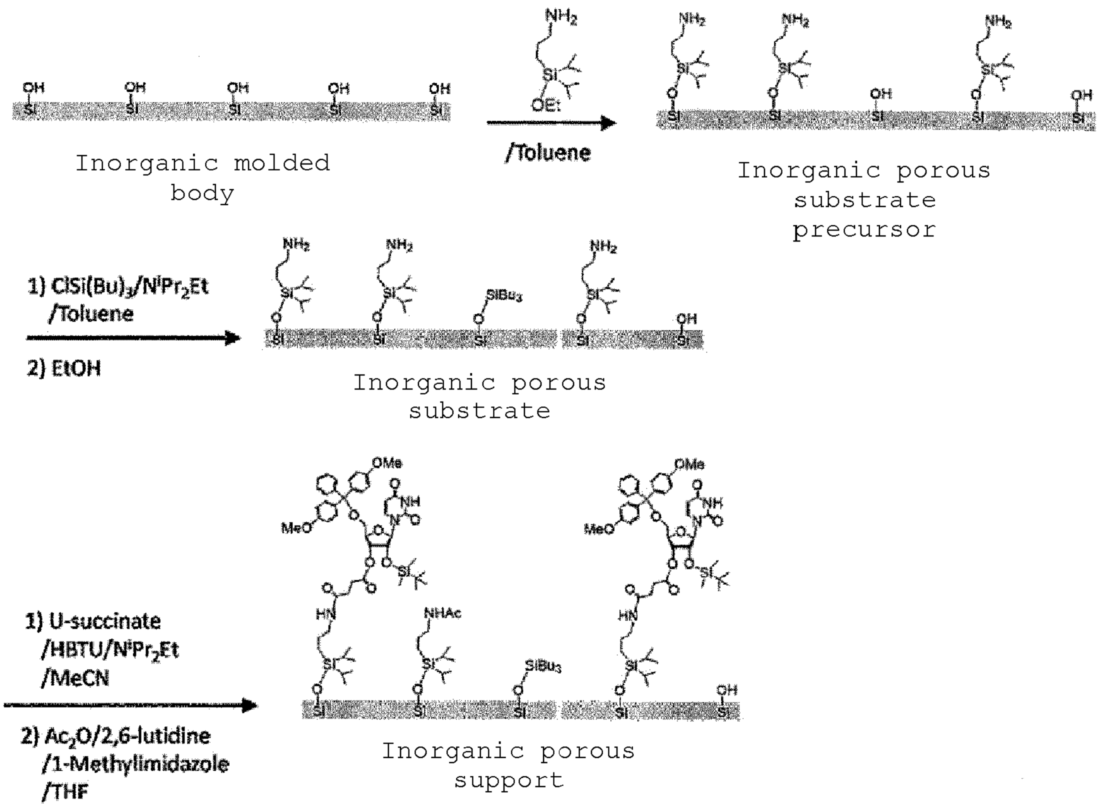


<Production of inorganic molded body>

(Production Example 1)

A zeolite molded body (1) was obtained in the same manner as in Example 1 described in JP 5875843 B2. The zeolite molded body (1) was sieved with a sieve having an opening of 38 μm to remove a particulate component. The sieved product (50 g) was placed in an autoclave, a 1.6 mol/L aqueous potassium hydroxide solution (500 g) was added thereto, and after the mixture was stirred at about 20°C for 5 hours, a solid was separated by filtration. Thereafter, water washing was repeated three times with water (500 g). Next, washing was performed three times with a 20 wt% ammonium chloride aqueous solution (500 g), and then water washing was further repeated three times with water (500 g). Finally, drying was performed to obtain an inorganic molded body SP (1).

<Production of inorganic porous substrate>

(Production Example 2)

3-Aminopropyldiisopropylethoxysilane (87 mg) and toluene (12.43 g) were mixed in a glass vial to prepare a 3-aminopropyldiisopropylethoxysilane/toluene solution. The inorganic molded body SP (1) (7.13 g) was placed in a round-bottom flask, toluene (61 g) was added thereto, and then the prepared 3-aminopropyldiisopropylethoxysilane/toluene solution (3.11 g) was added thereto under stirring at room temperature. The round-bottom flask was heated in an oil bath and refluxed for 11.5 hours. Thereafter, the mixture was once cooled to room temperature, left to stand for 10 hours, and then refluxed for another 5 hours. The reaction mixture was filtered, and the solid content was washed with toluene and then dried under reduced pressure to obtain an inorganic porous substrate precursor 1.

(Example 1)

The inorganic porous substrate precursor 1 (0.40 g) was placed in a round-bottom flask, and toluene (61 g) was added thereto. A mixture of N,N-diisopropylethylamine (210 mg) and toluene (4.0 g) was further added under stirring at

room temperature, then a mixture of tributylchlorosilane (379 mg) and toluene (4.0 g) was added, and the flask was heated in an oil bath and refluxed for 5 hours.
Thereafter, the reaction solution was filtered, and the solid content was washed with a 5 vol% N,N-diisopropylethylamine/ethanol solution (13 mL), and then washed with tetrahydrofuran (14 mL). The washed product was dried under reduced pressure to obtain an inorganic porous substrate 1.

(Example 2)

An inorganic porous substrate 2 was obtained by performing synthesis in the same manner as in Example 1 except that chloro (hexyl) dimethylsilane (287 mg) was used in place of tributylchlorosilane (379 mg) used in Example 1.

(Example 3)

An inorganic porous substrate 3 was obtained by performing synthesis in the same manner as in Example 1 except that benzylchlorodimethylsilane (293 mg) was used in place of tributylchlorosilane (379 mg) used in Example 1.

(Example 4)

An inorganic porous substrate 4 was obtained by performing synthesis in the same manner as in Example 1 except that (3-cyanopropyl) dimethylchlorosilane (261 mg) was used in place of tributylchlorosilane (379 mg) used in Example 1.

(Example 5)

An inorganic porous substrate 5 was obtained by performing synthesis in the same manner as in Example 1 except that 2-acetoxyethyldimethylchlorosilane (290 mg) was used in place of tributylchlorosilane (379 mg) used in Example 1.

(Reference Example 1)

The inorganic porous substrate precursor 1 (0.40 g) was placed in a round-bottom flask, and toluene (61 g) was added thereto. A mixture of hexamethyldisilazane (262 mg) and toluene (4.0 g) was added thereto under stirring at room temperature, and the flask was heated in an oil bath and refluxed for 5 hours. Thereafter, the reaction solution was filtered, and the solid content was washed with a 5 vol% N,N-diisopropylethylamine/ethanol solution (13 mL), and then washed with tetrahydrofuran (14 mL). The washed product was dried under reduced pressure to obtain an inorganic porous substrate 6.

(Reference Example 2)

An inorganic porous substrate 7 was obtained by performing synthesis in the same manner as in Reference Example 1 except that trimethoxy (methyl) silane (52 mg) was used in place of hexamethyldisilazane (262 mg) used in Reference Example 1.

(Reference Example 3)

An inorganic porous substrate 8 was obtained by performing synthesis in the same manner as in Reference Example 1 except that triethoxyphenylsilane (57 mg) was used in place of hexamethyldisilazane (262 mg) used in Reference Example 1.

(Reference Example 4)

An inorganic porous substrate 9 was obtained by performing synthesis in the same manner as in Reference Example 1 except that 2-(4-pyridylethyl) triethoxysilane (61 mg) was used in place of hexamethyldisilazane (262 mg) used in Reference Example 1.

<Production of inorganic porous support>

(Example 6)

In a glass vial, U-succinate (5'-O-dimethoxytrityl-2'-O-tert-butyldimethylsilyl-3'-O-succinyluridine) (311.3 mg), 1-[bis(dimethylamino)methylene]-1H-1,2,3-benzotriazolium 3-oxid hexafluorophosphate (HBTU) (155.2 mg), N,N-diisopropylethylamine (90.1 μL), and acetonitrile (25 mL) were mixed. The prepared mixed solution (3.08 mL) and the inorganic porous substrate 1 (300 mg) were added to a test tube. The mixture was left to stand at 25°C for 18 hours and then filtered, and a solid component was washed with acetonitrile (10 mL). A tetrahydrofuran solution of acetic anhydride and 2,6-lutidine (acetic anhydride/2,6-lutidine/tetrahydrofuran, volume ratio: 1/1/8) (1 mL) and a tetrahydrofuran solution of 1-methylimidazole (1-methylimidazole/tetrahydrofuran, volume ratio: 16/84) (1 mL) were added to the solid content after washing. The mixture was left to stand for 1 minute and then filtered, and the solid content was washed with acetonitrile (10 mL). The solid content after washing was vacuum-dried to obtain an inorganic porous support 1 supporting a group having a nucleoside structure.

[Chemical Formula 58]

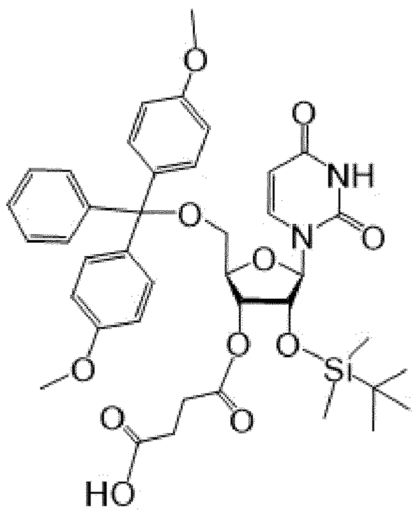

(U-succinate)

(Example 7)

A reaction was performed according to Example 6 using the inorganic porous substrate 2 instead of the inorganic porous substrate 1 to obtain an inorganic porous support 2.

(Example 8)

A reaction was performed according to Example 6 using the inorganic porous substrate 3 instead of the inorganic porous substrate 1 to obtain an inorganic porous support 3.

(Example 9)

A reaction was performed according to Example 6 using the inorganic porous substrate 4 instead of the inorganic porous substrate 1 to obtain an inorganic porous support 4.

(Example 10)

A reaction was performed according to Example 6 using the inorganic porous substrate 5 instead of the inorganic porous substrate 1 to obtain an inorganic porous support 5.

(Comparative Example 1)

A reaction was performed according to Example 6 using the inorganic porous substrate precursor 1 instead of the

inorganic porous substrate 1 to obtain an inorganic porous support 6. The data of Comparative Example 1 is data in the case of no cap.

(Reference Example 5)

A reaction was performed according to Example 6 using the inorganic porous substrate 6 instead of the inorganic porous substrate 1 to obtain an inorganic porous support 7.

(Reference Example 6)

A reaction was performed according to Example 6 using the inorganic porous substrate 7 instead of the inorganic porous substrate 1 to obtain an inorganic porous support 8.

(Reference Example 7)

A reaction was performed according to Example 6 using the inorganic porous substrate 8 instead of the inorganic porous substrate 1 to obtain an inorganic porous support 9.

(Reference Example 8)

A reaction was performed according to Example 6 using the inorganic porous substrate 9 instead of the inorganic porous substrate 1 to obtain an inorganic porous support 10.

For the obtained series of inorganic molded bodies, inorganic porous substrate precursors, inorganic porous substrates, and inorganic porous supports, the pore diameter by a mercury intrusion method, the particle diameter by scanning electron microscope measurement, the cumulative pore volume in the pore diameter range of 40 nm to 1000 nm, the specific surface area by a nitrogen adsorption method, the active NH group loading, the silyl group (B) loading, and the nucleoside loading were each measured using the above-described methods. The results are shown in Table 2 described later.

<Solid-phase synthesis of oligonucleic acid>

The oligonucleotide consisting of the following sequence (A) was synthesized from the 3' side to the 5' side according to the phosphoramidite method by using a nucleic acid synthesizer (trade name: NTS M-4-MX-E, produced by Nihon Techno Service Co., Ltd.) (see the reaction route (condensation reaction, oxidation, and deprotection)). For such solid-phase synthesis, the inorganic porous support produced above was used.

As the amidite monomer, the adenosine EMM amidite (described in Example 4 of US 2012/035246 A1), the cytidine EMM amidite (described in Example 3 of the same US patent literature), the guanosine EMM amidite (described in Example 5 of the same US patent literature), and the uridine EMM amidite (described in Example 2 of the same US patent literature) as shown below were used.

Sequence (A): 5'-

AUAACUCAAUUUGUAAAAAAGUUUUAGAGCUAGAAAUAGCAAGUUAAAAUAAGGCUAGU

CCGUUAUCAACUUGAAAAAGUGGCACCGAGUCGGUGCUUUU-3' (SEQ ID NO: 1)

[Chemical Formula 59]

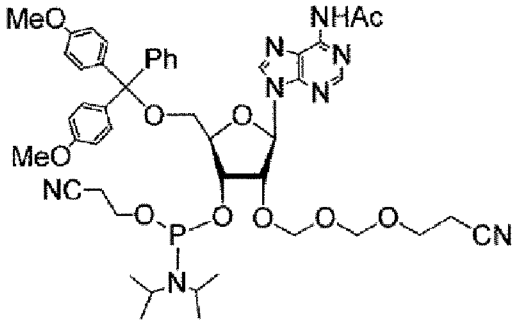

Adenosine EMM

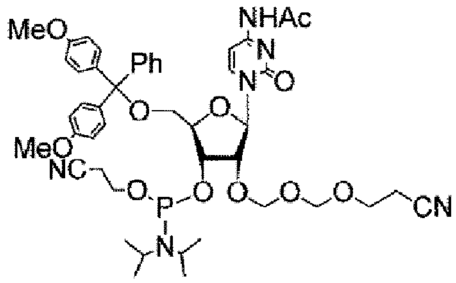

Cytidine EMM

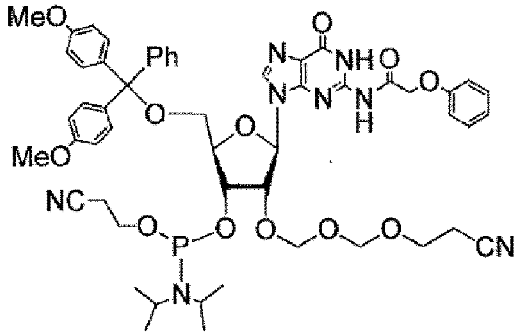

Guanosine EMM

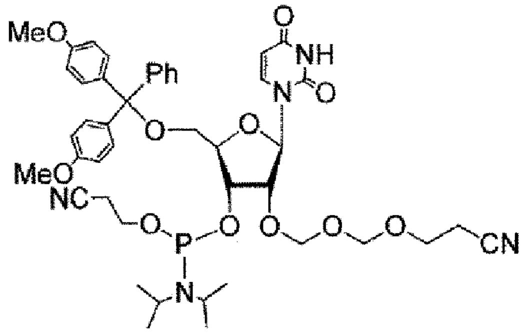

Uridine EMM

In the solid-phase synthesis, a high-purity trichloroacetic acid toluene solution was used as a deblocking solution, 5-benzylmercapto-1H-tetrazole was used as a condensing agent, an iodine solution was used as an oxidizing agent, and a phenoxyacetic acid solution and a 1-methyl imidazole solution were used as a capping solution.

The inorganic porous support after the completion of synthesis was placed in a glass vial with a lid, and a solution of 28% aqueous ammonia and EtOH at a ratio of 1 : 1 to 2 : 1 was added thereto. Thereafter, the mixture was left to stand at 40°C for 4 hours. The solution after the reaction was filtered and washed with water and EtOH. The resulting solution was dried to obtain a crude oligonucleotide having a protected group, and then the crude oligonucleotide was deprotected by the treatment with tetra-n-butyl ammonium fluoride (TBAF) in the presence of nitromethane to obtain a crude product.

[Measurement of purity of oligonucleic acid]

The solution prepared using the obtained crude oligonucleotide was separated into components by high performance liquid chromatography HPLC (wavelength: 260 nm, column DNAPac (trademark) PA100 4 x 250 mm), and a peak width at 10% of a height of an LC peak vertex of a main product in the obtained chromatogram was determined as "10% width".

In order to verify the effect by each inorganic porous support, a value obtained by dividing the value of "10% width" in each inorganic porous support by the value of "10% width" in the inorganic porous support 6 (Comparative Example 2) having no silyl group (B) was defined as "relative 10% width" and calculated. Here, when the purity is high, the "relative 10% width" is a small value, and when the purity is low, the "relative 10% width" is a large value.

(Example 11)

Using the inorganic porous support 1, solid-phase synthesis of the oligonucleic acid was performed according to the above method for the sequence (A).

(Example 12)

Using the inorganic porous support 2, solid-phase synthesis of the oligonucleic acid was performed according to the above method for the sequence (A).

(Example 13)

Using the inorganic porous support 3, solid-phase synthesis of the oligonucleic acid was performed according to the above method for the sequence (A).

(Example 14)

Using the inorganic porous support 4, solid-phase synthesis of the oligonucleic acid was performed according to the above method for the sequence (A).

(Example 15)

Using the inorganic porous support 5, solid-phase synthesis of the oligonucleic acid was performed according to the above method for the sequence (A).

(Comparative Example 2)

Using the inorganic porous support 6, solid-phase synthesis of the oligonucleic acid was performed according to the above method for the sequence (A). The data of Comparative Example 2 is data in the case of no cap.

(Reference Example 9)

Using the inorganic porous support 7, solid-phase synthesis of the oligonucleic acid was performed according to the above method for the sequence (A).

(Reference Example 10)

Using the inorganic porous support 8, solid-phase synthesis of the oligonucleic acid was performed according to the above method for the sequence (A).

(Reference Example 11)

Using the inorganic porous support 9, solid-phase synthesis of the oligonucleic was performed according to the above method for the sequence (A).

(Reference Example 12)

Using the inorganic porous support 10, solid-phase synthesis of the oligonucleic acid was performed according to the above method for the sequence (A).

The results of the solid-phase synthesis of the oligonucleic acids of a series of the sequences (A) are shown in Table 1.

[Table 1]

| | Inorganic porous substrate | Inorganic material of inorganic porous substrate | Inorganic porous support | Pore diameter (nm) | Particle diameter (μm) | Cumulative pore volume in pore diameter range of 40 nm to 1000 nm (mL/g) | Specific surface area (m2/g) | Silyl group (A) | Active NH group loading in inorganic porous substrate (μmol/-m2) | Silyl group (B) | Silyl group (B) loading (μmol/-m2) | Nucleoside loading in inorganic porous support (μmol/m2) | Oligonucleic acid strand length | Relative 10% width |
|---|---|---|---|---|---|---|---|---|---|---|---|---|---|---|
| Example 11 | Inorganic porous substrate 1 | Zeolite | Inorganic porous support 1 | 109 | 48.9 | 0.88 | 24.7 | 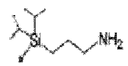 | 0.67 | *-Si($C_4H_9$)$_3$ | 0.92 | 0.40 | 100mar (RNA) | 0.73 |
| Example 12 | Inorganic porous substrate 2 | Zeolite | Inorganic porous support 2 | 109 | 48.9 | 0.88 | 24.7 | 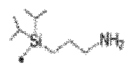 | 0.67 | 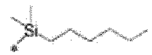 | 1. 95 | 0.41 | 100mer (RNA) | 0.77 |
| Example 13 | Inorganic porous substrate 3 | Zeolite | Inorganic porous support 3 | 109 | 48.9 | 0.88 | 24.7 | 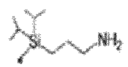 | 0.67 | 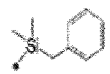 | 1.40 | 0.43 | 100mer (RNA) | 0.59 |
| Example 14 | Inorganic porous substrate 4 | Zeolite | Inorganic porous support 4 | 109 | 48.9 | 0.88 | 24.7 | 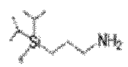 | 0.67 | 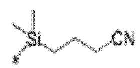 | 2. 64 | 0.43 | 100mer (RNA) | 0.78 |
| Example 15 | Inorganic porous substrate 5 | Zeolite | Inorganic porous support 5 | 109 | 48.9 | 0.88 | 24.7 | 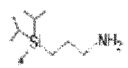 | 0.67 | 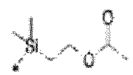 | 2.62 | 0.41 | 100mer (RNA) | 0.81 |
| Comparative Example 2 | (Inorganic porous substrate precursor 1) | Zeolite | Inorganic porous support 6 | 109 | 48.9 | 0.88 | 24.7 | 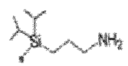 | 0.67 | - | - | 0.46 | 100mer (RNA) | 1.00 |

(continued)

| | Inorganic porous substrate | Inorganic material of inorganic porous substrate | Inorganic porous support | Pore diameter (nm) | Particle diameter (μm) | Cumulative pore volume in pore diameter range of 40 nm to 1000 nm (mL/g) | Specific surface area (m2/g) | Silyl group (A) | Active NH group loading in inorganic porous substrate (μmol/-m2) | Silyl group (B) | Silyl group (B) loading (μmol/-m2) | Nucleoside loading in inorganic porous support (μmol/m2) | Oligonucleic acid strand length | Relative 10% width |
|---|---|---|---|---|---|---|---|---|---|---|---|---|---|---|
| Reference Example 9 | Inorganic porous substrate 6 | Zeolite | Inorganic porous support 7 | 109 | 48.9 | 0.88 | 24.7 | 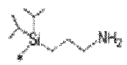 | 0.67 | *-$Si(CH_3)_3$ | 2.71 | 0.45 | 100mer (RNA) | 1.29 |
| Reference Example 10 | Inorganic porous substrate 7 | Zeolite | Inorganic porous support 8 | 109 | 48.9 | 0.88 | 24.7 | 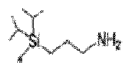 | 0.67 | 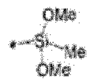 | 0.21 | 0.38 | 100mer (RNA) | 1.44 |
| Reference Example 11 | Inorganic porous substrate 8 | Zeolite | Inorganic porous support 9 | 109 | 48.9 | 0.88 | 24.7 | 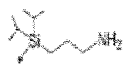 | 0.67 | 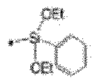 | 0.04 | 0.38 | 100mer (RNA) | 0.97 |
| Reference Example 12 | Inorganic porous substrate 9 | Zeolite | Inorganic porous support 10 | 109 | 48.9 | 0.88 | 24.7 | 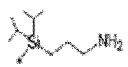 | 0.67 | 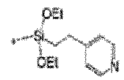 | 0.37 | 0.39 | 100mer (RNA) | 1.00 |

[In Table 1, * represents a bond with the inorganic porous substrate, a bond with the inorganic porous support, or a bond with the inorganic porous substrate precursor.]

In the solid-phase synthesis result of the oligonucleic acid of the sequence (A), the inorganic porous support used in Examples 11 to 15 showed a lower relative 10% width as compared with the inorganic porous support used in Comparative Example 2 and Reference Examples 9 to 12, and it was found that the obtained oligonucleic acid had a higher purity.

INDUSTRIAL APPLICABILITY

According to the present invention, there are provided the inorganic porous support, the inorganic porous substrate as the raw material of the inorganic porous support, and the oligonucleic acid production method, which can improve the purity of the oligonucleic acid in production of the oligonucleic acid. The oligonucleic acid obtained by the present invention is useful as a raw material for pharmaceuticals and the like.

Sequence Listing Free Text

SEQ ID NO: 1 in the Sequence Listing represents the base sequence of an oligonucleotide produced according to the production method of the present invention.

## Claims

1. An inorganic porous substrate that comprises silyl groups represented by the following (i) and (ii) and has the following characteristics (iii) to (v):

(i) a silyl group (A): a silyl group represented by the following formula (i-1),
(ii) a silyl group (B): at least one silyl group selected from the group consisting of silyl groups represented by the following formulas (ii-1), (ii-2), and (ii-3),
(iii) a particle diameter of 1 μm or more, as determined by scanning electron microscopy as an average value of the major diameters of 50 arbitrary particles,
(iv) a pore diameter of 20 nm or more, as determined by the mercury intrusion method, and
(v) a cumulative pore volume in a pore diameter range of 40 nm to 1000 nm of more than 0.32 mL/g and 4 mL/g or less, as determined by the mercury intrusion method:

$$(X1)(Y1)_a(Z1)_bSi\text{-}A1\text{-}NH\text{-}B1 \cdots \qquad (i - 1)$$

wherein in the formula (i-1),
X1 represents a bond with the inorganic porous substrate,
Y1 each independently represents any one selected from the group consisting of a bond with the inorganic porous substrate, a hydroxyl group, an amino group, an alkoxy group having 1 to 6 carbon atoms, and an alkylamino group having 1 to 12 carbon atoms,
Z1 each independently represents an alkyl group having 1 to 6 carbon atoms or an aryl group having 6 to 12 carbon atoms,
a represents an integer represented by 2-b,
b represents an integer of 0 to 2,
A1 represents an organic group having 1 to 20 carbon atoms, and
B1 represents any one selected from the group consisting of a hydrogen atom, an alkyl group having 1 to 6 carbon atoms, and an aryl group having 6 to 12 carbon atoms,

$$(P1)(Q1)(R1)Si\text{-}J1 \cdots \qquad (ii - 1),$$

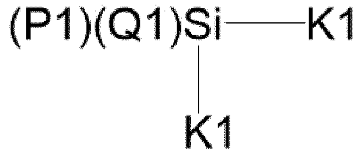

・・・ (ii−2)

,

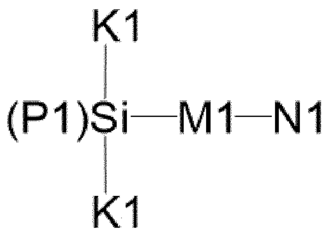

・・・ (ii−3)

,

wherein in the formula (ii-1), (ii-2), or (ii-3),
P1 represents a bond with the inorganic porous substrate,
Q1 and R1 each independently represents any one selected from the group consisting of a bond with the inorganic porous substrate, a hydroxyl group, an amino group, an alkoxy group having 1 to 6 carbon atoms, and an alkylamino group having 1 to 12 carbon atoms,
J1 represents an alkyl group having 2 to 20 carbon atoms or an aryl group having 7 to 20 carbon atoms,
K1 each independently represents an alkyl group having 1 to 20 carbon atoms or an aryl group having 6 to 20 carbon atoms,
M1 represents an alkylene group having 1 to 6 carbon atoms, and
N1 represents an alkyl group having 2 to 20 carbon atoms or an aryl group having 6 to 20 carbon atoms.

**2.** The inorganic porous substrate according to claim 1, wherein the inorganic porous substrate has a pore diameter of from 40 nm to 500 nm, as determined by the mercury intrusion method.

**3.** The inorganic porous substrate according to any one of claims 1 to 2, wherein the inorganic porous substrate has a specific surface area of from 0.1 $m^2/g$ to 200 $m^2/g$, as determined by nitrogen adsorption/desorption isotherm measurement.

**4.** The inorganic porous substrate according to any one of claims 1 to 3, comprising silica, silica gel, zeolite, or glass.

**5.** The inorganic porous substrate according to any one of claims 1 to 4, wherein the inorganic porous substrate comprises an active NH group represented by the following formula (NH-1), wherein the amount of the active NH group satisfies the following mathematical formula (NH#1) :

Si-A1-NH-B1 ··· (NH- 1)

wherein in the formula (NH-1),
A1 represents an organic group having 1 to 20 carbon atoms,
B1 represents any one selected from the group consisting of a hydrogen atom, an alkyl group having 1 to 6 carbon atoms, and an aryl group having 6 to 12 carbon atoms

$$0.05 \leq I1/S1 \leq 6.0 \quad \cdots \quad (NH\#1)$$

I1: the amount of the active NH group in the inorganic porous substrate (μmol/g), and
S1: the specific surface area ($m^2/g$) of the inorganic porous substrate obtained by nitrogen adsorption/desorption isotherm measurement.

**6.** The inorganic porous substrate according to any one of claims 1 to 5, wherein the silyl group (B) is represented by the formula (ii-3).

7. The inorganic porous substrate according to any one of claims 1 to 6, wherein the silyl group represented by the formula (ii-3) is a silyl group represented by the following formula (ii-3-1):

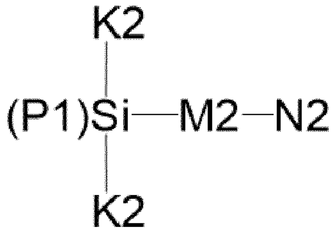

・・・ (ii-3-1)

wherein in the formula (ii-3-1),
P1 represents the bond with the inorganic porous substrate,
K2 each independently represents an alkyl group having 1 to 6 carbon atoms or an aryl group having 6 to 10 carbon atoms,
M2 represents an alkylene group having 1 to 4 carbon atoms, and
N2 represents an alkyl group having 2 to 6 carbon atoms or an aryl group having 6 to 10 carbon atoms.

8. The inorganic porous substrate according to any one of claims 1 to 7, wherein A1 in the formula (i-1) is an alkylene group having 1 to 20 carbon atoms which may optionally contain any one or more of an imino group, an oxy group, and a thio group.

9. The inorganic porous substrate according to any one of claims 1 to 8, wherein the silyl group (A) is represented by the following formula (i-1-1):

$(X1)(Z1)_2Si\text{-}A2\text{-}NH\text{-}B2$ ··· (i-1-1)

wherein in the formula (i-1-1),
X1 represents the bond with the inorganic porous substrate,
Z1 each independently represents an alkyl group having 1 to 6 carbon atoms or an aryl group having 6 to 12 carbon atoms,
A2 represents an alkylene group having 1 to 15 carbon atoms which may optionally contain any one or more of an imino group, an oxy group, and a thio group, and
B2 represents any one selected from the group consisting of a hydrogen atom and an alkyl group having 1 to 2 carbon atoms.

10. An inorganic porous support that comprises silyl groups of the following (vi) and (vii) and has the following characteristic (viii):

(vi) a silyl group (C): a silyl group represented by the following formula (vi-1),
(vii) a silyl group (D): at least one silyl group selected from the group consisting of silyl groups represented by the following formulas (vii-1), (vii-2), and (vii-3), and
(viii) a pore diameter of 20 nm or more, as determined by the mercury intrusion method:

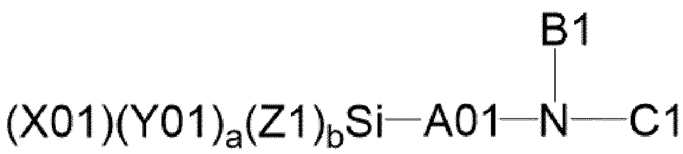

・・・(vi-1)

wherein in the formula (vi-1),
X01 represents a bond with the inorganic porous support,
Y01 each independently represents any one selected from the group consisting of the bond with the inorganic

porous support, a hydroxyl group, an amino group, an alkoxy group having 1 to 6 carbon atoms, and an alkylamino group having 1 to 12 carbon atoms,
Z1 each independently represents an alkyl group having 1 to 6 carbon atoms or an aryl group having 6 to 12 carbon atoms,
a represents an integer represented by 2-b,
b represents an integer of 0 to 2,
A01 represents an organic group having 1 to 20 carbon atoms,
B1 represents any one selected from the group consisting of a hydrogen atom, an alkyl group having 1 to 6 carbon atoms, and an aryl group having 6 to 12 carbon atoms, and
C1 represents a group having a nucleoside or nucleotide structure in which a reactive group is protected or deprotected,

(P01)(Q01)(R01)Si-J1 ··· (vii - 1) ,

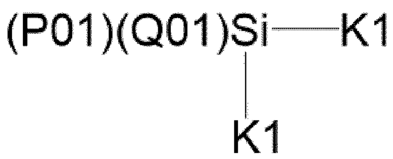
・・・ (vii－2)
,

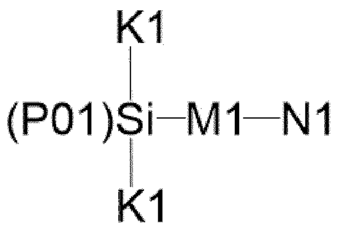
・・・ (vii－3)
,

wherein in the formula (vii-1), (vii-2), or (vii-3),
P01 represents the bond with the inorganic porous support,
Q01 and R01 each independently represents any one selected from the group consisting of the bond with the inorganic porous support, a hydroxyl group, an amino group, an alkoxy group having 1 to 6 carbon atoms, and an alkylamino group having 1 to 12 carbon atoms,
J1 represents an alkyl group having 2 to 20 carbon atoms or an aryl group having 7 to 20 carbon atoms,
K1 each independently represents an alkyl group having 1 to 20 carbon atoms or an aryl group having 6 to 20 carbon atoms,
M1 represents an alkylene group having 1 to 6 carbon atoms, and
N1 represents an alkyl group having 2 to 20 carbon atoms or an aryl group having 6 to 20 carbon atoms.

**11.** The inorganic porous support according to claim 10, wherein the inorganic porous support has a pore diameter of from 40 nm to 500 nm, as determined by the mercury intrusion method.

**12.** The inorganic porous support according to any one of claims 10 to 11, wherein the inorganic porous support has a cumulative pore volume in a pore diameter range of 40 nm to 1000 nm of more than 0.32 mL/g and 4 mL/g or less, as determined by the mercury intrusion method.

**13.** The inorganic porous support according to any one of claims 10 to 12, wherein the inorganic porous support has a specific surface area of 0.1 $m^2/g$ or more and 200 $m^2/g$ or less, as determined by nitrogen adsorption/desorption isotherm measurement.

**14.** The inorganic porous support according to any one of claims 10 to 13, wherein the inorganic porous support comprises an inorganic porous body which is composed of silica, silica gel, zeolite, or glass.

**15.** The inorganic porous support according to any one of claims 10 to 14, wherein an amount of a group containing a nucleoside or a nucleotide structure in which a reactive group is protected or deprotected satisfies the following

mathematical formula (Nu#1):

$$0.05 \leq I01/S01 \leq 3.0 \cdots (Nu\#1)$$

I01: a group (μmol/g) having a nucleoside or nucleotide structure in which a reactive group is protected or deprotected in the inorganic porous support, and
S01: a specific surface area ($m^2/g$) of the inorganic porous support obtained by nitrogen adsorption/desorption isotherm measurement.

16. The inorganic porous support according to any one of claims 10 to 15, wherein C1 in the general formula (vi-1) contains a succinyl linker.

17. The inorganic porous support according to any one of claims 10 to 16, comprising a silyl group represented by the formula (vii-3) as a silyl group (D).

18. The inorganic porous support according to any one of claims 10 to 17, wherein the silyl group represented by the formula (vii-3) is a silyl group represented by the following formula (vii-3-1):

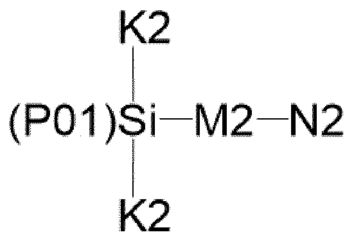

· · · (vii − 3 − 1)

wherein in the formula (ii-3-1),
P01 represents the bond with the inorganic porous support,
K2 each independently represents an alkyl group having 1 to 6 carbon atoms or an aryl group having 6 to 10 carbon atoms,
M2 represents an alkylene group having 1 to 4 carbon atoms, and
N2 represents an alkyl group having 2 to 6 carbon atoms or an aryl group having 6 to 10 carbon atoms.

19. The inorganic porous support according to any one of claims 10 to 18, wherein A01 in the formula (vi-1) is an alkylene group having 1 to 20 carbon atoms which may optionally contain any one or more of an acylimino group, an oxy group, and a thio group.

20. The inorganic porous support according to any one of claims 10 to 19, wherein the silyl group (C) is represented by the following formula (vi-1-1):

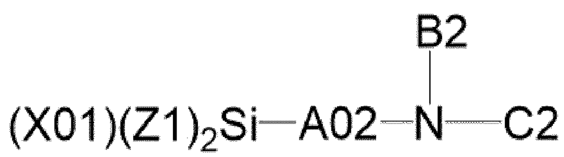

· · · (vi − 1 − 1)

wherein in the formula (vi-1-1),
X01 represents the bond with the inorganic porous support,
Z1 each independently represents an alkyl group having 1 to 6 carbon atoms or an aryl group having 6 to 12 carbon atoms,
A02 represents an alkylene group having 1 to 15 carbon atoms which may optionally contain any one or more of an acylimino group, an oxy group, and a thio group,
B2 represents any one selected from the group consisting of a hydrogen atom and an alkyl group having 1 to 2 carbon atoms, and
C2 represents a group including a succinyl linker and having a nucleoside or nucleotide structure in which a

reactive group is protected or deprotected.

**21.** A nucleic acid production method using the inorganic porous support according to any one of claims 10 to 20, in which C1 in the formula (vi-1) has a group having nucleoside or nucleotide structure in which the hydroxyl group as a reactive group is protected, the nucleic acid production method comprising:

a step (A) of deprotecting a protecting group of the hydroxyl group at the 5'-position of the nucleoside;
a step (B) of producing a phosphite by subjecting the hydroxyl group at the 5'-position of the nucleoside produced in the step (A) to a condensation reaction with an amidite compound having a second nucleoside base;
a step (C) of oxidizing the phosphite produced in the step (B) to produce a nucleotide; and
a step (D) of deprotecting the protecting group of the hydroxyl group at the 5'-position of the nucleotide produced in the step (C).

**22.** The nucleic acid production method according to claim 21, further comprising:

a step (B') of further subjecting a product produced in the step (D) to a condensation reaction with an amidite compound having a nucleoside base to be introduced next to produce a phosphite;
a step (C') of oxidizing the phosphite produced in the step (B') to produce an oligonucleotide; and
a step (D') of deprotecting the protecting group for the hydroxyl group at the 5'-position of a terminal of an oligonucleotide chain produced in the step (C').

**23.** The nucleic acid production method according to claim 22, comprising a step (E) of further repeating a series of steps including the step (B'), the step (C'), and the step (D') m times (m represents an integer of 1 or more) to react m amidite compounds, and then cutting out an elongated nucleic acid.

**24.** Use of the inorganic porous support according to any one of claims 10 to 20 in production of a nucleic acid by a phosphoramidite method.

## Patentansprüche

**1.** Anorganisches poröses Substrat, das Silylgruppen umfasst, die durch die folgenden Punkte (i) und (ii) dargestellt sind, und die folgenden Eigenschaften (iii) bis (v) aufweist:

(i) eine Silylgruppe (A): eine Silylgruppe, die durch die folgende Formel (i-1) dargestellt ist
(ii) eine Silylgruppe (B): mindestens eine Silylgruppe, ausgewählt aus der Gruppe bestehend aus Silylgruppen, die durch die folgenden Formeln (ii-1), (ii-2) und (ii-3) dargestellt sind,
(iii) einen Partikeldurchmesser von 1 μm oder mehr, bestimmt durch Rasterelektronenmikroskopie als Mittelwert der größten Durchmesser von 50 beliebigen Partikeln,
(iv) einen Porendurchmesser von 20 nm oder mehr, wie mit der Quecksilberporosimetrie bestimmt wurde, und
(v) ein kumulatives Porenvolumen im Porendurchmesserbereich von 40 nm bis 1000 nm oder mehr als 0,32 mL/g und 4 mL/g oder weniger, wie mit der Quecksilberporosimetrie bestimmt wurde:

$$(X1)(Y1)_a(Z1)_bSi\text{-}A1\text{-}NH\text{-}B1 \cdots \qquad (i - 1)$$

wobei in der Formel (i-1)
X1 eine Bindung mit dem anorganischen porösen Substrat darstellt,
Y1 jeweils unabhängig eines, ausgewählt aus der Gruppe darstellt, die aus einer Bindung mit dem anorganischen porösen Substrat, einer Hydroxylgruppe, einer Aminogruppe, einer Alkoxygruppe mit 1 bis 6 Kohlenstoffatomen und einer Alkylaminogruppe mit 1 bis 12 Kohlenstoffatomen besteht,
Z1 jeweils unabhängig eine Alkylgruppe mit 1 bis 6 Kohlenstoffatomen oder eine Arylgruppe mit 6 bis 12 Kohlenstoffatomen darstellt,
a eine ganze Zahl darstellt, die durch 2-b dargestellt ist,
b eine ganze Zahl von 0 bis 2 darstellt,
A1 eine organische Gruppe mit 1 bis 20 Kohlenstoffatomen darstellt und
B1 eines, ausgewählt aus der Gruppe darstellt, die aus einem Wasserstoffatom, einer Alkylgruppe mit 1 bis 6 Kohlenstoffatomen und einer Arylgruppe mit 6 bis 12 Kohlenstoffatomen besteht,

(P1)(Q1)(R1)Si-J1 ⋯ (ii - 1),

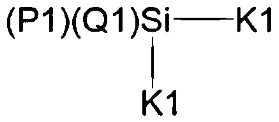

・・・ (ii − 2) ,

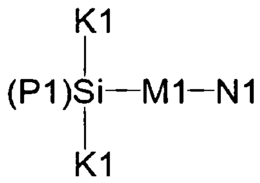

・・・ (ii − 3) ,

wobei in der Formel (ii-1), (ii-2) oder (ii-3)
P1 eine Bindung mit dem anorganischen porösen Substrat darstellt,
Q1 und R1 jeweils unabhängig eines, ausgewählt aus der Gruppe darstellen, die aus einer Bindung mit dem anorganischen porösen Substrat, einer Hydroxylgruppe, einer Aminogruppe, einer Alkoxygruppe mit 1 bis 6 Kohlenstoffatomen und einer Alkylaminogruppe mit 1 bis 12 Kohlenstoffatomen besteht,
J1 eine Alkylgruppe mit 2 bis 20 Kohlenstoffatomen oder eine Arylgruppe mit 7 bis 20 Kohlenstoffatomen darstellt,
K1 jeweils unabhängig eine Alkylgruppe mit 1 bis 20 Kohlenstoffatomen oder eine Arylgruppe mit 6 bis 20 Kohlenstoffatomen darstellt,
M1 eine Alkylengruppe mit 1 bis 6 Kohlenstoffatomen darstellt und
N1 eine Alkylgruppe mit 2 bis 20 Kohlenstoffatomen oder eine Arylgruppe mit 6 bis 20 Kohlenstoffatomen darstellt.

2. Anorganisches poröses Substrat nach Anspruch 1, wobei das anorganische poröse Substrat einen Porendurchmesser von 40 nm bis 500 nm aufweist, wie mit der Quecksilberporosimetrie bestimmt wurde.

3. Anorganisches poröses Substrat nach einem der Ansprüche 1 bis 2, wobei das anorganische poröse Substrat eine spezifische Oberfläche von 0,1 $m^2/g$ bis 200 $m^2/g$ aufweist, die durch Stickstoffadsorption/-desorption-Isothermenmessung bestimmt wird.

4. Anorganisches poröses Substrat nach einem der Ansprüche 1 bis 3, umfassend Siliciumdioxid, Kieselgel, Zeolith oder Glas.

5. Anorganisches poröses Substrat nach einem der Ansprüche 1 bis 4, wobei das anorganische poröse Substrat eine aktive NH-Gruppe, dargestellt durch die folgende Formel (NH-1), umfasst, wobei die Menge der aktiven NH-Gruppe die folgende mathematische Formel (NH#1) erfüllt:

Si-A1-NH-B1 ⋯ (NH- 1)

wobei in der Formel (NH-1)
A1 eine organische Gruppe mit 1 bis 20 Kohlenstoffatomen darstellt,
B1 eines, ausgewählt aus der Gruppe darstellt, die, aus einem Wasserstoffatom, einer Alkylgruppe mit 1 bis 6 Kohlenstoffatomen und einer Arylgruppe mit 6 bis 12 Kohlenstoffatomen besteht

$$0{,}05 \leq I1/S1 \leq 6{,}0 \cdots (NH\#1)$$

I1: die Menge der aktiven NH-Gruppe im anorganischen porösen Substrat (μmol/g) und
S1: die spezifische Oberfläche ($m^2/g$) des anorganischen porösen Substrats, die durch Stickstoffadsorption/-desorption-Isothermenmessung erhalten wird.

6. Anorganisches poröses Substrat nach einem der Ansprüche 1 bis 5, wobei die Silylgruppe (B) durch die Formel (ii-3) dargestellt ist.

7. Anorganisches poröses Substrat nach einem der Ansprüche 1 bis 6, wobei die Silylgruppe, die durch die Formel (ii-3) dargestellt ist, eine Silylgruppe ist, die durch die folgende Formel (ii-3-1) dargestellt ist:

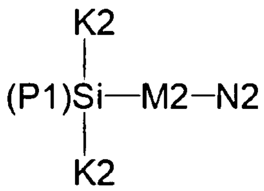

• • • (ii − 3 − 1)

wobei in der Formel (ii-3-1)
P1 die Bindung mit dem anorganischen porösen Substrat darstellt,
K2 jeweils unabhängig eine Alkylgruppe mit 1 bis 6 Kohlenstoffatomen oder eine Arylgruppe mit 6 bis 10 Kohlenstoffatomen darstellt,
M2 eine Alkylengruppe mit 1 bis 4 Kohlenstoffatomen darstellt und
N2 eine Alkylgruppe mit 2 bis 6 Kohlenstoffatomen oder eine Arylgruppe mit 6 bis 10 Kohlenstoffatomen darstellt.

8. Anorganisches poröses Substrat nach einem der Ansprüche 1 bis 7, wobei A1 in der Formel (i-1) eine Alkylengruppe mit 1 bis 20 Kohlenstoffatomen ist, die optional eine oder mehrere von einer Iminogruppe, einer Oxygruppe und/oder einer Thiogruppe enthalten kann.

9. Anorganisches poröses Substrat nach einem der Ansprüche 1 bis 8, wobei die Silylgruppe (A) durch die folgende Formel (i-1-1) dargestellt ist:

$(X1)(Z1)_2Si\text{-}A2\text{-}NH\text{-}B2$ ··· (i - 1 - 1)

wobei in der Formel (i-1-1)
X1 die Bindung mit dem anorganischen porösen Substrat darstellt,
Z1 jeweils unabhängig eine Alkylgruppe mit 1 bis 6 Kohlenstoffatomen oder eine Arylgruppe mit 6 bis 12 Kohlenstoffatomen darstellt,
A2 eine Alkylengruppe mit 1 bis 15 Kohlenstoffatomen darstellt, die optional eine oder mehrere von einer Iminogruppe, einer Oxygruppe und/oder einer Thiogruppe enthalten kann,
B2 eines, ausgewählt aus der Gruppe darstellt, die aus einem Wasserstoffatom und einer Alkylgruppe mit 1 bis 2 Kohlenstoffatomen besteht.

10. Anorganischer poröser Träger, der Silylgruppen der folgenden Punkte (vi) und (vii) umfasst und die folgende Eigenschaft (viii) aufweist:

(vi) eine Silylgruppe (C): eine Silylgruppe, die durch die folgende Formel (vi-1) dargestellt ist,
(vii) eine Silylgruppe (D): mindestens eine Silylgruppe, ausgewählt aus der Gruppe bestehend aus Silylgruppen, die durch die folgenden Formeln (vii-1), (vii-2) und (vii-3) dargestellt sind, und
(viii) einen Porendurchmesser von 20 nm oder mehr, wie mit der Quecksilberporosimetrie bestimmt wurde:

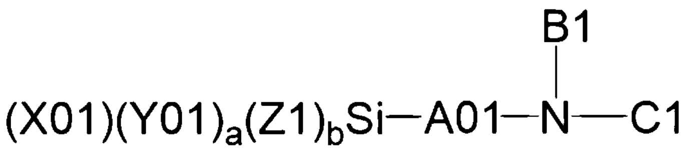

• • • (vi − 1)

wobei in der Formel (vi-1),

X01 eine Bindung mit dem anorganischen porösen Träger darstellt,
Y01 jeweils unabhängig eines, ausgewählt aus der Gruppe darstellt, die aus der Bindung mit dem anorganischen porösen Träger, einer Hydroxylgruppe, einer Aminogruppe, einer Alkoxygruppe mit 1 bis 6 Kohlenstoffatomen und einer Alkylaminogruppe mit 1 bis 12 Kohlenstoffatomen besteht,
Z1 jeweils unabhängig eine Alkylgruppe mit 1 bis 6 Kohlenstoffatomen oder eine Arylgruppe mit 6 bis 12 Kohlenstoffatomen darstellt,
a eine ganze Zahl darstellt, die durch 2-b dargestellt ist,
b eine ganze Zahl von 0 bis 2 darstellt,
A01 eine organische Gruppe mit 1 bis 20 Kohlenstoffatomen darstellt,
B1 eines, ausgewählt aus der Gruppe darstellt, die aus einem Wasserstoffatom, einer Alkylgruppe mit 1 bis 6 Kohlenstoffatomen und einer Arylgruppe mit 6 bis 12 Kohlenstoffatomen besteht, und
C1 eine Gruppe mit einer Nukleosid- oder Nukleotidstruktur darstellt, in der eine reaktive Gruppe geschützt oder entschützt ist,

(P01)(Q01)(R01)Si-J1 ··· (vii - 1),

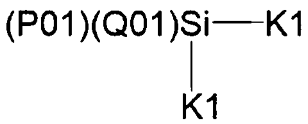 · · · (vii − 2)

,

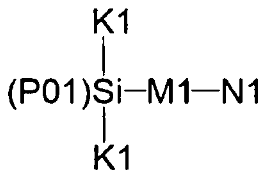 · · · (vii − 3)

,

wobei in der Formel (vii-1), (vii-2) oder (vii-3)
P01 die Bindung mit dem anorganischen porösen Träger darstellt,
Q01 und R01 jeweils unabhängig voneinander eines, ausgewählt aus der Gruppe darstellen, die aus der Bindung mit dem anorganischen porösen Träger, einer Hydroxylgruppe, einer Aminogruppe,
eine Alkoxygruppe mit 1 bis 6 Kohlenstoffatomen und eine Alkylaminogruppe mit 1 bis 12 Kohlenstoffatomen besteht,
J1 eine Alkylgruppe mit 2 bis 20 Kohlenstoffatomen oder eine Arylgruppe mit 7 bis 20 Kohlenstoffatomen darstellt,
K1 jeweils unabhängig eine Alkylgruppe mit 1 bis 20 Kohlenstoffatomen oder eine Arylgruppe mit 6 bis 20 Kohlenstoffatomen darstellt,
M1 eine Alkylengruppe mit 1 bis 6 Kohlenstoffatomen darstellt und
N1 eine Alkylgruppe mit 2 bis 20 Kohlenstoffatomen oder eine Arylgruppe mit 6 bis 20 Kohlenstoffatomen darstellt.

**11.** Anorganischer poröser Träger nach Anspruch 10, wobei der anorganische poröse Träger einen Porendurchmesser von 40 nm bis 500 nm aufweist, wie mit der Quecksilberporosimetrie bestimmt wurde.

**12.** Anorganischer poröser Träger nach einem der Ansprüche 10 oder 11, wobei der anorganische poröse Träger ein kumulatives Porenvolumen im Porendurchmesserbereich von 40 nm bis 1000 nm oder mehr als 0,32 mL/g und 4 mL/g oder weniger aufweist, wie mit der Quecksilberporosimetrie bestimmt wurde.

**13.** Anorganischer poröser Träger nach einem der Ansprüche 10 bis 12, wobei der anorganische poröse Träger eine spezifische Oberfläche von 0,1 $m^2/g$ oder mehr und 200 $m^2/g$ oder weniger aufweist, die durch Stickstoffadsorption/-desorption-Isothermenmessung bestimmt wird.

**14.** Anorganischer poröser Träger nach einem der Ansprüche 10 bis 13, wobei der anorganische poröse Träger einen anorganischen porösen Körper umfasst, der aus Siliciumdioxid, Kieselgel, Zeolith oder Glas gebildet ist.

**15.** Anorganischer poröser Träger nach einem der Ansprüche 10 bis 14, wobei eine Menge einer Gruppe, die ein Nukleosid oder eine Nukleotidstruktur enthält, in der eine reaktive Gruppe geschützt oder entschützt ist, die folgende mathematische Formel (Nu#1) erfüllt:

$$0{,}05 \leq I01/S01 \leq 3{,}0 \cdots \text{(Nu\#1)}$$

I01: eine Gruppe (μmol/g) mit einer Nukleosid- oder Nukleotidstruktur, in der eine reaktive Gruppe im anorganischen porösen Träger geschützt oder entschützt ist, und
S01: eine spezifische Oberfläche ($m^2/g$) des anorganischen porösen Trägers, die durch Stickstoffadsorption/-desorption-Isothermenmessung erhalten wird.

**16.** Anorganischer poröser Träger nach einem der Ansprüche 10 bis 15, wobei C1 in der allgemeinen Formel (vi-1) einen Succinyl-Linker enthält.

**17.** Anorganischer poröser Träger nach einem der Ansprüche 10 bis 16, umfassend eine Silylgruppe, die durch die Formel (vii-3) dargestellt ist, als Silylgruppe (D).

**18.** Anorganischer poröser Träger nach einem der Ansprüche 10 bis 17, wobei die Silylgruppe, die durch die Formel (vii-3) dargestellt ist, eine Silylgruppe der folgenden Formel (vii-3-1) ist:

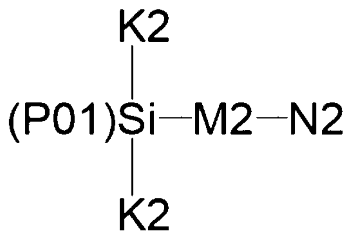

· · · (vii－3－1)

wobei in der Formel (ii-3-1)
P01 die Bindung mit dem anorganischen porösen Träger darstellt,
K2 jeweils unabhängig eine Alkylgruppe mit 1 bis 6 Kohlenstoffatomen oder eine Arylgruppe mit 6 bis 10 Kohlenstoffatomen darstellt,
M2 eine Alkylengruppe mit 1 bis 4 Kohlenstoffatomen darstellt und
N2 eine Alkylgruppe mit 2 bis 6 Kohlenstoffatomen oder eine Arylgruppe mit 6 bis 10 Kohlenstoffatomen darstellt.

**19.** Anorganischer poröser Träger nach einem der Ansprüche 10 bis 18, wobei A01 in der Formel (vi-1) eine Alkylengruppe mit 1 bis 20 Kohlenstoffatomen ist, die optional eine oder mehrere einer Acyliminogruppe, einer Oxygruppe und/oder einer Thiogruppe enthalten kann.

**20.** Anorganischer poröser Träger nach einem der Ansprüche 10 bis 19, wobei die Silylgruppe (C) durch die folgende Formel (vi-1-1) dargestellt ist:

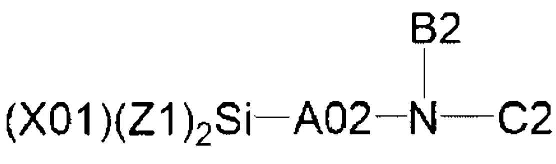

· · · (vi－1－1)

wobei in der Formel (vi-1-1)
X01 die Bindung mit dem anorganischen porösen Träger darstellt,
Z1 jeweils unabhängig eine Alkylgruppe mit 1 bis 6 Kohlenstoffatomen oder eine Arylgruppe mit 6 bis 12 Kohlenstoffatomen darstellt,
A02 eine Alkylengruppe mit 1 bis 15 Kohlenstoffatomen darstellt die optional eine oder mehrere einer Acyliminogruppe, einer Oxygruppe und/oder einer Thiogruppe enthalten kann,
B2 eines, ausgewählt aus der Gruppe darstellt, die aus einem Wasserstoffatom und einer Alkylgruppe mit 1 bis 2

Kohlenstoffatomen besteht, und
C2 eine Gruppe darstellt, die einen Succinyl-Linker enthält und eine Nukleosid- oder Nukleotidstruktur aufweist, in der eine reaktive Gruppe geschützt oder entschützt ist.

**21.** Nukleinsäureerzeugungsverfahren, das den anorganischen porösen Trägers nach einem der Ansprüche 10 bis 20 verwendet, wobei C1 in der Formel (vi-1) eine Gruppe mit Nukleosid- oder Nukleotidstruktur aufweist, in der die Hydroxylgruppe als reaktive Gruppe geschützt ist, wobei das Nukleinsäureerzeugungsverfahren Folgendes umfasst:

einen Schritt (A) zum Abspalten einer Schutzgruppe von der Hydroxylgruppe an der 5'-Position des Nukleosids;
einen Schritt (B) zum Erzeugen eines Phosphits durch Unterwerfen der Hydroxylgruppe an der 5'-Position des im Schritt (A) erzeugten Nukleosids einer Kondensationsreaktion mit einer Amiditverbindung, die eine zweite Nukleosidbase enthält;
einen Schritt (C) zum Oxidieren des im Schritt (B) erzeugten Phosphits zum Erzeugen eines Nukleotids; und
einen Schritt (D) zum Abspalten der Schutzgruppe der Hydroxylgruppe an der 5'-Position des im Schritt (C) erzeugten Nukleotids.

**22.** Nukleinsäureherzeugungsverfahren nach Anspruch 21, ferner umfassend:

einen Schritt (B') zum weiteren Unterwerfen eines im Schritt (D) erzeugten Produkts einer Kondensationsreaktion mit einer Amiditverbindung, die eine Nukleosidbase enthält, die als nächstes eingeführt wird, um ein Phosphit zu erzeugen;
einen Schritt (C') zum Oxidieren des im Schritt (B') erzeugten Phosphits zum Erzeugen eines Oligonukleotids; und
einen Schritt (D') zum Abspalten der Schutzgruppe von der Hydroxylgruppe an der 5'-Position eines Endes einer im Schritt (C') erzeugten Oligonukleotidkette.

**23.** Nukleinsäureerzeugungsverfahren nach Anspruch 22, umfassend einen Schritt (E) zum weiteren Wiederholen einer Reihe von Schritten, die den Schritt (B'), den Schritt (C') und den Schritt (D') beinhaltet, m-mal (m stellt eine ganze Zahl von 1 oder mehr dar), um m Amiditverbindungen reagieren zu lassen, und zum anschließendem Ausschneiden einer verlängerten Nukleinsäure.

**24.** Verwendung des anorganischen porösen Trägers nach einem der Ansprüche 10 bis 20 in der Erzeugung einer Nukleinsäure mittels eines Phosphoramiditverfahrens.

## Revendications

**1.** Substrat poreux inorganique qui comprend des groupes silyles représentés par les éléments (i) et (ii) suivants et qui présente les caractéristiques (iii) à (v) suivantes :

(i) un groupe silyle (A) : un groupe silyle représenté par la formule (i-1) suivante,
(ii) un groupe silyle (B) : au moins un groupe silyle sélectionné dans le groupe constitué par des groupes silyles représentés par les formules (ii-1), (ii-2) et (ii-3) suivantes,
(iii) un diamètre de particule de 1 μm ou plus, tel que déterminé par microscopie électronique à balayage comme valeur moyenne des principaux diamètres de 50 particules arbitraires,
(iv) un diamètre de pore de 20 nm ou plus, tel que déterminé par le procédé d'intrusion de mercure, et
(v) un volume de pore cumulé dans une plage de diamètres de pores de 40 nm à 1000 nm de plus de 0,32 ml/g et de 4 ml/g ou moins, tel que déterminé par le procédé d'intrusion de mercure :

$$(X1)(Y1)_a(Z1)_bSi\text{-}A1\text{-}NH\text{-}B1 \cdots \qquad (i - 1)$$

dans lequel dans la formule (i-1),
X1 représente une liaison avec le substrat poreux inorganique,
Y1 représente indépendamment n'importe quel élément sélectionné dans le groupe constitué par une liaison avec le substrat poreux inorganique, un groupe hydroxyle, un groupe amino, un groupe alcoxy ayant 1 à 6 atomes de carbone et un groupe alkylamino ayant 1 à 12 atomes de carbone,
Z1 représente chacun indépendamment un groupe alkyle ayant 1 à 6 atomes de carbone ou un groupe aryle ayant 6 à 12 atomes de carbone,

a représente un entier représenté par 2-b,
b représente un entier de 0 à 2,
A1 représente un groupe organique ayant 1 à 20 atomes de carbone, et
B1 représente n'importe quel élément sélectionné dans le groupe constitué par un atome d'hydrogène, un groupe alkyle ayant 1 à 6 atomes de carbone et un groupe aryle ayant 6 à 12 atomes de carbone,

(P1)(Q1)(R1)Si-J1 ··· (ii - 1),

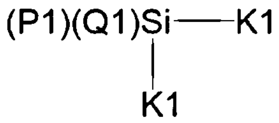

· · · (ii − 2)

,

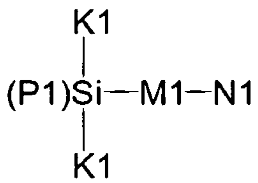

· · · (ii − 3)

,

dans lequel dans la formule (ii-1), (ii-2) ou (ii-3),
P1 représente une liaison avec le substrat poreux inorganique,
Q1 et R1 représentent chacun indépendamment n'importe quel élément sélectionné dans le groupe constitué par une liaison avec le substrat poreux inorganique, un groupe hydroxyle, un groupe amino, un groupe alcoxy ayant 1 à 6 atomes de carbone et un groupe alkylamino ayant 1 à 12 atomes de carbone,
J1 représente un groupe alkyle ayant 2 à 20 atomes de carbone ou un groupe aryle ayant 7 à 20 atomes de carbone,
K1 représente chacun indépendamment un groupe alkyle ayant 1 à 20 atomes de carbone ou un groupe aryle ayant 6 à 20 atomes de carbone,
M1 représente un groupe alkylène ayant 1 à 6 atomes de carbone, et
N1 représente un groupe alkyle ayant 2 à 20 atomes de carbone ou un groupe aryle ayant 6 à 20 atomes de carbone.

2. Substrat poreux inorganique selon la revendication 1, dans lequel le substrat poreux inorganique présente un diamètre de pore de 40 nm à 500 nm, tel que déterminé par le procédé d'intrusion de mercure.

3. Substrat poreux inorganique selon l'une quelconque des revendications 1 à 2, dans lequel le substrat poreux inorganique présente une surface spécifique de 0,1 $m^2/g$ jusqu'à 200 $m^2/g$, telle que déterminée par une mesure des isothermes d'adsorption/de désorption d'azote.

4. Substrat poreux inorganique selon l'une quelconque des revendications 1 à 3, comprenant de la silice, du gel de silice, de la zéolite ou du verre.

5. Substrat poreux inorganique selon l'une quelconque des revendications 1 à 4, dans lequel le substrat poreux inorganique comprend un groupe NH actif représenté par la formule (NH-1) suivante, dans lequel la quantité de groupe NH actif satisfait la formule mathématique (NH#1) suivante :

Si-A1-NH-B1 ··· (NH- 1)

dans lequel dans la formule (NH-1),
A1 représente un groupe organique ayant 1 à 20 atomes de carbone,
B1 représente n'importe quel élément sélectionné dans le groupe constitué par un atome d'hydrogène, un groupe alkyle ayant 1 à 6 atomes de carbone et un groupe aryle ayant 6 à 12 atomes de carbone

$$0{,}05 \leq I1/S1 \leq 6{,}0 \cdots \text{(NH\#1)}$$

I1: la quantité du groupe NH actif dans le substrat poreux inorganique (μmol/g), et
S1: la surface spécifique ($m^2/g$) du substrat poreux inorganique obtenu par une mesure des isothermes d'adsorption/de désorption d'azote.

6. Substrat poreux inorganique selon l'une quelconque des revendications 1 à 5, dans lequel le groupe silyle (B) est représenté par la formule (ii-3).

7. Substrat poreux inorganique selon l'une quelconque des revendications 1 à 6, dans lequel le groupe silyle représenté par la formule (ii-3) est un groupe silyle représenté par la formule (ii-3-1) suivante :

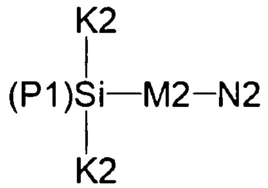

··· (ii-3-1)

dans lequel dans la formule (ii-3-1),
P1 représente la liaison avec le substrat poreux inorganique,
K2 représente indépendamment un groupe alkyle ayant 1 à 6 atomes de carbone ou un groupe aryle ayant 6 à 10 atomes de carbone,
M2 représente un groupe alkylène ayant 1 à 4 atomes de carbone, et
N2 représente un groupe alkyle ayant 2 à 6 atomes de carbone ou un groupe aryle ayant 6 à 10 atomes de carbone.

8. Substrat poreux inorganique selon l'une quelconque des revendications 1 à 7, dans lequel A1 dans la formule (i-1) est un groupe alkylène ayant 1 à 20 atomes de carbone qui peut, facultativement, contenir un quelconque ou plusieurs d'un groupe imino, d'un groupe oxy et d'un groupe thio.

9. Substrat poreux inorganique selon l'une quelconque des revendications 1 à 8, dans lequel le groupe silyle (A) est représenté par la formule (i-1-1) suivante :

$$(X1)(Z1)_2Si\text{-}A2\text{-}NH\text{-}B2 \cdots \quad \text{(i-1-1)}$$

dans lequel dans la formule (i-1-1),
X1 représente la liaison avec le substrat poreux inorganique,
Z1 représente chacun indépendamment un groupe alkyle ayant 1 à 6 atomes de carbone ou un groupe aryle ayant 6 à 12 atomes de carbone,
A2 représente un groupe alkylène ayant 1 à 15 atomes de carbone qui peut, facultativement, contenir un quelconque ou plusieurs d'un groupe imino, d'un groupe oxy et d'un groupe thio et
B2 représente n'importe quel élément sélectionné dans le groupe constitué par un atome d'hydrogène et un groupe alkyle ayant 1 à 2 atomes de carbone.

10. Support poreux inorganique qui comprend des groupes silyles des éléments (vi) et (vii) suivants et qui présente la caractéristique (viii) suivante :

(vi) un groupe silyle (C) : un groupe silyle représenté par la formule (vi-1) suivante,
(vii) un groupe silyle (D) : au moins un groupe silyle sélectionné dans le groupe constitué par des groupes silyles représentés par les formules (vii-1), (vii-2) et (vii-3) suivantes, et
(viii) un diamètre de pore de 20 nm ou plus, tel que déterminé par le procédé d'intrusion de mercure :

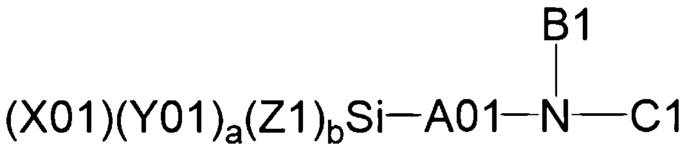

··· (vi−1)

dans lequel dans la formule (vi-1),
X01 représente une liaison avec le support poreux inorganique,
Y01 représente indépendamment n'importe quel élément sélectionné dans le groupe constitué par la liaison avec le support poreux inorganique, un groupe hydroxyle, un groupe amino, un groupe alcoxy ayant 1 à 6 atomes de carbone et un groupe alkylamino ayant 1 à 12 atomes de carbone,
Z1 représente chacun indépendamment un groupe alkyle ayant 1 à 6 atomes de carbone ou un groupe aryle ayant 6 à 12 atomes de carbone,
a représente un entier représenté par 2-b,
b représente un entier de 0 à 2,
A01 représente un groupe organique ayant 1 à 20 atomes de carbone,
B1 représente n'importe quel élément sélectionné dans le groupe constitué par un atome d'hydrogène, un groupe alkyle ayant 1 à 6 atomes de carbone et un groupe aryle ayant 6 à 12 atomes de carbone,
C1 représente un groupe présentant une structure nucléosidique ou nucléotidique dans laquelle un groupe réactif est protégé ou déprotégé,

(P01)(Q01)(R01)Si-J1 ··· (vii-1),

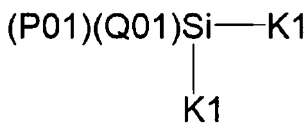

··· (vii−2)

,

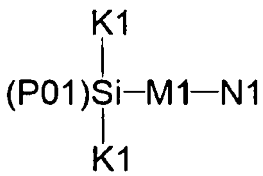

··· (vii−3)

,

dans lequel dans la formule (vii-1), (vii-2) ou (vii-3),
P01 représente la liaison avec le support poreux inorganique,
Q01 et R01 représentent chacun indépendamment n'importe quel élément sélectionné dans le groupe constitué par la liaison avec le support poreux inorganique, un groupe hydroxyle, un groupe amino, un groupe alcoxy ayant 1 à 6 atomes de carbone, et un groupe alkylamino ayant 1 à 12 atomes de carbone,
J1 représente un groupe alkyle ayant 2 à 20 atomes de carbone ou un groupe aryle ayant 7 à 20 atomes de carbone,
K1 représente chacun indépendamment un groupe alkyle ayant 1 à 20 atomes de carbone ou un groupe aryle ayant 6 à 20 atomes de carbone,
M1 représente un groupe alkylène ayant 1 à 6 atomes de carbone, et
N1 représente un groupe alkyle ayant 2 à 20 atomes de carbone ou un groupe aryle ayant 6 à 20 atomes de carbone.

**11.** Support poreux inorganique selon la revendication 10, dans lequel le support poreux inorganique présente un diamètre de pore de 40 nm à 500 nm, tel que déterminé par le procédé d'intrusion de mercure.

**12.** Support poreux inorganique selon l'une quelconque des revendications 10 à 11, dans lequel le support poreux inorganique présente un volume de pore cumulé dans une plage de diamètres de pores de 40 nm à 1000 nm est

supérieur à 0,32 ml/g et 4 ml/g ou moins, selon le procédé d'intrusion de mercure.

**13.** Support poreux inorganique selon l'une quelconque des revendications 10 à 12, dans lequel le support poreux inorganique présente une surface spécifique de 0,1 $m^2/g$ ou plus et 200 $m^2/g$ ou moins, telle que déterminée par une mesure des isothermes teneur d'adsorption/de désorption d'azote.

**14.** Support poreux inorganique selon l'une quelconque des revendications 10 à 13, dans lequel le support poreux inorganique comprend un corps poreux inorganique qui est composé de silice, de gel de silice, de zéolite ou de verre.

**15.** Support poreux inorganique selon l'une quelconque des revendications 10 à 14, dans lequel une quantité d'un groupe contenant une structure nucléosidique ou nucléotidique dans laquelle un groupe réactif est protégé ou déprotégé satisfait à la formule mathématique (Nu#1)suivante :

$$0{,}05 \leq I01/S01 \leq 3{,}0 \cdots \text{(Nu\#1)}$$

I01 : un groupe (μmol/g) présentant une structure nucléosidique ou nucléotidique dans laquelle un groupe réactif est protégé ou déprotégé dans le support poreux inorganique, et
S01 : une surface spécifique ($m^2/g$) du support poreux inorganique obtenu par une mesure des isothermes d'adsorption/de désorption d'azote.

**16.** Support poreux inorganique selon l'une quelconque des revendications 10 à 15, dans lequel C1 dans la formule générale (vi-1) contient un lieur succinyle.

**17.** Support poreux inorganique selon l'une quelconque des revendications 10 à 16, comprenant un groupe silyle représenté par la formule (vii-3) en tant que groupe silyle (D).

**18.** Support poreux inorganique selon l'une quelconque des revendications 10 à 17, dans lequel le groupe silyle représenté par la formule (vii-3) est un groupe silyle représenté par la formule (vii-3-1) suivante :

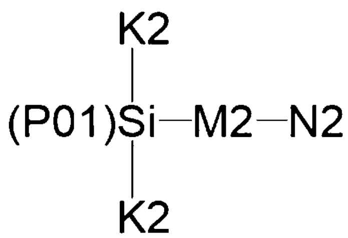

・・・ (vii－3－1)

dans lequel dans la formule (ii-3-1),
P01 représente la liaison avec le support poreux inorganique,
K2 représente indépendamment un groupe alkyle ayant 1 à 6 atomes de carbone ou un groupe aryle ayant 6 à 10 atomes de carbone,
M2 représente un groupe alkylène ayant 1 à 4 atomes de carbone, et
N2 représente un groupe alkyle ayant 2 à 6 atomes de carbone ou un groupe aryle ayant 6 à 10 atomes de carbone.

**19.** Support poreux inorganique selon l'une quelconque des revendications 10 à 18, dans lequel A01 dans la formule (vi-1) est un groupe alkylène ayant 1 à 20 atomes de carbone qui peut, facultativement, contenir un quelconque ou plusieurs d'un groupe acylimino, d'un groupe oxy et d'un groupe thio.

**20.** Support poreux inorganique selon l'une quelconque des revendications 10 à 19, dans lequel le groupe silyle (C) est représenté par la formule (vi-1-1) suivante :

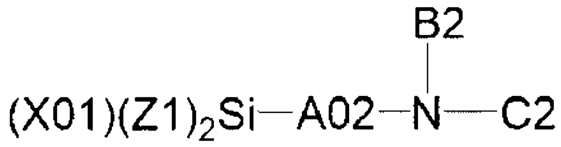

• • • (vi−1−1)

dans lequel dans la formule (vi-1-1),
X01 représente la liaison avec le support poreux inorganique,
Z1 représente chacun indépendamment un groupe alkyle ayant 1 à 6 atomes de carbone ou un groupe aryle ayant 6 à 12 atomes de carbone,
A02 représente un groupe alkylène ayant 1 à 15 atomes de carbone qui peut, facultativement, contenir un quelconque ou plusieurs d'un groupe acylimino, d'un groupe oxy et d'un groupe thio,
B2 représente n'importe quel élément sélectionné dans le groupe constitué par un atome d'hydrogène et un groupe alkyle ayant 1 à 2 atomes de carbone, et
C2 représente un groupe incluant un lieur succinyle et présentant une structure nucléosidiquee ou nucléotidique dans laquelle un groupe réactif est protégé ou déprotégé.

**21.** Procédé de production d'acide nucléique à l'aide d'un support poreux inorganique selon l'une quelconque des revendications 10 à 20, dans lequel C1 dans la formule (vi-1) comporte un groupe ayant une structure nucléosidique ou nucléotidique dans laquelle le groupe hydroxyle, en tant que groupe réactif, est protégé, le procédé de production d'acide nucléique comprenant :

une étape (A) de déprotection d'un groupe protecteur du groupe hydroxyle à la position 5' du nucléoside ;
une étape (B) de production d'un phosphite en soumettant le groupe hydroxyle à la position 5' du nucléoside produit à l'étape (A) à une réaction de condensation avec un composé amidite ayant une seconde base nucléosidique ;
une étape (C) d'oxydation du phosphite produit à l'étape (B) pour produire un nucléotide ; et
une étape (D) de déprotection du groupe protecteur du groupe hydroxyle à la position 5' du nucléotide produit à l'étape (C).

**22.** Procédé de production d'acide nucléique selon la revendication 21, comprenant en outre :

une étape (B') de soumission d'un produit obtenu à l'étape (D) à une réaction de condensation avec un composé amidite ayant une base nucléosidique à introduire ensuite pour produire un phosphite ;
une étape (C') d'oxydation du phosphite produit à l'étape (B') pour produire un oligonucléotide ; et
une étape (D') de déprotection du groupe protecteur du groupe hydroxyle à la position 5' d'une extrémité d'une chaîne oligonucléotidique produite à l'étape (C').

**23.** Procédé de production d'acide nucléique selon la revendication 22, comprenant une étape (E) de répétition m fois d'une série d'étapes incluant l'étape (B'), l'étape (C') et l'étape (D') (m représente un entier de 1 ou plus) pour faire réagir m composés amidites, et, ensuite, de découpe d'un acide nucléique allongé.

**24.** Utilisation du support poreux inorganique selon l'une quelconque des revendications 10 à 20 dans la production d'un acide nucléique par un procédé de phosphoramidite.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2020084641 A
- EP 3950126 A1
- WO 0050432 A2
- US 2014135478 A1
- JP 2003104996 A
- JP 2016202097 A
- JP 2006502856 A
- CN 108176387 A
- WO 2013062105 A1
- WO 2020202951 A1
- WO 2020202952 A1
- JP 3181334 A
- US 3687808 A
- WO 2013027843 A1
- WO 2012017919 A1
- WO 2013103146 A1
- JP 5875843 B
- US 2012035246 A1


### Non-patent literature cited in the description


- **FARRE, C. et al.** *Langmuir*, 2010, vol. 26 (7), 4941-4950
- *TOSOH Research & Technology Review*, 2001, vol. 45
- **A.P. GUZAEV** ; **M. MANOHARAN**. *J Am Chem Soc*, 2003, vol. 125, 2380-2381
- **R.K. KUMAR** ; **A.P. GUZAEV** ; **C. RENTEL** ; **V.T RAVIKUMAR**. *Tetrahedron*, 2006, vol. 62, 4528
- Concise Encyclopedia Of Polymer Science And Engineering. John Wiley & Sons, 1990, 858-859
- **ENGLISCH et al.** Angewandte Chemie. 1991, vol. 30, 613
- Reviews by Chakhmakhcheva: Protective Groups in the Chemical Synthesis of Oligoribonucleotides. *Russian Journal of Bioorganic Chemistry*, 2013, vol. 39 (1), 1-21